# EUROPEAN PATENT SPECIFICATION

(11) **EP 2 288 340 B1**
(45) Date of publication and mention of the grant of the patent: **24.06.2020**
(21) Application number: 09729316.1
(22) Date of filing: 10.04.2009
(51) Int. Cl.: A61K 9/19, A61K 9/51, A61K 31/337

(54) **NANOPARTICLE FORMULATIONS AND USES THEREOF**
NANOTEILCHEN-FORMULIERUNGEN UND IHRE VERWENDUNGEN
FORMULATIONS DE NANOPARTICULES ET UTILISATIONS DE CELLES-CI

(30) Priority: 10.04.2008 US 44006 P; 12.09.2008 US 96664 P
(43) Date of publication of application: 02.03.2011
(73) Proprietor: Abraxis BioScience, LLC, Summit, NJ 07901 (US)
(72) Inventor: DESAI, Neil, P., Pacific Palisades, CA 90272 (US); TAO, Chunlin, Summit, NJ 07901 (US); DE, Tapas, Los Angeles CA 90034 (US); CI, Sherry, Xiaopei, San Marino CA 91108 (US); TRIEU, Vuong, Agoura Hills, CA 91301 (US)
(74) Representative: Jones Day
(86) International application number: PCT/US2009/040281
(87) International publication number: WO 2009/126938

(56) References cited:
- WO-A-97/10234
- WO-A-2006/089207
- WO-A-2006/091780
- US-A1- 2004 024 051
- US-B1- 6 749 868
- ALI S ET AL: "Hydrolyzable hydrophobic taxanes: synthesis and anti-cancer activities" ANTI-CANCER DRUGS, RAPID COMMUNICATIONS, OXFORD, vol. 12, no. 2, 1 February 2001 (2001-02-01), pages 117-128, XP009103673 ISSN: 0959-4973

## Description

### BACKGROUND OF THE INVENTION

Taxanes, in particular the two currently available taxane drugs, paclitaxel and docetaxel, are potent antitumor agents. Paclitaxel is very poorly water soluble (less than 10 µg/mL), and as a result, cannot be practically formulated with an aqueous medium for IV administration. Currently, paclitaxel is formulated for IV administration to patients with cancer in a solution with polyoxyethylated castor oil (Polyoxyl 35 or Cremophor®) as the primary solvent/surfactant, with high concentrations of ethanol employed as co-solvent. One of the major difficulties in the administration of paclitaxel is the occurrence of hypersensitivity reactions. These reactions, which include severe skin rashes, hives, flushing, dyspnea, tachycardia and others, may be attributed at least in part to the high concentrations of ethanol and Cremophor® used as solvents in the formulation. Docetaxel, a derivative of paclitaxel, is semisynthetically produced from 10-deacetyl baccatin III, a noncytotoxic precursor extracted from the needles of Taxus baccata and esterified with a chemically synthesized side chain. Like paclitaxel, docetaxel is very poorly soluble in water. Currently, the most preferred solvent/surfactant used to dissolve docetaxel is polysorbate 80 (Tween 80). Like Cremophor® Tween often causes hypersensitivity reactions in patients. Further, Tween 80 cannot be used with PVC delivery apparatus because of its tendency to leach diethylhexyl phthalate, which is highly toxic.

Great efforts have been invested on the development of water soluble prodrugs and new taxane derivatives with higher hydrophilic groups (such as water soluble polymers) to enhance water solubility. For example, US 2003/0203961 described taxane- polyethylene glycol (PEG) conjugates, which operate as prodrugs and hydrolyzes under normal physiological conditions to provide therapeutically active taxanes. While conjugates of paclitaxel with high molecular weight PEG polymers have increased solubility, they also result in a corresponding decrease in drug load, due to the high molecular weight PEG necessary to achieve adequate solubility. Similarly, WO94/13324 disclosed phospholipid prodrugs to enhance water solubility.

Another approach to address the problem associated with the poor water solubility of taxane is the development of various formulations of taxane such as nanoparticles, oil-in-water emulsions, and liposomes. For example, Abraxane® is a nanoparticle composition of paclitaxel and albumin. Nanoparticle compositions of substantially poorly water soluble drugs and uses thereof have been disclosed, for example, in U.S. Patent Nos. 5,916,596; 6,096,331; 6,749,868; and 6,537,579; and PCT Application Pub. Nos. WO98/14174, WO99/00113, WO07/027941 and WO07/027819.

Various Taxane derivatives have been disclosed in *e.g.*, Kingston, J. Nat. Prod. 2000, 63, 726-734; Deutsch et al, J. Med. Chem. 1989, 32, 788-792; Mathew et al, J. Med. Chem. 1992, 35, 145-151, EP Pat. No. 1 063 234; and U.S. Pat. Nos. 5,059,699; 4,942,184; 6,482,850; and 6,602,902.

### BRIEF SUMMARY OF THE INVENTION

The present invention provides a composition comprising nanoparticles, wherein the nanoparticles comprise a hydrophobic taxane derivative coated with albumin, wherein at least about 5% of the albumin in the nanoparticle portion of the composition is crosslinked, and wherein the hydrophobic taxane derivative is of the formula: 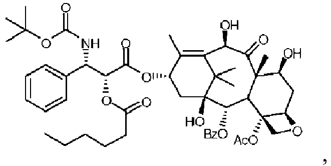 or a pharmaceutically acceptable salt, isomer, or solvate thereof.

In one embodiment, the albumin is human serum albumin.

In one embodiment, the composition comprises albumin in both nanoparticle form and non-nanoparticle form, and wherein less than about 25% of the albumin is in nanoparticle form.

In one embodiment, the average diameter of the nanoparticles in the composition is less than about 200 nm, or is less than about 100 nm.

In one embodiment, greater than 80%, or greater than 90% of the nanoparticles in the composition have a diameter of less than about 100 nm.

In one embodiment, the nanoparticles have a size of more than about 20 nm at 10 µg/ml in a dissolution study in 5% HSA at 37 °C by HPLC.

In one embodiment, the nanoparticles have a size of more than about 30 nm at 50 µg/ml in a dissolution study in 5% HSA at 37 °C by HPLC.

In one embodiment, the nanoparticles exhibit one or more of the following dissolution profile when measured in 5% HSA at 37 °C by HPLC:
a) more than about 50 nm at 200 µg/ml;
b) more than about 40 nm at 100 µg/ml;and
c) more than about 20 nm at 10 µg/ml.

In one embodiment, the nanoparticles exhibit one or more of the following dissolution profile when measured in 5% HSA at 37 °C by HPLC:
a) more than about 60 nm at 400 µg/ml;
b) more than about 50 nm at 200 µg/ml;
c) more than about 40 nm at 100 µg/ml;
d) more than about 20 nm at 10 µg/ml;
e) more than about 20 nm at 5 µg/ml.

In one embodiment, the EC50 of the dissolution profile when measured in 5% HSA at 37 °C is less than about 25% of the EC50 for the unmodified taxane in the same nanoparticle formulation.

In one embodiment, the nanoparticle composition when administered to a primate exhibits a Cmax in the blood at between about 0.05 hour to about 0.3 hour after administration.

In one embodiment, the nanoparticle composition when administered in a primate exhibits break down in blood with terminal half life of about 1 hour to about 5 hours.

The present invention also provides a composition of the invention for use in a method of treating a proliferative disease in an individual, said method comprising administering to the individual an effective amount of the composition.

In one embodiment, the proliferative disease is cancer, optionally wherein the cancer is a solid tumor; or wherein the cancer is selected from multiple myeloma, renal cell carcinoma, prostate cancer, lung cancer, melanoma, colon cancer, ovarian cancer, or breast cancer.

In one embodiment, said method comprises administration of the composition parenterally or intravenously.

The drug/hydrophobic drug derivative/compound mentioned herein in the context of the present invention is a hydrophobic taxane derivative as defined in the claims. The carrier protein mentioned herein in the context of the present invention is albumin.

Disclosed herein are compositions comprising nanoparticles, wherein the nanoparticles comprise a drug and a carrier protein. In some examples, the nanoparticles comprise a hydrophobic drug derivative and a carrier protein. In some examples, the nanoparticles comprise a hydrophobic taxane derivative and a carrier protein. In some examples, the nanoparticles comprise a drug that is not a hydrophobic drug derivative (*e.g.*, not a hydrophobic taxane derivative), and a carrier protein. In some examples, the nanoparticles comprise a hydrophobic drug derivative that is not a hydrophobic taxane derivative, and a carrier protein. In some examples, the carrier protein is albumin (such as human serum albumin).

In some embodiments, the nanoparticles in the composition provided herein have an average diameter of no greater than about 150 nm, including for example no greater than about any one of 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 150 nm, including for example no greater than about any one of 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 150 nm, including for example any one of about 30 to about 140 nm, and any one of about 40 to about 130, about 50 to about 120, and about 60 to about 100 nm.

In some embodiments, the carrier protein has sulfhydral groups that can form disulfide bonds. For the purpose of the present invention, at least about 5% (including for example at least about any one of 10%, 15%, or 20%) of the carrier protein in the nanoparticle portion of the composition are crosslinked (for example crosslinked through one or more disulfide bonds).

For the purpose of the invention, the nanoparticles comprise the drug coated with albumin (*e.g.*, human serum albumin). In some embodiments, the composition comprises the drug, in non-nanoparticle form, wherein at least about any one of 50%, 60%, 70%, 80%, 90%, 95%, or 99% of the hydrophobic taxane derivative in the composition are in nanoparticle form. In some embodiments, the drug in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of the drug that is substantially free of polymeric materials (such as polymeric matrix).

In some embodiments, the composition is substantially free (such as free) of surfactants (such as Cremophor® Tween 80, or other organic solvents used for the administration of taxanes). In some embodiments, the composition contains less than about any one of 20%, 15%, 10%, 7.5%, 5%, 2.5%, or 1% organic solvent. In some embodiments, the weight ratio of carrier protein (i.e., albumin) and the drug in the composition is about 18:1 or less, such as about 15:1 or less, for example about 10:1 or less. In some embodiments, the weight ratio of carrier protein (i.e., albumin) and hydrophobic taxane derivative in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of the carrier protein and hydrophobic taxane derivative in the nanoparticle portion of the composition is about any one of 1:2, 1:3, 1:4, 1:5, 1:10, 1:15, or less.

In some embodiments, the particle composition comprises one or more of the above characteristics.

In some examples, the hydrophobic taxane derivative has a hydrophobic group attached to the 2'-hydroxyl position of the taxane. In some examples, the hydrophobic group is an acyl group, such as -C(O)-C₄-C₁₀ alkyl, for example -C(O)-C₆ alkyl. In some examples, the hydrophobic group is an acyl group attached to the 2'-hydroxyl of the taxane. In some example, the hydrophobic taxane derivative is a prodrug of the taxane.

In some examples, the hydrophobic drug derivative (*e.g.*, a hydrophobic taxane derivative, such as any one of compounds 1, 2, 3-23 and any compound of Formula I, II, III, IV, V, or VI) has an improved binding to albumin over the corresponding unmodified taxane (*e.g.*, improved over paclitaxel and/or docetaxel). In some examples, the hydrophobic drug derivative (*e.g*., hydrophobic taxane derivative) in the protein nanoparticle composition shows improved therapeutic efficacy over nanoparticle compositions of the corresponding unmodified drug (*e.g.*, taxane, such as paclitaxel and/or docetaxel) at an equal-toxicity dose.

In some examples, the hydrophobic taxane derivative is a compound of formula I described herein. In some examples, the hydrophobic taxane derivative is a compound of formula II described herein. In some examples, the hydrophobic taxane derivative is a compound of formula III described herein. In some examples, the hydrophobic taxane derivative is a compound of formula IV described herein. In some examples, the hydrophobic taxane derivative is a compound of formula V described herein. In some examples, the hydrophobic taxane derivative is a compound of formula VI described herein. In some examples, the hydrophobic taxane derivative is any one of compounds 1-23 described herein. The hydrophobic taxane derivative of the present invention is compound 2 described herein.

Also provided herein are the compositons of the invention for use in methods for treating diseases (such as cancer), as well as kits and unit doses for uses described herein.

### BRIEF DESCRIPTION OF THE FIGURES

Figure 1 shows the amount of docetaxel produced versus incubation time for hydrophobic taxane derivatives.
Figure 2A shows the effects of increasing concentrations of nanoparticles containing a hydrophobic taxane derivative and albumin compared with Taxotere® on tumor growth in a breast cancer xenograft model.
Figure 2B shows the effects of increasing concentrations of nanoparticles containing a hydrophobic taxane derivative and albumin compared with Taxotere® on body weight change in a breast cancer xenograft model.
Figure 3A shows the effects of nanoparticles containing a hydrophobic taxane derivative and albumin compared with Taxotere® on tumor volume changes in a H358 lung cancer xenograft model.
Figure 3B shows the effects of nanoparticles containing a hydrophobic taxane derivative and albumin compared with Taxotere® on body weight change in a H358 lung cancer xenograft model.
Figure 4A shows the effects of nanoparticles containing a hydrophobic taxane derivative and albumin compared to Nab-docetaxel on tumor growth in a HT29 colon cancer xenograft model (Study number CA-AB-6).
Figure 4B shows the effects of nanoparticles containing a hydrophobic taxane derivative and albumin compared to Nab-docetaxel on body weight change in a HT29 colon cancer xenograft model (Study number CA-AB-6).
Figure 5A shows the effects of nanoparticles containing a hydrophobic taxane derivative and albumin compared to Taxotere® on tumor growth in a colon cancer HT29 xenograft model (Study number CA-AB-6).
Figure 5B shows the effects of nanoparticles containing a hydrophobic taxane derivative and albumin compared to Taxotere® on body weight change in a colon cancer HT29 xenograft model (Study number CA-AB-6).
Figure 6A shows the effects of increasing concentrations of nanoparticles containing a hydrophobic taxane derivative and albumin compared with Taxotere® on tumor growth in a colon cancer HT29 xenograft model (Study number ABS-18).
Figure 6B shows the effects of increasing concentrations of nanoparticles containing a hydrophobic taxane derivative and albumin compared with Taxotere® on weight change in a colon cancer HT29 xenograft model (Study number ABS-18).
Figure 7 shows the repeat-dose toxicity for nanoparticles containing a hydrophobic taxane derivative and albumin.
Figure 8 shows the particle distribution and mean particle size of nanoparticles containing a hydrophobic taxane derivative and albumin.
Figure 9 shows a particle dissolution profile for the nanoparticle composition Nab-**2**.
Reference Figure 10 shows a particle dissolution profile for the nanoparticle composition Nab-docetaxel.
Figure 11 shows normalized dissolution profiles for the nanoparticle compositions Nab-**2** and Nab-docetaxel.
Reference Figure 12 shows mean particle size and zeta potential of Nab-paclitaxel nanoparticles.
Reference Figure 13 shows Cryo and standard TEM of Nab-paclitaxel nanoparticles.
Reference Figure 14 shows x-ray characterization of paclitaxel and Nab-paclitaxel.
Reference Figure 15 shows particle size of Nab-paclitaxel at various concentrations in simulated plasma (5% HAS).
Reference Figure 16 shows particle size of Nab-paclitaxel at various concentrations in pig plasma.
Reference Figure 17 shows particle size of Nab-paclitaxel at various concentrations in pig whole blood.
Reference Figure 18 shows plasma paclitaxel concentration (by HPLC) and particle size (by DLS) versus time after dosing Yucatan mini pigs with Nab-paclitaxel nanoparticles over 30 min.

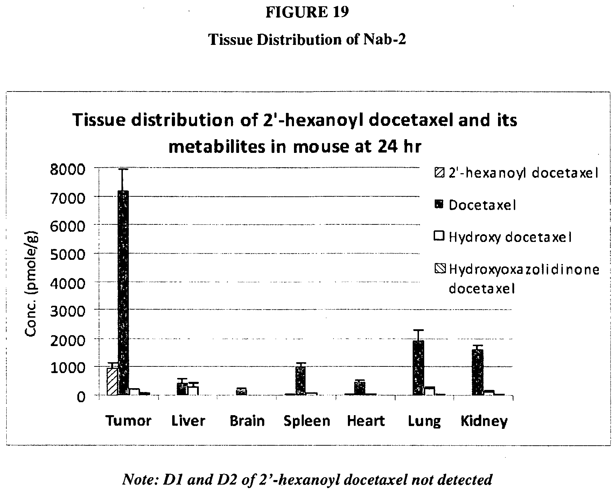

Figure 19 shows tissue distribution of Nab-**2**.

### DETAILED DESCRIPTION OF THE INVENTION

Disclosed herein are drugs, for example taxane derivatives that are formulated in protein-based nanoparticles. Some drug derivatives, such as taxane derivatives, have a hydrophobic group attached to the corresponding drug and have increased hydrophobicity as compared to the unmodified drug.

We have found that taxane derivatives containing a hydrophobic group (such as an acyl group, for example a -C(O)-C₄-C₁₀ alkyl group, particularly a -C(O)-C₆ alkyl group attached to the 2'-hydroxyl of a taxane), when formulated into protein nanoparticle compositions, produce nanoparticles with significantly improved properties over protein nanoparticles of an unmodified taxane. These properties may include, but are not limited to, one or more of the following: small particle sizes (for example an average diameter of less than about 100 nm), narrow size distribution for the particles, enhanced delivery of the compound to their intended site(s) of action, delayed or sustained release, delayed clearance, and increased efficacy against cancer. The compositions described here are therefore particularly suitable for use in treating diseases such as cancer.

Accordingly, disclosed herein is a composition comprising nanoparticles comprising: 1) a hydrophobic drug derivative; and 2) a carrier protein. In some examples, the hydrophobic drug derivative is a prodrug.

Further disclosed herein is a composition comprising nanoparticles comprising: 1) a hydrophobic taxane derivative; and 2) a carrier protein. In some embodiments, the hydrophobic taxane derivative is a prodrug.

Also disclosed herein is a method of treating diseases (such as cancer) using the compositions described herein.

Also disclosed are kits and unit dosage forms.

### Abbreviations and Definitions

The abbreviations used herein have their conventional meaning within the chemical and biological arts.
As used herein, "hydrophobic drug derivative" refers to a drug substituted with a hydrophobic group. For example, a "hydrophobic taxane derivative" refers to a taxane substituted with a hydrophobic group. A "hydrophobic group" refers to a moiety which when substituted on a taxane, results in a taxane derivative with increased hydrophobic character compared to the unsubstituted taxane. Increased hydrophobic character may be determined, for example, by decreased water solubility, decreased polarity, decreased potential for hydrogen bonding, and/or an increased oil/water partition coefficient. "Taxane" as used herein includes paclitaxel and docetaxel. The term "hydrophobic taxane derivative" thus does not include paclitaxel or docetaxel.
The terms "halo" or "halogen," by themselves or as part of another substituent, mean, unless otherwise stated, a fluorine, chlorine, bromine, or iodine atom.
The term "alkyl," by itself or as part of another substituent, means, unless otherwise stated, a fully saturated straight-chain (linear; unbranched) or branched chain, or combination thereof, having the number of carbon atoms specified, if designated (*i.e.* C₁-C₁₀ means one to ten carbons). Examples include, but are not limited to, groups such as methyl, ethyl, n-propyl, isopropyl, n-butyl, t-butyl, isobutyl, sec-butyl, homologs and isomers of, for example, n-pentyl, n-hexyl, n-heptyl, n-octyl, and the like. If no size is designated, the alkyl groups mentioned herein contain 1-20 carbon atoms, typically 1-10 carbon atoms, or 1-8 carbon atoms, or 1-6 carbon atoms, or 1-4 carbon atoms.
The term "alkenyl" refers to unsaturated aliphatic groups including straight-chain (linear; unbranched), branched-chain groups, and combinations thereof, having the number of carbon atoms specified, if designated, which contain at least one double bond (-C=C-). All double bonds may be independently either (*E*) or (*Z*) geometry, as well as mixtures thereof. Examples of alkenyl groups include, but are not limited to, -CH₂-CH=CH-CH₃; -CH=CH-CH=CH₂ and -CH₂-CH=CH-CH(CH₃)-CH₂-CH₃. If no size is designated, the alkenyl groups mentioned herein contain 2-20 carbon atoms, typically 2-10 carbon atoms, or 2-8 carbon atoms, or 2-6 carbon atoms, or 2-4 carbon atoms.
The term "alkynyl" refers to unsaturated aliphatic groups including straight-chain (linear; unbranched), branched-chain groups, and combinations thereof, having the number of carbon atoms specified, if designated, which contain at least one carbon-carbon triple bond (-C≡C-). Examples of alkynyl groups include, but are not limited to, -CH₂-C≡C-CH₃; -C≡C-C≡CH and -CH₂-C≡C-CH(CH₃)-CH₂-CH₃. If no size is designated, the alkynyl groups mentioned herein contain 2-20 carbon atoms, typically 2-10 carbon atoms, or 2-8 carbon atoms, or 2-6 carbon atoms, or 2-4 carbon atoms.
The term "cycloalkyl" by itself or in combination with other terms, represents, unless otherwise stated, cyclic versions of alkyl, alkenyl, or alkynyl, or mixtures thereof. Additionally, cycloalkyl may contain fused rings, but excludes fused aryl and heteroaryl groups. Examples of cycloalkyl include, but are not limited to, cyclopropyl, cyclobutyl, cyclopentyl, cyclohexyl, 1-cyclohexenyl, 3-cyclohexenyl, cycloheptyl, norbornyl, and the like. If no size is designated, the alkynyl groups mentioned herein contain 3-9 carbon atoms, typically 3-7 carbon atoms.
The term "heterocycloalkyl," by itself or in combination with other terms, represents a cycloalkyl radical containing of at least one annular carbon atom and at least one annular heteroatom selected from the group consisting of O, N, P, Si and S, and wherein the nitrogen and sulfur atoms may optionally be oxidized and the nitrogen heteroatom may optionally be quaternized. Often, these annular heteroatoms are selected from N, O and S. A heterocycloalkyl group can be attached to the remainder of the molecule at an annular carbon or annular heteroatom. Additionally, heterocycloalkyl may contain fused rings, but excludes fused aryl and heteroaryl groups. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1 -piperazinyl, 2-piperazinyl, and the like.
The terms "cycloalkyl-alkyl" and "heterocycloalkyl-alkyl" designate an alkyl-substituted cycloalkyl group and alkyl-substituted heterocycloalkyl, respectively, where the alkyl moiety is attached to the parent structure. Non-limiting examples include cyclopropyl-ethyl, cyclobutyl-propyl, cyclopentyl-hexyl, cyclohexyl-isopropyl, 1-cyclohexenyl-propyl, 3-cyclohexenyl-t-butyl, cycloheptylheptyl, norbornyl-methyl, 1-piperidinyl-ethyl, 4-morpholinyl-propyl, 3-morpholinyl-t-butyl, tetrahydrofuran-2-yl-hexyl, tetrahydrofuran-3-yl-isopropyl, and the like. Cycloalkyl-alkyl and heterocycloalkyl-alkyl also include substituents in which at least one carbon atom is present in the alkyl group and wherein another carbon atom of the alkyl group has been replaced by, for example, an oxygen, nitrogen or sulfur atom (e.g., cyclopropoxymethyl, 2-piperidinyloxy-t-butyl, and the like).
The term "aryl" means, unless otherwise stated, a polyunsaturated, aromatic, hydrocarbon substituent which can be a single ring or multiple rings (e.g., from 1 to 3 rings) which are fused together or linked covalently. Additionally, aryl may contain fused rings, wherein one or more of the rings are optionally cycloalkyl or heterocycloalkyl. Examples of aryl groups include, but are not limited to, phenyl, 1-naphthyl, 2-naphthyl, 4-biphenyl.
The term "heteroaryl" refers to aryl groups (or rings) that contain from one to four annular heteroatoms selected from N, O, and S, wherein the nitrogen and sulfur atoms are optionally oxidized, and the nitrogen atom(s) are optionally quaternized. A heteroaryl group can be attached to the remainder of the molecule at an annular carbon or annular heteroatom. Additionally, heteroaryl may contain fused rings, wherein one or more of the rings is optionally cycloalkyl or heterocycloalkyl. Non-limiting examples of heteroaryl groups are 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems may be selected from the group of acceptable substituents described below.
The term "aralkyl" designates an alkyl-substituted aryl group, where the alkyl portion is attached to the parent structure. Examples are benzyl, phenethyl, and the like. "Heteroaralkyl" designates a heteroaryl moiety attached to the parent structure via an alkyl residue. Examples include furanylmethyl, pyridinylmethyl, pyrimidinylethyl, and the like. Aralkyl and heteroaralkyl also include substituents in which at least one carbon atom of the alkyl group is present in the alkyl group and wherein another carbon of the alkyl group has been replaced by, for example, an oxygen atom (e.g., phenoxymethyl, 2-pyridylmethoxy, 3-(1-naphthyloxy)propyl, and the like).
Each of the above terms (e.g., "alkyl," "alkenyl," "alkynyl," "cycloalkyl," "heterocycloalkyl," "cycloalkyl-alkyl," "heterocycloalkyl-alkyl," "aryl," "heteroaryl," "aralkyl," and "heteroaralkyl") are meant to include both substituted and unsubstituted forms of the indicated radical.
"Optionally substituted" or "substituted" refers to the replacement of one or more hydrogen atoms with a monovalent or divalent radical. Suitable substituent groups include, for example, hydroxyl, nitro, amino, imino, cyano, halo (such as F, Cl, Br, I), haloalkyl (such as -CCl₃ or -CF₃), thio, sulfonyl, thioamido, amidino, imidino, oxo, oxamidino, methoxamidino, imidino, guanidino, sulfonamido, carboxyl, formyl, alkyl, alkoxy, alkoxy-alkyl, alkylcarbonyl, alkylcarbonyloxy (- OCOR), aminocarbonyl, arylcarbonyl, aralkylcarbonyl, carbonylamino, heteroarylcarbonyl, heteroaralkyl-carbonyl, alkylthio, aminoalkyl, cyanoalkyl, carbamoyl (-NHCOOR- or -OCONHR-), urea (-NHCONHR-), aryl and the like. In some examples, the above groups (*e.g.*, alkyl groups) are substituted with, for example, amino, heterocycloalkyl, such as morpholine, piperazine, piperidine, azetidine, hydroxyl, methoxy, or heteroaryl groups such as pyrrolidine.
   A substituent group can itself be substituted. The group substituted onto the substitution group can be carboxyl, halo, nitro, amino, cyano, hydroxyl, alkyl, alkenyl, alkynyl, alkoxy, aminocarbonyl, -SR, thioamido, -SO₃H, -SO₂R or cycloalkyl, where R is typically hydrogen or alkyl.
   When the substituted substituent includes a straight chain group, the substituent can occur either within the chain (*e.g.*, 2-hydroxypropyl, 2-aminobutyl, and the like) or at the chain terminus (*e.g.*, 2-hydroxyethyl, 3-cyanopropyl, and the like). Substituted substituents can be straight chain, branched or cyclic arrangements of covalently bonded carbon or heteroatoms (N, O or S).
As used herein, "isomer" includes all stereoisomers of the compounds referred to in the formulas herein, including enantiomers, diastereomers, as well as all conformers, rotamers, and tautomers, unless otherwise indicated. The invention includes all enantiomers of the compounds of the invention, in either substantially pure levorotatory or dextrorotatory form, or in a racemic mixture, or in any ratio of enantiomers. For compounds disclosed as an (R)-enantiomer, also disclosed is the (S)-enantiomer; for compounds disclosed as the (S)-enantiomer, also disclosed is the (R)-enantiomer. The invention includes any diastereomers of the compounds of the invention in diastereomerically pure form and in the form of mixtures in all ratios.
   Unless stereochemistry is explicitly indicated in a chemical structure or chemical name, the chemical structure or chemical name is intended to embrace all possible stereoisomers, conformers, rotamers, and tautomers of the compound depicted. For example, a compound containing a chiral carbon atom is intended to embrace both the (R) enantiomer and the (S) enantiomer, as well as mixtures of enantiomers, including racemic mixtures; and a compound containing two chiral carbons is intended to embrace all enantiomers and diastereomers (including (R,R), (S,S), (R,S), and (R,S) isomers).
   In all uses of the compounds, the invention also includes use of any or all of the stereochemical, enantiomeric, diastereomeric, conformational, rotomeric, tautomeric, solvate, hydrate, polymorphic, crystalline form, non-crystalline form, and, pharmaceutically acceptable salt variations thereof.
   Certain compounds of the present invention can exist in unsolvated forms as well as solvated forms (*i.e.*, solvates). Compounds of the invention may also include hydrated forms (*i.e.*, hydrates). In general, the solvated and hydrated forms are equivalent to unsolvated forms for purposes of biological utility and are encompassed within the scope of the present invention. The invention also includes all polymorphs, including crystalline and non-crystalline forms. In general, all physical forms are equivalent for the uses contemplated by the present invention and are intended to be within the scope of the present invention.
   Disclosed herein are all salts of the compounds described herein, as well as methods of using such salts of the compounds. The invention also embraces all non-salt forms of the compounds of the invention. The invention also embraces pharmaceutically acceptable salts of the compounds of the invention. "Pharmaceutically acceptable salts" are those salts which retain the biological activity of the free compounds and which can be administered as drugs or pharmaceuticals to and individual (*e.g.*, a human). The desired salt of a basic functional group of a compound may be prepared by methods known to those of skill in the art by treating the compound with an acid. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, hippuric, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. The desired salt of an acidic functional group of a compound can be prepared by methods known to those of skill in the art by treating the compound with a base. Examples of inorganic salts of acid compounds include, but are not limited to, alkali metal and alkaline earth salts, such as sodium salts, potassium salts, magnesium salts, and calcium salts; ammonium salts; and aluminum salts. Examples of organic salts of acid compounds include, but are not limited to, procaine, dibenzylamine, N-ethylpiperidine, N,N'-dibenzylethylenediamine, and triethylamine salts.
The term "prodrug" refers to a compound which itself is relatively inactive, but is transformed into a more active compound following administration to the individual in which it is used, by a chemical or biological process *in vivo* (*e.g.*, by hydrolysis and/or an enzymatic conversion). Prodrugs include, for example, compounds wherein hydroxy, amine or thiol groups are bonded to any group that, when administered to an individual, becomes cleaved to form a free hydroxy, amino, or thiol group, respectively. Examples of prodrugs include, but are not limited to, acetate, formate and benzoate derivatives of alcohol and amine functional groups. Pharmaceutically acceptable prodrugs of the compounds include, but are not limited to, esters, carbonates, thiocarbonates, N-acyl derivatives, N-acyloxyalkyl derivatives, quaternary derivatives of tertiary amines, N-Mannich bases, Schiff bases, amino acid conjugates, phosphate esters, metal salts and sulfonate esters. A thorough discussion is provided in T. Higuchi and V. Stella, PRO-DRUGS AS NOVEL DELIVERY SYSTEMS, Vol. 14 of the A.C.S. Symposium Series, and in Edward B. Roche, ed., BIOREVERSIBLE CARRIERS IN DRUG DESIGN, American Pharmaceutical Association and Pergamon Press, 1987 In some examples, the taxane derivatives used are themselves prodrugs. In some examples, the taxane derivatives used are not prodrugs.
   A substantially pure compound means that the compound is present with no more than about 15% or no more than about 10% or no more than about 5% or no more than about 3% or no more than about 1% of the total amount of compound as impurity and/or in a different form. For instance, substantially pure S,S compound means that no more than about 15% or no more than about 10% or no more than about 5% or no more than about 3% or no more than about 1% of the total R,R; S,R; and R,S form is present.
"Protecting group" refers to a chemical group that exhibits the following characteristics: 1) is stable to the projected reactions for which protection is desired; 2) is removable from the protected substrate to yield the desired functionality; and 3) is removable by reagents compatible with the other functional group(s) present or generated in such projected reactions. Selection of suitable protecting groups for use in the methods described herein is within the ordinary skill level in the art. Examples of suitable protecting groups can be found in Greene et al. (1991) PROTECTIVE GROUPS IN ORGANIC SYNTHESIS, 3rd Ed. (John Wiley & Sons, Inc., New York), In some examples, a protecting group is not removed from the hydrophobic taxane derivative. A "hydroxy protecting group" as used herein denotes a group capable of protecting a free hydroxy group to generate a "protected hydroxyl" which, subsequent to the reaction for which protection is employed, may be removed without disturbing the remainder of the compound. Exemplary hydroxy protecting groups include, but are not limited to, ethers (*e.g.*, allyl, triphenylmethyt (trityl or Tr), benzyl, p-methoxybenzyl (PMB), p- methoxyphenyl (PMP)), acetals (*e.g.*, methoxymethyl (MOM), 3- methoxyethoxymethyl (MEM), tetrahydropyranyl (THP), ethoxy ethyl (EE), methylthiomethyl (MTM), 2- methoxy-2-propyl (MOP), 2-trimethylsilylethoxymethyl (SEM)), esters (*e.g.*, benzoate (Bz), allyl carbonate, 2,2,2-trichloroethyl carbonate (Troc), 2- trimethylsilylethyl carbonate), silyl ethers (*e.g.*, trimethylsilyl (TMS), triethylsilyl (TES), triisopropylsilyl (TI PS), triphenylsilyl (TPS), t- butyldimethylsilyl (TBDMS), t-butyldiphenylsilyt (TBDPS) and the like.
As used herein, "treatment" or "treating" is an approach for obtaining beneficial or desired results including clinical results. For purposes of this invention, beneficial or desired clinical results include, but are not limited to, one or more of the following: decreasing one more symptoms resulting from the disease, diminishing the extent of the disease, stabilizing the disease (*e.g.*, preventing or delaying the worsening of the disease), delay or slowing the progression of the disease, ameliorating the disease state, decreasing the dose of one or more other medications required to treat the disease, increasing the quality of life, and/or prolonging survival (including overall survival and progression free survival. Also encompassed by "treatment" is a reduction of pathological consequence of cancer. The uses of the invention contemplate any one or more of these aspects of treatment.
As used herein, "delaying" the development of cancer means to defer, hinder, slow, retard, stabilize, and/or postpone development of the disease. This delay can be of varying lengths of time, depending on the history of the disease and/or individual being treated. As is evident to one skilled in the art, a sufficient or significant delay can, in effect, encompass prevention, in that the individual does not develop the disease. A method that "delays" development of cancer is a method that reduces probability of disease development in a given time frame and/or reduces the extent of the disease in a given time frame, when compared to not using the method. Such comparisons are typically based on clinical studies, using a statistically significant number of subjects. Cancer development can be detectable using standard methods, such as routine physical exams or x-ray. Development may also refer to disease progression that may be initially undetectable and includes occurrence and onset.
As used herein, an "at risk" individual is an individual who is at risk of developing a condition (*e.g.*, cancer). An individual "at risk" may or may not have detectable disease, and may or may not have displayed a detectable disease prior to the treatment methods described herein. "At risk" denotes that an individual has one or more so-called risk factors, which are measurable parameters that correlate with development of the condition, which are described herein. An individual having one or more of these risk factors has a higher probability of developing the condition than an individual without these risk factor(s).
As used herein, by "pharmaceutically active compound", "therapeutic agent", "drug", and cognates of these terms, is meant a chemical compound that induces a desired effect, *e.g.*, treating, stabilizing, preventing, and/or delaying cancer.
As used herein, the term "additional pharmaceutical agent," and cognates thereof, are intended to refer to active agents other than the taxane derivatives, for example, drugs, which are administered to elicit a therapeutic effect. The pharmaceutical agent(s) may be directed to a therapeutic effect related to the condition that taxane derivative(s) are intended to treat or prevent (*e.g.*, cancer) or, the pharmaceutical agent may be intended to treat or prevent a symptom of the underlying condition (*e.g.*, tumor growth, hemorrhage, ulceration, pain, enlarged lymph nodes, cough, jaundice, swelling, weight loss, cachexia, sweating, anemia, paraneoplastic phenomena, thrombosis, etc.) or to further reduce the appearance or severity of side effects of administering taxane derivatives.
As used herein, by "pharmaceutically acceptable" or "pharmacologically compatible" is meant a material that is not biologically or otherwise undesirable, *e.g.*, the material may be incorporated into a pharmaceutical composition administered to a patient without causing any significant undesirable biological effects or interacting in a deleterious manner with any of the other components of the composition in which it is contained. As used herein, the term "pharmaceutically acceptable carrier," and cognates thereof, refers to adjuvants, binders, diluents, etc. known to the skilled artisan that are suitable for administration to an individual (*e.g.*, a mammal or non-mammal). Combinations of two or more carriers are also contemplated in the present invention. The pharmaceutically acceptable carrier(s) and any additional components, as described herein, should be compatible for use in the intended route of administration (*e.g.*, oral, parenteral) for a particular dosage form. Such suitability will be easily recognized by the skilled artisan, particularly in view of the teaching provided herein. Pharmaceutically acceptable carriers or excipients have preferably met the required standards of toxicological and manufacturing testing and/or are included on the Inactive Ingredient Guide prepared by the U.S. Food and Drug administration.
The terms, "pharmaceutically effective amount," "therapeutically effective amount," "effective amount," and cognates of these terms, as used herein refer to an amount that results in a desired pharmacological and/or physiological effect for a specified condition (*e.g.*, disease, disorder, etc.) or one or more of its symptoms and/or to completely or partially prevent the occurrence of the condition or symptom thereof and/or may be therapeutic in terms of a partial or complete cure for the condition and/or adverse effect attributable to the condition (*e.g.*, cancer). In reference to conditions described herein (*e.g.*, cancer), a pharmaceutically or therapeutically effective amount may comprise an amount sufficient to, among other things, reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; prevent growth and/or kill existing cancer cells; be cytostatic and/or cytotoxic; restore or maintain vasculostasis or prevention of the compromise or loss or vasculostasis; reduction of tumor burden; reduction of morbidity and/or mortality; and/or relieve to some extent one or more of the symptoms associated with the cancer. The effective amount may extend progression free survival (*e.g.* as measured by Response Evaluation Criteria for Solid Tumors, RECIST, or CA-125 changes), result in an objective response (including a partial response or a complete response), increase overall survival time, and/or improve one or more symptoms of cancer (*e.g.* as assessed by FOSI). In certain embodiments, the pharmaceutically effective amount is sufficient to prevent the condition, as in being administered to an individual prophylactically.
The "pharmaceutically effective amount" or "therapeutically effective amount" may vary depending on the composition being administered, the condition being treated/prevented (*e.g.*, the type of cancer), the severity of the condition being treated or prevented, the age and relative health of the individual, the route and form of administration, the judgment of the attending medical or veterinary practitioner, and other factors appreciated by the skilled artisan in view of the teaching provided herein.
As is understood in the art, an "effective amount" may be in one or more doses, i.e., a single dose or multiple doses may be required to achieve the desired treatment endpoint. An effective amount may be considered in the context of administering one or more therapeutic agents, and a nanoparticle composition (*e.g.*, a composition including a taxane derivative and a carrier protein) may be considered to be given in an effective amount if, in conjunction with one or more other agents, a desirable or beneficial result may be or is achieved.
Unless clearly indicated otherwise, an "individual" as used herein intends a mammal, including but not limited to a primate, human, bovine, horse, feline, canine, and/or rodent.
When used with respect to methods of treatment/prevention and the use of the compounds and nanoparticle compositions thereof described herein, an individual "in need thereof' may be an individual who has been diagnosed with or previously treated for the condition to be treated. With respect to prevention, the individual in need thereof may also be an individual who is at risk for a condition (*e.g.*, a family history of the condition, life-style factors indicative of risk for the condition, etc.).
As used herein, by "combination therapy" is meant a first therapy that includes nanoparticles comprising a hydrophobic taxane derivative and a carrier protein in conjunction with a second therapy (*e.g.*, surgery or an additional therapeutic agent) useful for treating, stabilizing, preventing, and/or delaying cancer. Administration in "conjunction with" another compound includes administration in the same or different composition(s), either sequentially, simultaneously, or continuously. In some embodiments, the combination therapy optionally includes one or more pharmaceutically acceptable carriers or excipients, non-pharmaceutically active compounds, and/or inert substances.
The term "antimicrobial agent" used herein refers to an agent that is capable of inhibiting (*e.g.*, delaying, reducing, slowing, and/or preventing) the growth of one or more microorganisms. Significant microbial growth can be measured or indicated by a number of ways known in the art, such as one or more of the following: (i) microbial growth in a composition that is enough to cause one or more adverse effects to an individual when the composition is administered to the individual; (ii) more than about 10-fold increase in microbial growth over a certain period of time (for example over a 24 hour period) upon extrinsic contamination (*e.g.*, exposure to 10-103 colony forming units at a temperature in the range of 20 to 25 °C). Other indicia of significant microbial growth are described in US 2007/0117744.
"Sugar" as used herein includes, but is not limited to, monosaccharides, disaccharides, polysaccharides, and derivatives or modifications thereof. Suitable sugars for compositions described herein include, for example, mannitol, sucrose, fructose, lactose, maltose, and trehalose.
The term "protein" refers to polypeptide or polymer of amino acids of any length (including full length or fragments), which may be linear or branched, comprise modified amino acids, and/or be interrupted by non-amino acids. The term also encompasses an amino acid polymer that has been modified naturally or by intervention; for example, disulfide bond formation, glycosylation, lipidation, acetylation, phosphorylation, or any other manipulation or modification. Also included within this term are, for example, polypeptides containing one or more derivatives of an amino acid (including, for example, unnatural amino acids, etc.), as well as other modifications known in the art.
"Survival" refers to the patient remaining alive, and includes overall survival as well as progression free survival. "Overall survival" refers to the patient remaining alive for a defined period of time, such as 1 year, 5 years, etc. from the time of diagnosis or treatment. "Progression free survival" refers to the patient remaining alive, without the cancer progressing or getting worse. By "prolonging survival" is meant increasing overall or progression free survival in a treated patient relative to an untreated patient (*e.g.* relative to a patient not treated with a taxane nanoparticle composition).
As used herein, reference to "not" a value or parameter generally means and describes "other than" a value or parameter. For example, if a taxane is not administered, it means an agent other than a taxane is administered.
Reference to "about" a value or parameter herein includes (and describes) variations that are directed to that value or parameter per se. For example, description referring to "about X" includes description of "X".
As used herein and in the appended claims, the singular forms "a," "or," and "the" include plural referents unless the context clearly dictates otherwise. It is understood that aspect and variations of the invention described herein include "consisting" and/or "consisting essentially of' aspects and variations.

Unless defined otherwise or clearly indicated by context, all technical and scientific terms used herein have the same meaning as commonly understood by one of ordinary skill in the art to which this invention belongs.

### Nanoparticle compositions

Disclosed herein are compositions comprising nanoparticles, wherein the nanoparticles comprise a drug or hydrophobic drug derivative (*e.g.*, a hydrophobic taxane derivative such as any one of compounds 1, 2, 3-23 and any compound of Formula I, II, III, IV, V, or VI) and a carrier protein (such as albumin, for example human serum albumin).

In some examples, the composition comprising nanoparticles, wherein the nanoparticles comprise a drug and a carrier protein (such as albumin, for example human serum albumin). In some examples, the drug is a taxane. In some examples, the drug is not a taxane. In some examples, the drug is paclitaxel. In some examples, the drug is not paclitaxel. In some examples, the drug is docetaxel. In some examples, the drug is not docetaxel.

In some examples, the composition comprising nanoparticles, wherein the nanoparticles comprise a hydrophobic drug derivative (*e.g.*, a hydrophobic taxane derivative such as any one of compounds 1, 2, 3-23 and any compound of Formula I, II, III, IV, V, or VI) and a carrier protein (such as albumin, for example human serum albumin). In some examples, the hydrophobic drug derivative is a hydrophobic taxane derivative. In some examples, the hydrophobic drug derivative is not a hydrophobic taxane derivative. In some examples, the hydrophobic drug derivative is a hydrophobic paclitaxel derivative. In some examples, the hydrophobic drug derivative is not a hydrophobic paclitaxel derivative. In some examples, the hydrophobic drug derivative is a hydrophobic docetaxel derivative. In some examples, the hydrophobic drug derivative is not a hydrophobic docetaxel derivative. In some examples, the hydrophobic drug derivative is not compound (**1**) described herein. In some examples, the hydrophobic drug derivative is not compound (**2**) described herein.

In some embodiments, there is provided a composition comprising nanoparticles, wherein the nanoparticles comprise a hydrophobic drug derivative and a carrier protein (i.e., albumin, for example human serum albumin), and wherein the hydrophobic drug derivative has improved binding to albumin (as compared to the unmodified drug). In some embodiments, the composition is a pharmaceutical composition.

In some examples, there is a composition comprising nanoparticles, wherein the nanoparticles comprise a hydrophobic drug derivative (*e.g.*, a hydrophobic taxane derivative such as any one of compounds 1, 2, 3-23 and any compound of Formula I, II, III, IV, V, or VI) and a carrier protein (such as albumin, for example human serum albumin), and wherein the composition shows improved therapeutic efficacy compared to drug (*e.g.*, taxane). In some examples, there is a composition comprising nanoparticles, wherein the nanoparticles comprise a hydrophobic drug derivative (*e.g.*, hydrophobic taxane derivative) and a carrier protein (such as albumin, for example human serum albumin), and wherein the hydrophobic drug derivative is a prodrug of the drug (*e.g.*, taxane). In some examples, the hydrophobic drug derivative is not a hydrophobic taxane derivative. In some examples, the hydrophobic drug derivative is not a hydrophobic paclitaxel derivative. In some examples, the hydrophobic drug derivative is not a hydrophobic docetaxel derivative. In some examples, the hydrophobic drug derivative is not compound (**1**) described herein. In some examples, the hydrophobic drug derivative is not compound (**2**) described herein.

In some examples, there is a composition comprising nanoparticles, wherein the nanoparticles comprise a hydrophobic taxane derivative of paclitaxel and a carrier protein (such as albumin, for example human serum albumin). In some examples, there is a composition comprising nanoparticles, wherein the nanoparticles comprise a hydrophobic taxane derivative of docetaxel and a carrier protein (such as albumin, for example human serum albumin). In some examples, there is a composition comprising nanoparticles, wherein the nanoparticles comprise a hydrophobic drug derivative and a carrier protein (such as albumin, for example human serum albumin), wherein the hydrophobic drug derivative is not a hydrophobic taxane derivative (*e.g.*, not a hydrophobic paclitaxel derivative and/or not a hydrophobic docetaxel derivative).

In some examples, there is a composition comprising nanoparticles, wherein the nanoparticles comprise a hydrophobic taxane derivative and a carrier protein, wherein the hydrophobic taxane derivative has a hydrophobic group attached to the 2'-hydroxyl position of the corresponding taxane. In some examples, there is a composition comprising nanoparticles, wherein the nanoparticles comprise a hydrophobic taxane derivative and a carrier protein, wherein the hydrophobic taxane derivative has an acyl group attached to the 2'-hydroxyl position of the corresponding taxane.

In some examples, there is a composition comprising nanoparticles, wherein the nanoparticles comprise a compound of formula I and a carrier protein. In some examples, there is a composition comprising nanoparticles, wherein the nanoparticles comprise a compound of formula II and a carrier protein. In some examples, there is a composition comprising nanoparticles, wherein the nanoparticles comprise a compound of formula III and a carrier protein. In some examples, there is a composition comprising nanoparticles, wherein the nanoparticles comprise a compound of formula IV and a carrier protein. In some examples, there is provided a composition comprising nanoparticles, wherein the nanoparticles comprise a compound of formula V and a carrier protein. In some examples, there is a composition comprising nanoparticles, wherein the nanoparticles comprise a compound of formula VI and a carrier protein.

In some examples, there is a composition comprising nanoparticles, wherein the nanoparticles comprise a compound selected from compounds 1-23 and a carrier protein. The present invention provides a composition comprising nanoparticles, wherein the nanoparticles comprise compound 2 coated with albumin as claimed.

In some examples, there is a composition comprising nanoparticles, wherein the nanoparticles do not comprise albumin-bound paclitaxel. In some examples, the composition comprises nanoparticles, wherein the nanoparticles do not comprise albumin-bound compound 2. In some examples, the composition comprises nanoparticles, wherein the nanoparticles do not comprise albumin-bound docetaxel. In some examples, the composition comprises nanoparticles, wherein the nanoparticles do not comprise albumin-bound paclitaxel.

In some examples, the nanoparticles comprise a drug or hydrophobic drug derivative (*e.g.*, a hydrophobic taxane derivative such as any one of compounds 1, 2, 3-23 and any compound of Formula I, II, III, IV, V, or VI) coated with a carrier protein, such as albumin (*e.g.*, human serum albumin).

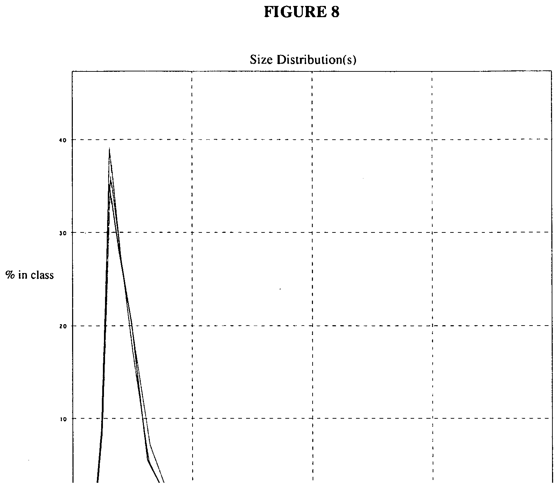

The nanoparticles described herein may have significantly different (*e.g.*, smaller) diameter compared to a nanoparticle composition comprising a drug *(e.g.,* taxane) which is not substituted with a hydrophobic group (see Figure 8). By altering the hydrophobic nature of the drug, the nanoparticle size may be attenuated resulting in improved and/or desired therapeutic effects. Nanoparticles typically have an average diameter (*e.g.*, in dry form) of no greater than about 1000 nanometers (nm), such as no greater than about any one of 900 nm, 800 nm, 700 nm, 600 nm, 500 nm, 400 nm, 300 nm, 200 nm, or 100 nm. In some embodiments, the average diameter of the particles is no greater than about 200 nm. In some embodiments, the average diameter of the particles is between about 20 to about 400 nm. In some embodiments, the average diameter of the particles is between about 40 to about 200 nm. In some embodiments, the particles are sterile-filterable. In some embodiments, the nanoparticles in the composition provided herein have an average diameter of no greater than about 150 nm, including for example no greater than about any one of 100, 90, 80, 70, 60 or 50 nm. The smaller particle size may be beneficial in aiding transport, as described below. In some embodiments, at least about 50% (for example at least any one of 60%, 70%, 80%, 90%, 95%, or 99%) of all the nanoparticles in the composition have a diameter of no greater than about 150 nm, including for example no greater than about any one of 100, 90, 80, 70, or 60 nm. In some embodiments, at least about 50% (for example at least about any one of 60%, 70%, 80%, 90%, 95%, or 99%) of all the nanoparticles in the composition fall within the range of 20-150 nm, including for example about any one of 30-140 nm, 40-130 nm, 50-120 nm, and 60-100 nm. The nanoparticles described herein may be of any shape (*e.g.*, a spherical or non-spherical shape). In some embodiments, the average diameter of the nanoparticles in blood circulation is no greater than about 1000 nanometers (nm), such as no greater than about any one of 900 nm, 800 nm, 700 nm, 600 nm, 500 nm, 400 nm, 300 nm, 200 nm, or 100 nm at a blood concentration of about any one of 25, 50, 75, 100, 125, 150, 175, 200, 250, 300, 350, or 400 ug/mL. In some embodiments, at least about 50% (for example at least any one of 60%, 70%, 80%, 90%, 95%, or 99%) of all the nanoparticles *in vivo* have a diameter of no greater than about 150 nm, including for example no greater than any one of 100, 90, 80, 70, or 60 nm. In some embodiments, the average diameter of the nanoparticles in the blood is between about any one of 5 nm and 80 nm, 10 nm and 70 nm, 20 nm and 60 nm, 30 and 50 nm, or about 45 nm at a blood concentration of between about any one of 10 ug/mL and 300 ug/mL, 25 ug/mL and 150 ug/mL, or 50 ug/mL and 100 ug/mL.

In some embodiments, the carrier protein has sulfhydral groups that can form disulfide bonds. For the purpose of the invention, at least about 5% (including for example at least about any one of 10%, 15%, or 20%) of the carrier protein in the nanoparticle portion of the composition are crosslinked (for example, crosslinked by S-S).

In some embodiments, the composition comprises the drug in both nanoparticle forms and non-nanoparticle forms, wherein more than about any one of 50%, 60%, 70%, 80%, 90%, 95%, or 99% of total hydrophobic taxane derivative is in nanoparticle forms. In some embodiments, the hydrophobic drug derivative constitutes more than about any one of 50%, 60%, 70%, 80%, 90%, 95%, or 99% of the nanoparticles by weight. In some embodiments, the nanoparticles are substantially free of polymeric core materials. In some embodiments, the hydrophobic drug derivative in the nanoparticles is amorphous. In some embodiments, the derivative used for making the nanoparticle compositions is in anhydrous form. In some embodiments, carrier protein (i.e., albumin) to hydrophobic taxane derivative weight ratio in the nanoparticle composition is about any one of 18:1 or less, 15:1 or less, 14:1 or less, 13:1 or less, 12:1 or less, 11:1 or less, 10:1 or less, 9:1 or less, 8:1 or less, 7.5:1 or less, 7:1 or less, 6:1 or less, 5:1 or less, 4:1 or less, or 3:1 or less. In some embodiments, the weight ratio of carrier protein (i.e., albumin) and hydrophobic drug derivative in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of carrier protein and hydrophobic taxane derivative in the nanoparticle portion of the composition is about any one of 1:2, 1:3, 1:4, 1:5, 1:10, 1:15, or less.

The nanoparticles described herein may be present in a dry formulation (*e.g.*, lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like. In some embodiments, the composition comprises a stable aqueous suspension of particles (i.e., nanoparticles) comprising the drug and carrier protein (i.e., particles of hydrophobic drug derivative coated with albumin).

In some embodiments, the composition is substantially free (such as free) of surfactants (such as Cremophor® Tween 80, or other organic solvents used for the administration of taxanes).

The nanoparticle compositions described herein may allow enhanced transport and/or binding of hydrophobic drug derivative (*e.g.*, hydrophobic taxane derivative) and/or a metabolite of the hydrophobic drug derivative to a cell (*e.g.*, a tumor cell). Tumor cells exhibit an enhanced uptake of proteins including, for example, albumin and transferrin, as compared to normal cells. Since tumor cells are dividing at a rapid rate, they require additional nutrient sources compared to normal cells. Tumor studies of the inventive pharmaceutical compositions containing paclitaxel and human serum albumin showed high uptake of albumin-paclitaxel into tumors. This has been found to be due to the previously unrecognized phenomenon of the albumin-drug transport by glycoprotein 60 ("gp60") receptors, which are specific for albumin.

In some embodiments, the nanoparticle composition comprises the drug and a carrier protein (i.e., albumin) capable of binding the gp60 receptor. In another embodiment, the nanoparticle composition comprises drug and a carrier protein i.e., albumin) capable of binding the SPARC receptor.

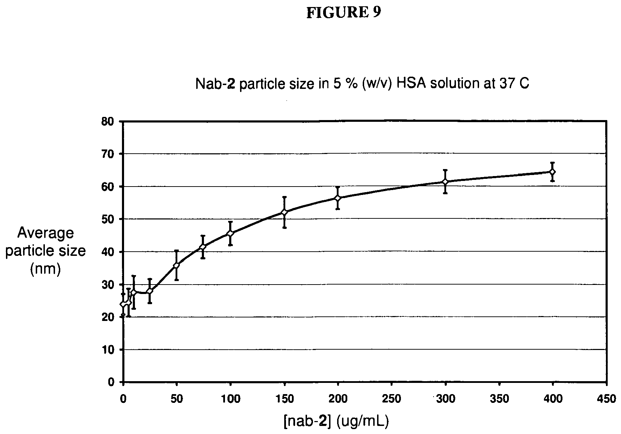

In some embodiments, nanoparticle compositions comprising the hydrophobic drug derivative have a different dissolution profile when compared to that of its corresponding non-derivatized drug (docetaxel) which can result in significant advantages. For example, certain nanoparticles containing the hydrophobic taxane derivatives have been shown to have strikingly lower dissolution when compared to their non-derivatized counterparts (see Example 21; Tables 9 and 10; and Figures 9-11). Decreased dissolution may keep the nanoparticles intact for an extended time during circulation. Accordingly, in one embodiment, the nanoparticle composition comprises the hydrophobic drug derivative VI) and the carrier protein (i.e., albumin) wherein the nanoparticle has a decreased aqueous dissolution rate (including a substantially decreased dissolution rate) compared to a nanoparticle composition comprising docetaxel. In some of these embodiments, aqueous dissolution of the nanoparticle composition comprising the hydrophobic drug derivative is decreased by greater than any one of about 2-fold, or 3-fold, or 5-, 7-, 10-, 12-, 15-, 17-, 20-, 25-, 30-, 35-, 40-, 50-, 75-, 100-, 200-, 500-, or 1000-fold when compared to a nanoparticle composition comprising docetaxel . In some embodiments, the nanoparticles have an average particle size of about any one of 10 nm to 100 nm, 20 to 75 nm, 15 to 50 nm, or more than about any one of 20 nm, 30 nm, 40 nm, 50 nm at any one of about 5, 10, 25, or 50 ug/mL, in a dissolution study in 5% HSA at 37 °C as measured by Dynamic light scattering using a Malvern Zetasizer. In some embodiments, the nanoparticles have an average particle size of about 20 nm to 75 nm, or more than about 30 nm at any one of about 5, 50, 75, or 100 ug/mL, in a dissolution study in 5% HSA at 37 °C. In some embodiments, the nanoparticles exhibit the following dissolution profile when measured in 5% HSA at 37 °C as measured by Dynamic light scattering using a Malvern Zetasizer: (1) a) about 40 nm to 75 nm or more than about 50 nm at 200 ug/mL; b) about 30 nm to 60 nm or more than about 40 nm at 100 ug/mL; and c) about 10 nm to 40 nm or more than about 20 nm at 10 ug/mL; or (2) a) about 50 nm to 100 nm or more than about 60 nm at about 400 ug/mL; b) about 40 nm to 75 nm or more than about 50 nm at 200 ug/mL; c) about 30 nm to 60 nm or more than about 40 nm at about 100 ug/mL; d) about 10 nm to 40 nm or more than about 20 nm at 10 ug/mL; and e) about 10 nm to 40 nm or more than about 20 nm at about 5 ug/mL. In some embodiments, the nanoparticles exhibit one or more of the following dissolution profile when measured in 5% HSA at 37 °C as measured by Dynamic light scattering using a Malvern Zetasizer: a) about 40 nm to 75 nm or more than about 50 nm at 200 ug/mL; b) about 30 nm to 60 nm or more than about 40 nm at 100 ug/mL; or c) about 10 nm to 40 nm or more than about 20 nm at 10 ug/mL. In some embodiments, the nanoparticles exhibit one or more of the following dissolution profile when measured in 5% HSA at 37 °C as measured by Dynamic light scattering using a Malvern Zetasizer: a) about 50 nm to 100 nm or more than about 60 nm at about 400 ug/mL; b) about 40 nm to 75 nm or more than about 50 nm at 200 ug/mL; c) about 30 nm to 60 nm or more than about 40 nm at about 100 ug/mL; d) about 10 nm to 40 nm or more than about 20 nm at 10 ug/mL; or e) about 10 nm to 40 nm or more than about 20 nm at about 5 ug/mL. In some embodiments, the nanoparticles exhibit a dissolution profile of Table 9 when measured in 5% HSA at 37 °C by Dynamic light scattering using a Malvern Zetasizer. In some embodiments, the EC50 (i.e., the half point) of the dissolution profile of the nanoparticle composition is lower than any one of about 200 ug/mL, 150 ug/mL, 120 mg/mL, 100 ug/mL, or 50 ug/mL when measured in 5% HSA at 37 °C by Dynamic light scattering using a Malvern Zetasizer. In some embodiments, the EC50 of the dissolution profile of the nanoparticle composition when measured in 5% HSA at 37 °C is less than any one of about 75%, 50%, 25%, 10%, or 5% of the EC50 for the unmodified drug (e.g., taxane) in the same nanoparticle formulation. In some embodiments, the E90 (i.e., the 90 dissolution point)of the dissolution profile of the nanoparticle composition is lower than any one of about 100 ug/mL, 75 ug/mL, 50 ug/mL, 30 ug/mL, 20 ug/mL, 15 ug/mL, or 10 ug/mL when measured in 5% HSA at 37 °C by Dynamic light scattering using a Malvern Zetasizer. In some embodiments, the nanoparticles are capable of maintaining an average diameter of about 30 nm to about 50 nm for at least about 5 minutes, 10 minutes, or 1 hour when administered intravenously.

The significantly decreased particle size and dissolution of nanoparticles comprising a hydrophobic drug derivative (*e.g.*, hydrophobic taxane derivative) as described above may allow intact nanoparticle to enter the caveolae for endothelial transport into tumor cells (the opening of which is roughly 30-50 nm and internal diameter of 100 nm; see Westermann et. al. Histochem Cell Biol (1999) 111:71-81). Accordingly, the transport of nanoparticles comprising a hydrophobic taxane derivative may be more efficient than the transport of nanoparticles comprising a drug (*e.g.*, taxane) which is not substituted with a hydrophobic group.

In some embodiments, the nanoparticles containing the hydrophobic drug derivative have improved physical and/or chemical stability compared to nanoparticles containing docetaxel. In some embodiments, the nanoparticle composition comprises the drug and the carrier protein (i.e., albumin) wherein the nanoparticle is in a substantially pure form (for example no more than about 15% or no more than about 10% or no more than about 5% or no more than about 3% or no more than about 1% of the total amount of composition as impurity and/or in a different form) after storage of any one of 5, 10, 30, 60, 90, 120, 180, 270, 360 days, or any one of 2, 3, 4, 5, 6, 7, 8, 9, or 10 years at 4 °C (or 25 °C) and pH of about any one of 6, 7, or 8. In some embodiments, the nanoparticles containing the hydrophobic drug derivative is suitable for infusion into humans after storage of any one of 5, 10, 30, 60, 90, 120, 180, 270, 360 days, or any one of 2, 3, 4, 5, 6, 7, 8, 9, or 10 years at 4 °C (or 25 °C). In some embodiments, the nanoparticles containing the drug are stable without further comprising a stabilizer (*e.g*., citrate).

In some embodiments, the nanoparticle composition has a Cmax in the blood of about 0.05 hour to about 0.3 hour after administration to a primate exhibit. In some embodiments, the nanoparticle composition exhibits break down in blood with terminal half life of about 1 hour to about 5 hours, including for example about 2 hours to about 4 hours, such as about 3 hours to about 3.7 hours, after administration to a primate. In some embodiments, the nanoparticle composition has a metabolite conversion rate for removal of the hydrophobic group from the hydrophobic taxane derivative from between any one of about 2% and 20%, about 3% and 10%, or about 4% and 7% after administration to a primate. In some embodiments, the primate is a monkey. In some embodiments, the primate is a human.

### Nanoparticle Composition Drugs

Some nanoparticle compositions described herein comprise a drug and/or a drug substituted with a hydrophobic group (hydrophobic drug derivative). Non-limiting examples of drugs contemplated for use in any of the nanoparticle compositions described herein and/or drugs contemplated for modification to a hydrophobic drug derivative as described herein and used thereof include pharmaceutically active agents, diagnostic agents, agents of nutritional value, and the like.

Examples of pharmaceutically active agents include: analgesics/antipyretics (*e.g.*, aspirin, acetaminophen, ibuprofen, naproxen sodium, buprenorphine hydrochloride, propoxyphene hydrochloride, propoxyphene napsylate, meperidine hydrochloride, hydromorphone hydrochloride, morphine sulfate, oxycodone hydrochloride, codeine phosphate, dihydrocodeine bitartrate, pentazocine hydrochloride, hydrocodone bitartrate, levorphanol tartrate, diflunisal, trolamine salicylate, nalbuphine hydrochloride, mefenamic acid, butorphanol tartrate, choline salicylate, butalbital, phenyltoloxamine citrate, diphenhydramine citrate, methotrimeprazine, cinnamedrine hydrochloride, meprobamate, and the like); anesthetics (*e.g.*, cyclopropane, enflurane, halothane, isoflurane, methoxyflurane, nitrous oxide, propofol, and the like); antiasthmatics (*e.g.*, Azelastine, Ketotifen, Traxanox, Amlexanox, Cromolyn, Ibudilast, Montelukast, Nedocromil, Oxatomide, Pranlukast, Seratrodast, Suplatast Tosylate, Tiaramide, Zafirlukast, Zileuton, Beclomethasone, Budesonide, Dexamethasone, Flunisolide, Trimcinolone Acetonide, and the like); antibiotics (*e.g.*, neomycin, streptomycin, chloramphenicol, cephalosporin, ampicillin, penicillin, tetracycline, and the like); antidepressants (*e.g.*, nefopam, oxypertine, doxepin hydrochloride, amoxapine, trazodone hydrochloride, amitriptyline hydrochloride, maprotiline hydrochloride, phenelzine sulfate, desipramine hydrochloride, nortriptyline hydrochloride, tranylcypromine sulfate, fluoxetine hydrochloride, doxepin hydrochloride, imipramine hydrochloride, imipramine pamoate, nortriptyline, amitriptyline hydrochloride, isocarboxazid, desipramine hydrochloride, trimipramine maleate, protriptyline hydrochloride, and the like); antidiabetics (*e.g.*, biguanides, hormones, sulfonylurea derivatives, and the like); antifungal agents (*e.g.*, griseofulvin, keloconazole, amphotericin B, Nystatin, candicidin, and the like); antihypertensive agents (*e.g.*, propanolol, propafenone, oxyprenolol, Nifedipine, reserpine, trimethaphan camsylate, phenoxybenzamine hydrochloride, pargyline hydrochloride, deserpidine, diazoxide, guanethidine monosulfate, minoxidil, rescinnamine, sodium nitroprusside, rauwolfia serpentina, alseroxylon, phentolamine mesylate, reserpine, and the like); anti-inflammatories (*e.g.*, (non-steroidal) indomethacin, naproxen, ibuprofen, ramifenazone, piroxicam, (steroidal) cortisone, dexamethasone, fluazacort, hydrocortisone, prednisolone, prednisone, and the like); antineoplastics (*e.g.*, adriamycin, cyclophosphamide, actinomycin, bleomycin, duanorubicin, doxorubicin, epirubicin, mitomycin, methotrexate, fluorouracil, carboplatin, carmustine (BCNU), methyl-CCNU, cisplatin, etoposide, interferons, camptothecin and derivatives thereof, phenesterine, Taxol and derivatives thereof, taxotere and derivatives thereof, vinblastine, vincristine, tamoxifen, etoposide, piposulfan, and the like); antianxiety agents (*e.g.*, lorazepam, buspirone hydrochloride, prazepam, chlordiazepoxide hydrochloride, oxazepam, clorazepate dipotassium, diazepam, hydroxyzine pamoate, hydroxyzine hydrochloride, alprazolam, droperidol, halazepam, chlormezanone, dantrolene, and the like); immunosuppressive agents (*e.g.*, cyclosporine, azathioprine, mizoribine, FK506 (tacrolimus), and the like); antimigraine agents (*e.g.*, ergotamine tartrate, propanolol hydrochloride, isometheptene mucate, dichloralphenazone, and the like); sedatives/hypnotics (*e.g.*, barbiturates (*e.g.*, pentobarbital, pentobarbital sodium, secobarbital sodium), benzodiazapines (*e.g.*, flurazepam hydrochloride, triazolam, tomazeparm, midazolam hydrochloride, and the like); antianginal agents (*e.g.*, beta-adrenergic blockers, calcium channel blockers (*e.g.*, nifedipine, diltiazem hydrochloride, and the like), nitrates (*e.g.*, nitroglycerin, isosorbide dinitrate, pentaerythritol tetranitrate, erythrityl tetranitrate, and the like)); antipsychotic agents (*e.g.*, haloperidol, loxapine succinate, loxapine hydrochloride, thioridazine, thioridazine hydrochloride, thiothixene, fluphenazine hydrochloride, fluphenazine decanoate, fluphenazine enanthate, trifluoperazine hydrochloride, chlorpromazine hydrochloride, perphenazine, lithium citrate, prochlorperazine, and the like); antimanic agents (*e.g.*, lithium carbonate); antiarrhythmics (*e.g.*, bretylium tosylate, esmolol hydrochloride, verapamil hydrochloride, amiodarone, encainide hydrochloride, digoxin, digitoxin, mexiletine hydrochloride, disopyramide phosphate, procainamide hydrochloride, quinidine sulfate, quinidine gluconate, quinidine polygalacturonate, flecainide acetate, tocainide hydrochloride, lidocaine hydrochloride, and the like) ;antiarthritic agents (*e.g.*, phenylbutazone, sulindac, penicillamine, salsalate, piroxicam, azathioprine, indomethacin, meclofenamate sodium, gold sodium thiomalate, ketoprofen, auranofin, aurothioglucose, tolmetin sodium, and the like); antigout agents (*e.g.*, colchicine, allopurinol, and the like); anticoagulants (*e.g.*, heparin, heparin sodium, warfarin sodium, and the like); thrombolytic agents (*e.g.*, urokinase, streptokinase, altoplase, and the like); antifibrinolytic agents (*e.g.*, aminocaproic acid) ; hemorheologic agents (*e.g.*, pentoxifylline); antiplatelet agents (*e.g.*, aspirin, empirin, ascriptin, and the like); anticonvulsants (*e.g.*, valproic acid, divalproate sodium, phenytoin, phenytoin sodium, clonazepam, primidone, phenobarbitol, phenobarbitol sodium, carbamazepine, amobarbital sodium, methsuximide, metharbital, mephobarbital, mephenytoin, phensuximide, paramethadione, ethotoin, phenacemide, secobarbitol sodium, clorazepate dipotassium, trimethadione, and the like); antiparkinson agents (*e.g.*, ethosuximide, and the like); antihistamines/antipruritics (*e.g.*, hydroxyzine hydrochloride, diphenhydramine hydrochloride, chlorpheniramine maleate, brompheniramine maleate, cyproheptadine hydrochloride, terfenadine, clemastine fumarate, triprolidine hydrochloride, carbinoxamine maleate, diphenylpyraline hydrochloride, phenindamine tartrate, azatadine maleate, tripelennamine hydrochloride, dexchlorpheniramine maleate, methdilazine hydrochloride, trimprazine tartrate and the like); agents useful for calcium regulation (*e.g.*, calcitonin, parathyroid hormone, and the like); antibacterial agents (*e.g.*, amikacin sulfate, aztreonam, chloramphenicol, chloramphenicol palmitate, chloramphenicol sodium succinate, ciprofloxacin hydrochloride, clindamycin hydrochloride, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, colistin sulfate, and the like); antiviral agents (*e.g.*, interferon gamma, zidovudine, amantadine hydrochloride, ribavirin, acyclovir, and the like); antimicrobials (*e.g.*, cephalosporins (*e.g.*, cefazolin sodium, cephradine, cefaclor, cephapirin sodium, ceftizoxime sodium, cefoperazone sodium, cefotetan disodium, cefutoxime azotil, cefotaxime sodium, cefadroxil monohydrate, ceftazidime, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, cefuroxime sodium, and the like), penicillins (*e.g.*, ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G potassium, penicillin G procaine, methicillin sodium, nafcillin sodium, and the like), erythromycins (*e.g.*, erythromycin ethylsuccinate, erythromycin, erythromycin estolate, erythromycin lactobionate, erythromycin siearate, erythromycin ethylsuccinate, and the like), tetracyclines (*e.g.*, tetracycline hydrochloride, doxycycline hyclate, minocycline hydrochloride, and the like), and the like); anti-infectives (*e.g.*, GM-CSF); bronchodialators (*e.g.*, sympathomimetics (*e.g.*, epinephrine hydrochloride, metaproterenol sulfate, terbutaline sulfate, isoetharine, isoetharine mesylate, isoetharine hydrochloride, albuterol sulfate, albuterol, bitolterol, mesylate isoproterenol hydrochloride, terbutaline sulfate, epinephrine bitartrate, metaproterenol sulfate, epinephrine, epinephrine bitartrate), anticholinergic agents (*e.g.*, ipratropium bromide), xanthines (*e.g.*, aminophylline, dyphylline, metaproterenol sulfate, aminophylline), mast cell stabilizers (*e.g.*, cromolyn sodium), inhalant corticosteroids (*e.g.*, flurisolidebeclomethasone dipropionate, beclomethasone dipropionate monohydrate), salbutamol, beclomethasone dipropionate (BDP), ipratropium bromide, budesonide, ketotifen, salmeterol, xinafoate, terbutaline sulfate, triamcinolone, theophylline, nedocromil sodium, metaproterenol sulfate, albuterol, flunisolide, and the like); hormones (*e.g.*, androgens (*e.g.*, danazol, testosterone cypionate, fluoxymesterone, ethyltostosterone, testosterone enanihate, methyltestosterone, fluoxymesterone, testosterone cypionate), estrogens (*e.g.*, estradiol, estropipate, conjugated estrogens), progestins (*e.g.*, methoxyprogesterone acetate, norethindrone acetate), corticosteroids (*e.g.*, triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate methylprednisolone sodium succinate, hydrocortisone sodium succinate, methylprednisolone sodium succinate, triamcinolone hexacatonide, hydrocortisone, hydrocortisone cypionate, prednisolone, fluorocortisone acetate, paramethasone acetate, prednisolone tebulate, prednisolone acetate, prednisolone sodium phosphate, hydrocortisone sodium succinate, and the like), thyroid hormones (*e.g.*, levothyroxine sodium) and the like), and the like; hypoglycemic agents (*e.g.*, human insulin, purified beef insulin, purified pork insulin, glyburide, chlorpropamide, glipizide, tolbutamide, tolazamide, and the like); hypolipidemic agents (*e.g.*, clofibrate, dextrothyroxine sodium, probucol, lovastatin, niacin, and the like); proteins (*e.g.*, DNase, alginase, superoxide dismutase, lipase, and the like); nucleic acids (*e.g.*, sense or anti-sense nucleic acids encoding any therapeutically useful protein, including any of the proteins described herein, and the like); agents useful for erythropoiesis stimulation (*e.g.*, erythropoietin); antiulcer/antireflux agents (*e.g.*, famotidine, cimetidine, ranitidine hydrochloride, and the like); antinauseants/antiemetics (*e.g.*, meclizine hydrochloride, nabilone, prochlorperazine, dimenhydrinate, promethazine hydrochloride, thiethylperazine, scopolamine, and the like); oil-soluble vitamins (*e.g.*, vitamins A, D, E, K, and the like); as well as other drugs such as mitotane, visadine, halonitrosoureas, anthrocyclines, ellipticine, and the like.

Examples of diagnostic agents contemplated for use include ultrasound contrast agents, radiocontrast agents (*e.g*., iodo-octanes, halocarbons, renografin, and the like), magnetic contrast agents (*e.g*., fluorocarbons, lipid soluble paramagnetic compounds, and the like), as well as other diagnostic agents which cannot readily be delivered without some physical and/or chemical modification to accommodate the substantially water insoluble nature thereof.

In some examples, the compositions described herein comprise poorly water soluble drugs and/or hydrophobic drug derivatives. For example, the solubility in water of the poorly water soluble drug at about 20-25 °C may be less than about 10 mg/ml, including for example less than about any of 5, 2, 1, 0.5, 0.2, 0.1, 0.05, 0.02, or 0.01 mg/ml. Poorly water soluble drugs described herein can be, for example, anticancer or antineoplastic agents, antimicrotubule agents, immunosuppressive agents, anesthetics, hormones, agents for use in cardiovascular disorders, antiarrhythmics, antibiotics, antifungals, antihypertensives, antiasthmatics, antiinflammatory agents, anti-arthritic agents, vasoactive agents, analgesics/antipyretics, antidepressants, antidiabetics, antifungal agents, anti-inflammatories, antianxiety agents, immunosuppressive agents, antimigraine agents, sedatives, antianginal agents, antipsychotic agents, antimanic agents, antiarthritic agents, antigout agents, anticoagulants, thrombolytic agents, antifibrinolytic agents, hemorheologic agents, antiplatelet agents, anticonvulsants, antiparkinson agents, antihistamines/antipruritics, agents useful for calcium regulation, antiviral agents, antimicrobials, anti-infectives, bronchodialators, hormones, hypoglycemic agents, hypolipidemic agents, antiulcer/antireflux agents, antinauseants/antiemetics, and oil-soluble vitamins (*e.g.*, vitamins A, D, E, K, and the like).

In some examples, the poorly water soluble drug is an antineoplastic agent. In some examples, the poorly water soluble pharmaceutical agent is a chemotherapeutic agent.

Suitable poorly water soluble drugs include, but are not limited to, taxanes (such as paclitaxel, docetaxel, ortataxel and other taxanes), epothilones, camptothecins, colchicines, geladanamycins, amiodarones, thyroid hormones, amphotericin, corticosteroids, propofol, melatonin, cyclosporine, rapamycin (sirolimus) and derivatives, tacrolimus, mycophenolic acids, ifosfamide, vinorelbine, vancomycin, gemcitabine, SU5416, thiotepa, bleomycin, diagnostic radiocontrast agents, and derivatives thereof. Other poorly water soluble pharmaceutical agents that are useful in the inventive compositions are described in, for example, U.S. Pat. Nos. 5,916,596, 6,096,331, 6,749,868, and 6,537,539. Additional examples of poorly water soluble pharmaceutical agents include those compounds which are poorly water soluble and which are listed in the "Therapeutic Category and Biological Activity Index" of The Merck Index (12th Edition, 1996).

In some examples, the poorly water soluble drug is any of (and in some embodiments selected from the group consisting of) paclitaxel, docetaxel, ortataxel or other taxane or taxane analog, 17-allyl amino geldanamycin (17-AAG), 18-derivatized geldanamycin, camptothecin, propofol, amiodarone, cyclosporine, epothilone, radicicol, combretastatin, rapamycin, amphotericin, liothyronine, epothilone, colchicine, thiocolchicine and its dimers, thyroid hormone, vasoactive intestinal peptide, corticosteroids, melatonin, tacrolimus, mycophenolic acids, epothilones, radicicols, combretastatins, and analog or derivative thereof. In some examples, the poorly water soluble pharmaceutical agent is any of (and in some embodiments selected from the group consisting of) paclitaxel, docetaxel, ortataxel or other taxanes, geldanamycin, 17-allyl amino geldanamycin, thiocolchicine and its dimers, rapamycin, cyclosporine, epothilone, radicicol, and combretastatin. In some examples, the poorly water soluble pharmaceutical agent is rapamycin. In some examples, the poorly water soluble pharmaceutical agent is 17-AAG. In some examples, the poorly water soluble pharmaceutical agent is a thiocolchicine dimer (such as IDN5404).

In some examples, the poorly water drug is a taxane or derivative thereof, which includes, but is not limited to, paclitaxel, docetaxel and IDN5109 (ortataxel), or a derivative thereof. In some examples, the composition comprises a non-crystalline and/or amorphous taxane (such as paclitaxel or a derivative thereof). In some examples, the composition is prepared by using an anhydrous taxane (such as anhydrous docetaxel or a derivative thereof). Anhydrous docetaxel has been shown to produce more stable formulation than can be made with a hydrated docetaxel such as docetaxel trihydrate or hemi-hydrate.

In some examples described herein, the drug is a taxane. In some examples described herein, the drug is paclitaxel:

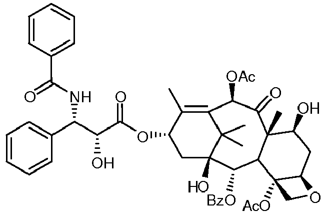

In some examples described herein, the drug is docetaxel:

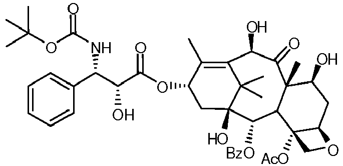

In some examples described herein, the drug is not a taxane. In some examples, the drug is not paclitaxel. In some examples, the drug is not docetaxel.

### Hydrophobic Drug Derivatives

The nanoparticle compositions described herein may comprise a hydrophobic drug derivative. Contemplated hydrophobic drug derivatives include and drug described (*e.g.*, any drug described above in the section "Nanoparticle Composition Drugs", such as poorly water soluble drugs and/or pharmaceutically active agents) wherein the drug is modified with one or more hydrophobic groups as described herein. Also embraced with any nanoparticle composition described herein is any one or more hydrophobic drugs described in WO2006/089207 (filed February 18, 2005), such as any one of compounds 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, and/or 39 described therein.

For example, in some examples, the hydrophobic drug derivative is of the formula: 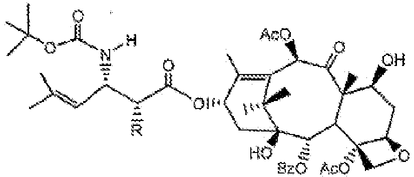 wherein R is OH, OCOPh or OCO(CH₂)₄CH₃.

In some examples, the hydrophobic drug derivative is of the formula: 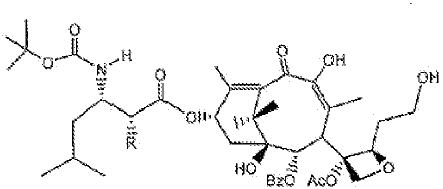 wherein R is OH, OCOPh or OCO(CH₂)₄CH₃.

In some examples, the hydrophobic drug derivative is of the formula: 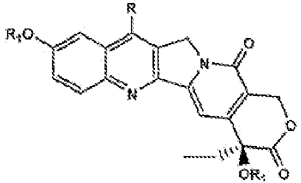 Wherein,
R = H; R₁ = H
R = Et; R₁ = H
R = H; R₁ = COCH₂CH₃
R = H; R₁ = COCH₂CH₂)CH₃
R = H; R₁ = COCH(CH₃)₂
R = H; R₁ = COCH₂CH₂CH₂CH₂CH₃
R = H; R₁ = COCH₂NH-COOtBu
R = H; R₁ = COCH₂OMe
R = H; R₁ = COCH₂NH₂
R = H; R₁ = COPh
R = Et; R1 = COCH₂CH₃
R = H; R₁ = CO(CH₂)₄CH₃
R = Et; R₁ = CO(CH₂)₈CH₃
R = Et; R₁ = CO(CH₂CH₃
R = Et, R₁ = CO(CH₂)₁₀CH₃
R = Et; R₁ = CO(CH₂)₁₆CH₃
R = Et; R₁ = CO(CH₂)₃CH(CH₃)CH₂CH₃
R = H; R₁ = CO(CH₂)₁₄CH₃

In some examples, the hydrophobic drug derivative is of the formula: 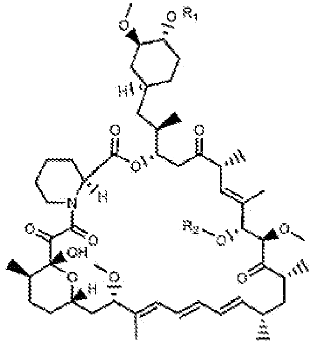 wherein, R₁ is H and R₂ is H or COPh.

In some examples, the hydrophobic drug derivative is of the formula: 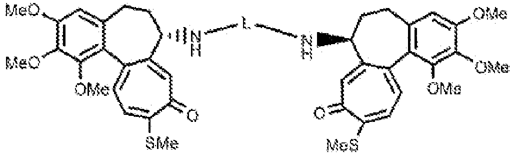 wherein L is:
(a) 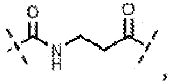
(b) 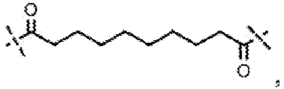
(c) 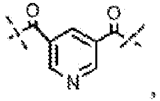 or
(d)

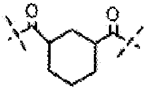

In some examples, the hydrophobic drug derivative is of the formula: 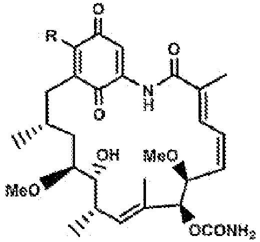 wherein, R is OMe, NHCHCH₂, NH(CH₂)₆CH₃, N(CH₂)₅, NCH₂CHCH₃, or NHCH(CH₃)(CH₂)₄CH₃.

In some examples, the hydrophobic drug derivative is of the formula: 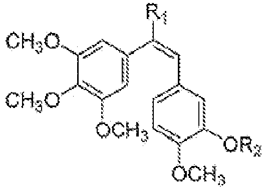
(a): R₁ is H; R₂ is H
(b): R₁ is CO₂H; R₂ is H
(c): R₁ is CO₂H; R₂ is COCH₃
(d): R₁ is H; R₂ is COCH₃
(e): R₁ is H; R₂ is CO(CH₂)₄CH₃
(f): R₁ is H; R₂ is CO(CH₂)₁₀CH₃
(g): R₁ is H; R₂ is CO(CH₂)₆(CH₂CH₂=CH)₂(CH₂)₄CH₃
(h); R₁ is H; R₂ is CO(CH₂)₇CH=CH(CH₂)₇CH₃

In some examples, the hydrophobic drug derivative is a prodrug of the drug. In some examples, the prodrug is an ester (*e.g.*, a hydrophobic ester). In some embodiments, the ester is an alkyl ester (*e.g.*, C₂-C₁₀ ester, such as a hexanoate ester or an acetate ester) or an aryl ester (*e.g.*, a benzoate ester). In some examples, the hydrophobic drug derivative is a prodrug of the drug and is capable of being converted to the drug by greater than about any one of 1, 2, 3, 4, 5, 8, 10, 12, 15, 18, 20, 25, or 30% as measured by the methods known in the art and/or described in the examples section herein (*e.g.*, conversion by human liver microsome).

In some examples, the hydrophobic drug derivative is not a hydrophobic taxane derivative. In some examples, the hydrophobic drug derivative is not a hydrophobic paclitaxel derivative. In some examples, the hydrophobic drug derivative is not a hydrophobic docetaxel derivative. In some examples, the hydrophobic drug derivative is not compound (**1**) described herein. In some examples, the hydrophobic drug derivative is not compound (**2**) described herein.

In some of these examples, the hydrophobic drug derivative contains one or more hydrophobic groups. In some examples, the hydrophobic drug derivative contains multiple hydrophobic groups. In some examples, the hydrophobic drug derivative contains only one hydrophobic group. In some examples, the hydrophobic group is -C(O)R⁶; wherein R⁶ is a substituted or unsubstituted moiety selected from alkyl, alkenyl, alkynyl, cycloalkyl, cycloalkylalkyl, aryl, heteroaryl, aralkyl, and heteraralkyl. In some examples, R⁶ is independently a substituted or unsubstituted moiety selected from alkyl, alkenyl, cycloalkyl, cycloalkyl-alkyl, aryl, and aralkyl. In some examples, R⁶ is a substituted or unsubstituted moiety selected from alkyl, alkenyl, cycloalkyl, cycloalkyl-alkyl, aryl, and aralkyl. In some examples, R⁶ is a substituted or unsubstituted moiety selected from alkyl, aryl, and aralkyl. In some examples, the alkyl, aryl, and aralkyl groups are unsubstituted. In some examples, R⁶ is an unsubstituted C₁-C₁₅ alkyl or an unsubstituted 6-membered aryl. In some examples, R⁶ is an unsubstituted C₁-C₁₀ alkyl or an unsubstituted phenyl. In some examples, R⁶ is an unsubstituted C₁-C₁₀ alkyl (*e.g.*, C₅ alkyl). In some examples, R⁶ is an unsubstituted phenyl.

Some nanoparticle compositions described herein comprise a hydrophobic taxane derivative (*e.g.*, a hydrophobic paclitaxel derivative or hydrophobic docetaxel derivative). Structural examples of taxanes, including paclitaxel and docetaxel, are shown below with the conventional numbering system as used herein:

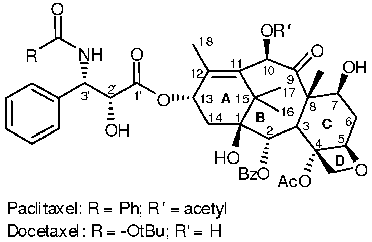

To illustrate an example of the nomenclature used herein, the notation C2' or 2' refers to the carbon atom labeled " 2' " shown above, and the A-ring is made up of the ring formed by the fewest number of ring carbons surrounding the letter A (i.e., the ring formed by C1, C15, C11, C12, C13, and C14). Accordingly, a "2'-hydroxyl group" refers to the hydroxyl moiety attached to the carbon atom labeled " 2' ". The pendant side-chain is the moiety made up of the atoms linked to C13 oxygen atom (*e.g.*, C1', C2', C3', etc.).

In some examples, hydrophobic taxane derivative is a derivative of paclitaxel. In some examples, hydrophobic taxane derivative is a derivative of docetaxel.

In some examples, the hydrophobic taxane derivative is a prodrug of the taxane. In some examples, the prodrug is an ester (*e.g.*, a hydrophobic ester). In some examples, the ester is an alkyl ester (*e.g.,* C₂-C₁₀ ester, such as a hexanoate ester or an acetate ester) or an aryl ester (*e.g.*, a benzoate ester). In some examples, the hydrophobic taxane derivative is a prodrug of the taxane (*e.g.*, docetaxel or paclitaxel) and is capable of being converted to the taxane (*e.g.*, docetaxel or paclitaxel) by greater than about any one of 1, 2, 3, 4, 5, 8, 10, 12, 15, 18, 20, 25, or 30% as measured by the methods known in the art and/or described in the examples section herein (*e.g.*, conversion by human liver microsome).

In some examples, the hydrophobic taxane derivative contains a hydrophobic group attached to an A-ring carbon or to an exocyclic atom which is directly linked to an A-ring carbon. In some examples, the hydrophobic taxane derivative contains a hydrophobic group attached to a B-ring carbon or to an exocyclic atom which is directly linked to a B-ring carbon. In some examples, the hydrophobic taxane derivative contains a hydrophobic group attached to a C-ring carbon or to an exocyclic atom which is directly linked to a C-ring carbon. In some examples, the hydrophobic taxane derivative contains a hydrophobic group attached to the pendant side-chain.

In some of these examples, the hydrophobic taxane derivative contains one or more hydrophobic groups. In some examples, the hydrophobic taxane derivative contains multiple hydrophobic groups. In some examples, the hydrophobic taxane derivative contains only one hydrophobic group. In some examples, the hydrophobic group is -C(O)R⁶; wherein R⁶ is a substituted or unsubstituted moiety selected from alkyl, alkenyl, alkynyl, cycloalkyl, cycloalkylalkyl, aryl, heteroaryl, aralkyl, and heteraralkyl. In some examples, R⁶ is independently a substituted or unsubstituted moiety selected from alkyl, alkenyl, cycloalkyl, cycloalkyl-alkyl, aryl, and aralkyl. In some examples, R⁶ is a substituted or unsubstituted moiety selected from alkyl, alkenyl, cycloalkyl, cycloalkyl-alkyl, aryl, and aralkyl. In some examples, R⁶ is a substituted or unsubstituted moiety selected from alkyl, aryl, and aralkyl. In some examples, the alkyl, aryl, and aralkyl groups are unsubstituted. In some examples, R⁶ is an unsubstituted C₁-C₁₅ alkyl or an unsubstituted 6-membered aryl. In some examples, R⁶ is an unsubstituted C₁-C₁₀ alkyl or an unsubstituted phenyl. In some examples, R⁶ is an unsubstituted C₁-C₁₀ alkyl (*e.g.*, C₅ alkyl). In some examples, R⁶ is an unsubstituted phenyl.

In some of these examples, the hydrophobic taxane derivative is of the formula: 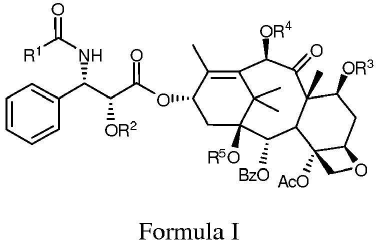 wherein R¹ is phenyl or -OtBu; R², R³, R⁴, and R⁵ are independently H or a hydrophobic group; and wherein at least one of R², R³, R⁴, and R⁵ is not H.

In some examples, the hydrophobic taxane derivative is of the formula: 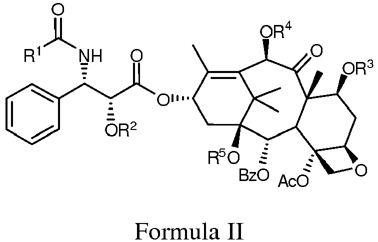 wherein R¹ is a phenyl or -OtBu; R², R³, R⁴, and R⁵ are independently H or -C(O)R⁶; each R⁶ is independently a substituted or unsubstituted moiety selected from alkyl, alkenyl, alkynyl, cycloalkyl, cycloalkyl-alkyl, aryl, heteroaryl, aralkyl, and heteraralkyl; and wherein at least one of R², R³, R⁴, and R⁵ is not H.

In some examples, the hydrophobic taxane derivative is of the formula: 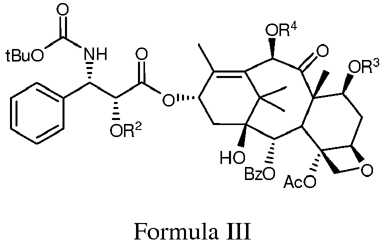 wherein R², R³, and R⁴ are independently H or -C(O)R⁶;each R⁶ is independently a substituted or unsubstituted moiety selected from alkyl, alkenyl, alkynyl, cycloalkyl, cycloalkyl-alkyl, aryl, heteroaryl, aralkyl, and heteraralkyl; and wherein at least one of R², R³, and R⁴ is not H.

In some examples, the hydrophobic taxane derivative is of the formula: 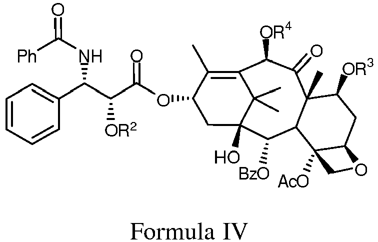 wherein R², R³, and R⁴ are independently H or -C(O)R⁶;each R⁶ is independently a substituted or unsubstituted moiety selected from alkyl, alkenyl, alkynyl, cycloalkyl, cycloalkyl-alkyl, aryl, heteroaryl, aralkyl, and heteraralkyl; and wherein at least one of R², R³, and R⁴ is not H. In some embodiments, when R², R³, and R⁵ are each H, then R⁴ is not an acetyl moiety.

In some examples, the hydrophobic taxane derivative is of the formula: 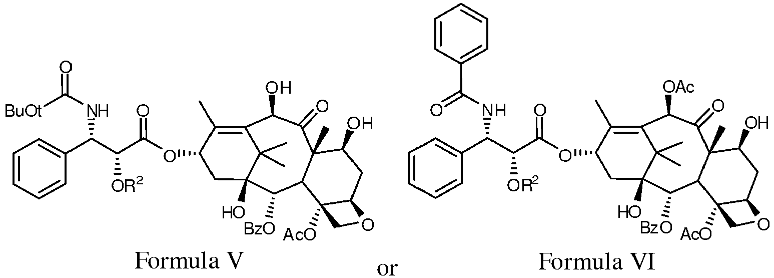 wherein R² is -C(O)R⁶; and R⁶ is independently a substituted or unsubstituted moiety selected from alkyl, alkenyl, alkynyl, cycloalkyl, cycloalkyl-alkyl, aryl, heteroaryl, aralkyl, and heteraralkyl; or a pharmaceutically acceptable salt, isomer, or solvate thereof.

The hydrophobic taxane derivative (2) illustrated in this paragraph is the compound of the invention. The other hydrophobic taxane derivatives, namely (1) and (3) to (23), illustrated in this paragraph are disclosed as reference.

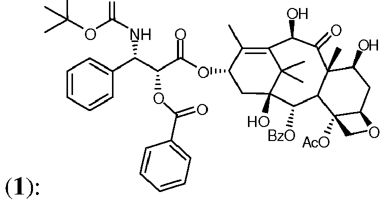

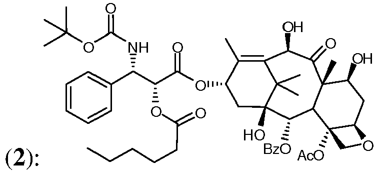

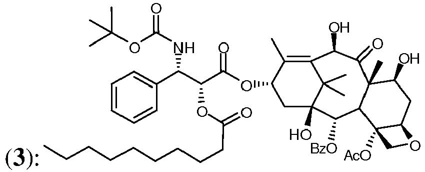

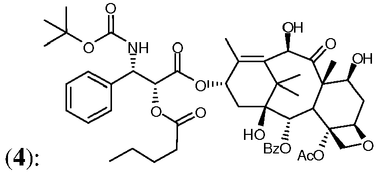

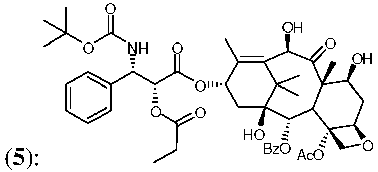

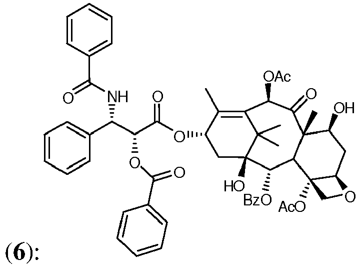

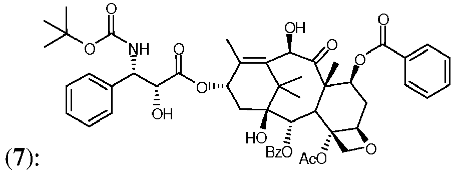

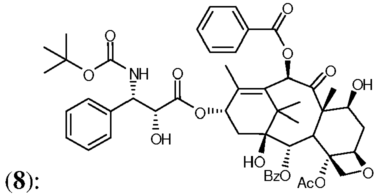

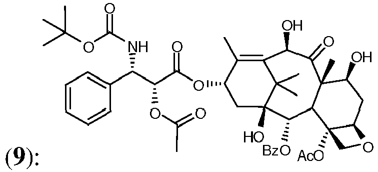

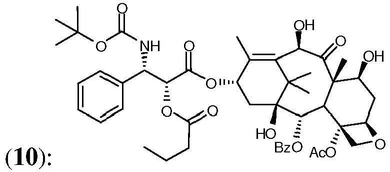

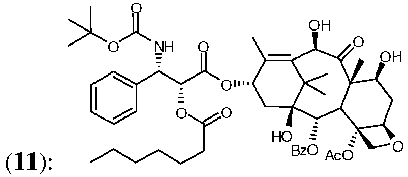

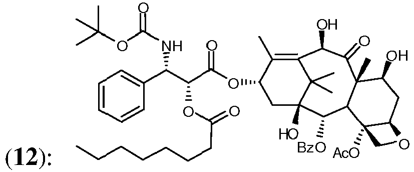

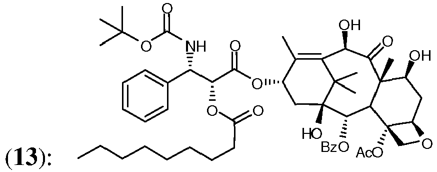

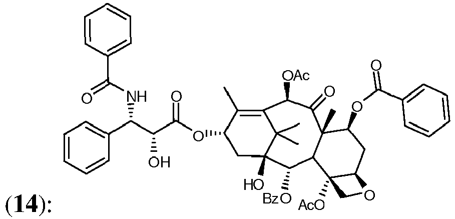

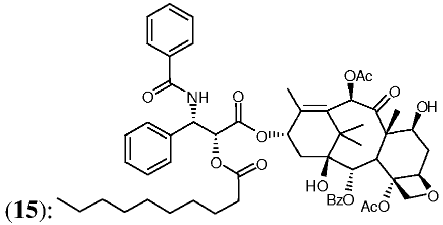

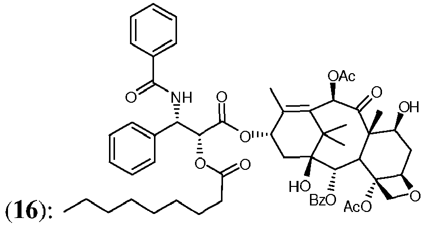

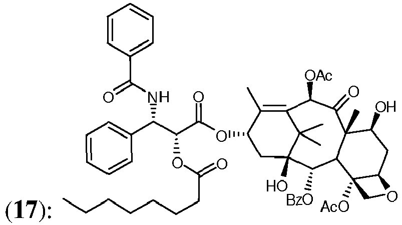

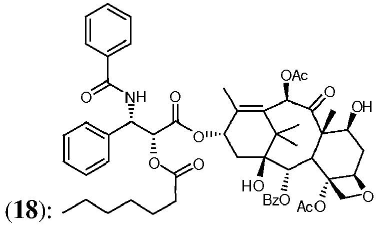

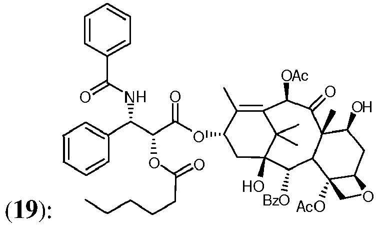

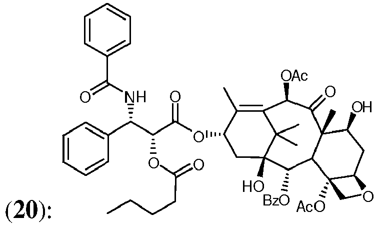

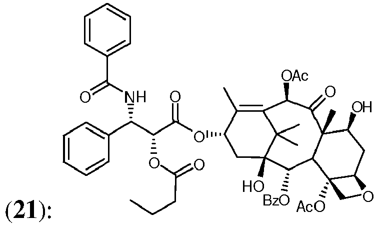

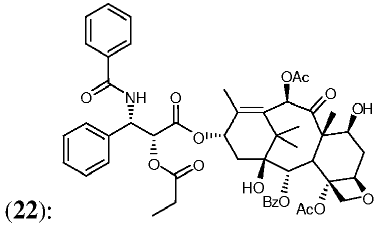

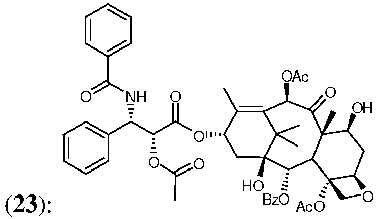

### Carrier proteins

The nanoparticle compositions described herein can utilize suitable naturally occurring or synthetic carrier proteins. Examples of suitable carrier proteins include proteins normally found in blood or plasma, which include, but are not limited to, albumin, immunoglobulin including IgA, lipoproteins, apolipoprotein B, α-acid glycoprotein, β-2-macroglobulin, thyroglobulin, transferin, fibronectin, vitronectin, fibrinogen, factor VII, factor VIII, factor IX, factor X, and the like. In some embodiments, the carrier protein is a non-blood protein, such as casein, α-lactalbumin, or β-lactoglobulin. The carrier proteins may either be natural in origin or synthetically prepared. For the purpose of the invention, the pharmaceutical acceptable carrier comprises albumin, such as human serum albumin (HSA). HSA is a highly soluble globular protein of Mᵣ 65K and consists of 585 amino acids. HSA is the most abundant protein in the plasma and accounts for 70-80% of the colloid osmotic pressure of human plasma. The amino acid sequence of HSA contains a total of 17 disulphide bridges, one free thiol (Cys 34), and a single tryptophan (Trp 214). Other albumins are contemplated, such as bovine serum albumin. Use of such non-human albumins could be appropriate, for example, in the context of use of these compositions in non-human mammals, such as the veterinary animals (including domestic pets and agricultural animals). In some examples, suitable proteins include insulin, hemoglobin, lysozyme, immunoglobulins, oc-2-macroglobulin, casein and the like, as well as combinations of any two or more thereof. In some examples, suitable proteins are selected from the group consisting of immunoglobulins including IgA, lipoproteins, apolipoprotein B, beta-2-macroglobulin, and thyroglobulin. For the purpose of the invention, the pharmaceutically acceptable carrier comprises albumin (*e.g*., human serum albumin). Proteins, including albumin, suitable for the invention may be natural in origin or synthetically prepared.

Human serum albumin (HSA) has multiple hydrophobic binding sites (a total of eight for fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, *e.g.*, Fehske et al., Biochem. Pharmcol., 30, 687-92 (1981), Vorum, Dan. Med. Bull., 46, 379-99 (1999), Kragh-Hansen, Dan. Med. Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (1992), and Carter et al., Adv. Protein. Chem., 45, 153-203 (1994)).

The carrier protein (*e.g*., albumin) in the composition generally serves as a carrier for the drug, such as a hydrophobic drug derivative, i.e., the carrier protein in the composition makes the drug (*e.g*., hydrophobic taxane derivative) more readily suspendable in an aqueous medium or helps maintain the suspension as compared to compositions not comprising a carrier protein. This can avoid the use of toxic solvents for solubilizing of the hydrophobic taxane derivative, and thereby can reduce one or more side effects of administration of the derivative into an individual (*e.g*., human). In some embodiments, the composition is substantially free (*e.g.* free) of organic solvents or surfactants. A composition is "substantially free of organic solvent" or "substantially free of surfactant" if the amount of organic solvent or surfactant in the composition is not sufficient to cause one or more side effect(s) in an individual when the composition is administered to the individual. In some embodiments, the nanoparticles in the composition have a solid core. In some embodiments, the nanoparticles in the composition have a core that is not aqueous (*i.e*., other than aqueous core). In some embodiments, the nanoparticles of the composition lack a polymeric matrix. In some embodiments, the nanoparticles of the composition are filter sterilizable. In some embodiments, the nanoparticles in the composition comprise at least ten-percent of carrier protein that is cross-linked.

The drug, *e.g*., hydrophobic drug derivative (*e.g*., hydrophobic taxane derivative) is "stabilized" in an aqueous suspension if it remains suspended in an aqueous medium (*e.g.*, without visible precipitation or sedimentation) for an extended period of time, such as for at least about any one of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (*e.g*., human). Stability of the suspension is generally (but not necessarily) evaluated at storage temperature, such as room temperature (*e.g*., 20-25 °C) or refrigerated conditions (*e.g*., 4 °C). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40 °C.

In some examples, the composition comprises nanoparticles comprising (in various variations consisting essentially of) a drug or hydrophobic drug derivative (*e.g*., a hydrophobic taxane derivative such as any one of compounds 1, 2, 3-23 and any compound of Formula I, II, III, IV, V, or VI) and a carrier protein. When the derivative is in a liquid form, the particles or nanoparticles are also referred to as droplets or nanodroplets. In some examples, the hydrophobic taxane derivative is coated with the carrier protein. Particles (such as nanoparticles) of poorly water soluble pharmaceutical agents have been disclosed in, for example, U.S. Pat. Nos. 5,916,596; 6,506,405; 6,096,331; 6,749,868; and 6,537,579; in U.S. Pat. App. Pub. No. 2005/0004002A1; and in PCT Application Pub. Nos. WO98/14174, WO99/00113, WO07/027941 and WO07/027819.

The amount of carrier protein in the composition described herein will vary, for example, depending on the specific drug, *e.g*., hydrophobic drug derivative (*e.g*., hydrophobic taxane derivative), other components in the composition, and/or the route of intended administration. In some embodiments, the composition comprises a carrier protein in an amount that is sufficient to stabilize the drug or derivative in an aqueous suspension, for example, in the form of a stable colloidal suspension (*e.g*., a stable suspension of nanoparticles). In some embodiments, the carrier protein is in an amount that reduces the sedimentation rate of the drug in an aqueous medium. In some embodiments, the amount of carrier protein included in the composition is an amount effective to reduce one or more side effects of the drug. The amount of the carrier protein may also depend on the size and density of particles of the drug or hydrophobic drug derivative (*e.g*., hydrophobic taxane derivative).

In some embodiments, the composition, in liquid form, comprises from about 0.1% to about 25% by weight (*e.g.* about 0.5% by weight, about 5% by weight, about 10% by weight, about 15% by weight, or about 20% by weight) of carrier protein (i.e., albumin). In some embodiments, the composition, in liquid form, comprises about 0.5% to about 5% by weight of carrier protein (i.e., albumin). The composition can be dehydrated, for example, by lyophilization, spray-drying, fluidized-bed drying, wet granulation, and other suitable methods known in the art. When the composition is prepared in solid form, such as by wet granulation, fluidized-bed drying, and other methods known to those skilled in the art, the carrier protein (i.e., albumin) is applied to the active pharmaceutical agent, and other excipients if present, as a solution. In some embodiments, the solution is from about 0.1% to about 25% by weight (about 0.5% by weight, about 5% by weight, about 10% by weight, about 15% by weight, or about 20% by weight of carrier protein (*e.g*., albumin).

In some embodiments, the composition comprises more than, equal to, or less than any one of about 5%, about 10%, about 20%, about 25%, about 30%, about 40%, about 45%, about 50%, about 55%, about 60%, about 65%, about 75% or about 80% of carrier protein (i.e., albumin) in nanoparticle form.

In some embodiments, the carrier protein is present in an effective amount to reduce one or more side effects associated with administration of the drug to a human compared to compositions without carrier protein. These side effects include, but are not limited to, myelosuppression, neurotoxicity, hypersensitivity, inflammation, venous irritation, phlebitis, pain, skin irritation, neutropenic fever, anaphylactic reaction, hematologic toxicity, and cerebral or neurologic toxicity, and combinations thereof. In some examples, there is disclosed a method of reducing hypersensitivity reactions associated with administration of the hydrophobic taxane derivative, including, for example, severe skin rashes, hives, flushing, dyspnea, tachycardia, pulmonary hypertension (*e.g*., lymphoma); chest pain; black, tarry stools; general feeling of illness, shortness of breath; swollen glands; weight loss; yellow skin and eyes, abdominal pain; unexplained anxiousness; bloody or cloudy urine; bone pain; chills; confusion; convulsions (seizures); cough; decreased urge to urinate; fast, slow, or irregular heartbeat; fever; frequent urge to urinate; increased thirst; loss of appetite; lower back or side pain; mood changes; muscle pain or cramps; nausea or vomiting; numbness or tingling around lips, hands, or feet; painful or difficult urination; rash; sore throat; sores or white spots on lips or in mouth; swelling of hands, ankles, feet, or lower legs; swollen glands; trouble breathing; unusual bleeding or bruising; unusual tiredness or weakness; weakness or heaviness of legs, skin ulcer or sores, weight gain, acne; constipation; diarrhea; difficulty in moving; headache; loss of energy or weakness; muscle pain or stiffness; pain; shaking or trembling; trouble sleeping; nosebleed; and/or swelling of the face. These side effects, however, are merely exemplary and other side effects, or combination of side effects, associated with the hydrophobic drug derivative (*e.g*., hydrophobic taxane derivative) can be reduced. The side effects may be immediate or delayed (such as not occurring for a few days, weeks, months, or years after treatment begins).

### Antimicrobial Agents in Compositions

In some embodiments, the compositions of the invention also includes an antimicrobial agent (*e.g*., an agent in addition to the hydrophobic taxane derivative) in an amount sufficient to significantly inhibit (*e.g*., delay, reduce, slow, and/or prevent) microbial growth in the composition for use in the methods of treatment, methods of administration, and dosage regimes described herein. Exemplary microbial agents and variations for the use of microbial agents are disclosed in U.S. Pat. App. Pub. No. 2007/0117744A1 (such as those described in paragraphs [0036] to [0058] therein). In some embodiments, the antimicrobial agent is a chelating agent, such as EDTA, edetate, citrate, pentetate, tromethamine, sorbate, ascorbate, derivatives thereof, or mixtures thereof. In some embodiments, the antimicrobial agent is a polydentate chelating agent. In some embodiments, the antimicrobial agent is a non-chelating agent, such as any of sulfites, benzoic acid, benzyl alcohol, chlorobutanol, and paraben. In some embodiments, an antimicrobial other than the taxane discussed above is not contained or used in the methods of treatment, methods of administration, and dosage regimes described herein.

### Sugar Containing Composition

In some embodiments, the compositions of the invention include a sugar for use in the methods of treatment described herein. In some embodiments, the compositions of the invention include both a sugar and an antimicrobial agent for use in the methods of treatment described herein. Exemplary sugars and variations for the use of sugars are disclosed in U.S. Pat. App. Pub. No. 2007/0117744A1 (such as those described in paragraphs [0084] to [0090] therein). In some embodiments, the sugar serves as a reconstitution enhancer which causes a lyophilized composition to dissolve or suspend in water and/or aqueous solution more quickly than the lyophilized composition would dissolve without the sugar. In some embodiments, the composition is a liquid (*e.g*., aqueous) composition obtained by reconstituting or resuspending a dry composition. In some embodiments, the concentration of sugar in the composition is greater than about 50 mg/ml. In some embodiments, the sugar is in an amount that is effective to increase the stability of the drug or hydrophobic drug derivative (*e.g*., hydrophobic taxane derivative) in the composition as compared to a composition without the sugar. In some embodiments, the sugar is in an amount that is effective to improve filterability of the composition as compared to a composition without the sugar.

The sugar-containing compositions described herein may further comprise one or more antimicrobial agents, such as the antimicrobial agents described herein or in U.S. Pat. App. Pub. No. 2007/0117744A1. In addition to one or more sugars, other reconstitution enhancers (such as those described in U.S. Pat. App. Publication No. 2005/0152979 can also be added to the compositions. In some embodiments, a sugar is not contained or used in the methods of treatment, methods of administration, and dosage regimes described herein.

### Stabilizing Agents in Composition

In some embodiments, the compositions of the invention also include a stabilizing agent for use in the methods of treatment, methods of administration, and dosage regimes described herein. In some embodiments, the compositions of the invention include an antimicrobial agent and/or a sugar and/or a stabilizing agent for use in the methods of treatment, methods of administration, and dosage regimes described herein. Exemplary stabilizing agents and variations for the use of stabilizing agents are disclosed in US 2007/0082838 (such as those described in paragraphs [0038] to [0083] and [0107] to [0114] therein). The present invention in another variation provides for compositions which retain the desirable therapeutic effects and remain physically and/or chemically stable upon exposure to certain conditions such as prolonged storage, elevated temperature, or dilution for parenteral administration. The stabilizing agent includes, for example, chelating agents (*e.g*., citrate, malic acid, edetate, or pentetate), sodium pyrophosphate, and sodium gluconate. In some embodiments, the invention provides pharmaceutical formulations of the hydrophobic drug derivative comprising citrate, sodium pyrophosphate, EDTA, sodium gluconate, citrate and/or sodium chloride. In another variation, the invention provides a composition comprising a hydrophobic drug derivative wherein the derivative used for preparing the formulation is in an anhydrous form prior to being incorporated into the composition.

In some embodiments, a stabilizing agent is not contained or used in the methods of treatment, methods of administration, and dosage regimes described herein.

### Pharmaceutical Compositions and Formulations

The compositions described herein may be used in the preparation of a formulation, such as a pharmaceutical composition or formulation, by combining the nanoparticle composition(s) described with a pharmaceutical acceptable carrier, excipients, stabilizing agents and/or other agents, which are known in the art, for use in the methods of treatment, methods of administration, and dosage regimes described herein.

To increase stability by increasing the negative zeta potential of nanoparticles, certain negatively charged components may be added. Such negatively charged components include, but are not limited to bile salts, bile acids, glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid, and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylcholine, stearoyloleoylphosphatidylcholine, stearoylarachidoylphosphatidylcholine, and dipalmitoylphosphatidylcholine. Other phospholipids including L-α-dimyristoylphosphatidylcholine (DMPC), dioleoylphosphatidylcholine (DOPC), distearoylphosphatidylcholine (DSPC), hydrogenated soy phosphatidylcholine (HSPC), and other related compounds. Negatively charged surfactants or emulsifiers are also suitable as additives, *e.g*., sodium cholesteryl sulfate and the like.

The nanoparticle compositions described herein can be stabilized with a pharmaceutically acceptable surfactant. The term "surfactants," as used herein, refers to surface active group(s) of amphiphile molecules. Surfactants can be anionic, cationic, nonionic, and zwitterionic. Any suitable surfactant can be included in the inventive pharmaceutical composition. Suitable surfactants include non-ionic surfactants such as phosphatides, polyoxyethylene sorbitan esters, and tocopheryl polyethylene glycol succinate. In some embodiments, the surfactant is egg lecithin, tween 80, or vitamin E-t d-ac-tocopheryl polyethylene glycol-1000 succinate (TPGS).

Suitable pharmaceutical carriers include sterile water; saline, dextrose; dextrose in water or saline; condensation products of castor oil and ethylene oxide combining about 30 to about 35 moles of ethylene oxide per mole of castor oil; liquid acid; lower alkanols; oils such as corn oil; peanut oil, sesame oil and the like, with emulsifiers such as mono- or di-glyceride of a fatty acid, or a phosphatide, *e.g*., lecithin, and the like; glycols; polyalkylene glycols; aqueous media in the presence of a suspending agent, for example, sodium carboxymethylcellulose; sodium alginate; poly(vinylpyrolidone) ; and the like, alone, or with suitable dispensing agents such as lecithin; polyoxyethylene stearate; and the like. The carrier may also contain adjuvants such as preserving stabilizing, wetting, emulsifying agents and the like together with the penetration enhancer. The final form may be sterile and may also be able to pass readily through an injection device such as a hollow needle. The proper viscosity may be achieved and maintained by the proper choice of solvents or excipients. Moreover, the use of molecular or particulate coatings such as lecithin, the proper selection of particle size in dispersions, or the use of materials with surfactant properties may be utilized.

The nanoparticle compositions described herein may include other agents, excipients, or stabilizers to improve properties of the composition. Examples of suitable excipients and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate and mineral oil. The formulations can additionally include lubricating agents, wetting agents, emulsifying and suspending agents, preserving agents, sweetening agents or flavoring agents. Examples of emulsifying agents include tocopherol esters such as tocopheryl polyethylene glycol succinate and the like, pluronic®, emulsifiers based on polyoxy ethylene compounds, Span 80 and related compounds and other emulsifiers known in the art and approved for use in animals or human dosage forms. The compositions can be formulated so as to provide rapid, sustained or delayed release of the active ingredient after administration to the patient by employing procedures well known in the art.

In some embodiments, the composition is formulated to have a pH in the range of about 4.5 to about 9.0, including for example pH ranges of any one of about 5.0 to about 8.0, about 6.5 to about 7.5, and about 6.5 to about 7.0. In some embodiments, the pH of the composition is formulated to no less than about 6, including for example no less than about any one of 6.5, 7, or 8 (*e.g*., about 8). The composition can also be made to be isotonic with blood by the addition of a suitable tonicity modifier, such as glycerol.

In some embodiments, the composition is suitable for administration to a human. There are a wide variety of suitable formulations of the inventive composition (see, *e.g*., U.S. Pat. Nos. 5,916,596 and 6,096,331. The following formulations and methods are merely exemplary and are in no way limiting.

Formulations suitable for oral administration can comprise (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, (d) suitable emulsions, and (e) powders. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

The nanoparticles of this invention can be enclosed in a hard or soft capsule, can be compressed into tablets, or can be incorporated with beverages or food or otherwise incorporated into the diet. Capsules can be formulated by mixing the nanoparticles with an inert pharmaceutical diluent and inserting the mixture into a hard gelatin capsule of the appropriate size. If soft capsules are desired, a slurry of the nanoparticles with an acceptable vegetable oil, light petroleum or other inert oil can be encapsulated by machine into a gelatin capsule.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizing agents, and preservatives. The formulations can be presented in unit-dose or multi-dose sealed containers, such as ampules and vials, and can be stored in a freeze-dried (lyophilized) condition requiring only the addition of the sterile liquid excipient methods of treatment, methods of administration, and dosage regimes described herein (*i.e*., water) for injection, immediately prior to use. Extemporaneous injection solutions and suspensions can be prepared from sterile powders, granules, and tablets of the kind previously described. Injectable formulations are preferred.

The invention also includes formulations of nanoparticle compositions comprising the drug and a carrier suitable for administration by inhalation for use in the methods of the invention. Formulations suitable for aerosol administration comprise the inventive composition include aqueous and non-aqueous, isotonic sterile solutions, which can contain anti-oxidants, buffers, bacteriostats, and solutes, as well as aqueous and non-aqueous sterile suspensions that can include suspending agents, solubilizers, thickening agents, stabilizing agents, and preservatives, alone or in combination with other suitable components, which can be made into aerosol formulations to be administered via inhalation. These aerosol formulations can be placed into pressurized acceptable propellants, such as dichlorodifluoromethane, propane, nitrogen, and the like. They also can be formulated as pharmaceuticals for non-pressured preparations, such as in a nebulizer or an atomizer.

The invention also includes formulations of nanoparticle compositions administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The invention also includes formulations of nanoparticle compositions administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

Also provided are unit dosage forms comprising the compositions and formulations provided herein. These unit dosage forms can be stored in a suitable packaging in single or multiple unit dosages and may also be further sterilized and sealed. For example, the pharmaceutical composition (*e.g*., a dosage or unit dosage form of a pharmaceutical composition) may include (i) nanoparticles that comprise the drug and the carrier protein and (ii) a pharmaceutically acceptable carrier. In some embodiments, the pharmaceutical composition also includes one or more other compounds (or pharmaceutically acceptable salts thereof) that are useful for treating cancer. In various variations, the amount of hydrophobic taxane derivative in the composition is included in any one of the following ranges: about 5 to about 50 mg, about 20 to about 50 mg, about 50 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of hydrophobic taxane derivative in the composition (*e.g*., a dosage or unit dosage form) is in the range of about 5 mg to about 500 mg, such as about 30 mg to about 300 mg or about 50 mg to about 200 mg, of the derivative. In some embodiments, the carrier is suitable for parental administration (*e.g*., intravenous administration). In some embodiments, the hydrophobic drug derivative is the only pharmaceutically active agent for the treatment of cancer that is contained in the composition.

In some embodiments, the invention features a dosage form (*e.g*., a unit dosage form) for the treatment of cancer comprising (i) nanoparticles that comprise the carrier protein and the hydrophobic drug derivative, wherein the amount of derivative in the unit dosage from is in the range of about 5 mg to about 500 mg, and (ii) a pharmaceutically acceptable carrier. In some embodiments, the amount of the hydrophobic drug derivative in the unit dosage form includes about 30 mg to about 300 mg.

Also provided are articles of manufacture comprising the compositions, formulations, and unit dosages provided herein in suitable packaging for use in the methods of treatment, methods of administration, and dosage regimes described herein. Suitable packaging for compositions described herein are known in the art, and include, for example, vials (such as sealed vials), vessels (such as sealed vessels), ampules, bottles, jars, flexible packaging (*e.g*., sealed Mylar or plastic bags), and the like. These articles of manufacture may further be sterilized and/or sealed.

### Kits

The invention also provides kits comprising the compositions, formulations, unit dosages, and articles of manufacture provided herein for use in the methods of treatment, methods of administration, and dosage regimes described herein. Kits of the invention include one or more containers comprising hydrophobic taxane derivative-containing nanoparticle compositions (formulations or unit dosage forms and/or articles of manufacture), and in some embodiments, further comprise instructions for use in accordance with any of the methods of treatment described herein. In some embodiments, the kit comprises i) a composition comprising nanoparticles comprising drug and the carrier protein (i.e., albumin) and ii) instructions for administering the nanoparticles and the chemotherapeutic agents simultaneously and/or sequentially, for treatment of cancer. In various variations, the amount of a hydrophobic drug derivative (*e.g*., hydrophobic taxane derivative) in the kit is included in any one of the following ranges: about 5 mg to about 20 mg, about 20 to about 50 mg, about 50 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of the hydrophobic taxane derivative in the kit is in the range of about 5 mg to about 500 mg, such as about 30 mg to about 300 mg or about 50 mg to about 200 mg. In some embodiments, the kit includes one or more other compounds (*i.e*., one or more compounds other than a hydrophobic drug derivative, such as other than a hydrophobic taxane derivative) that are useful for cancer.

Instructions supplied in the kits of the invention are typically written instructions on a label or package insert (*e.g*., a paper sheet included in the kit), but machine-readable instructions (*e.g.*, instructions carried on a magnetic or optical storage disk) are also acceptable. The instructions relating to the use of the nanoparticle compositions generally include information as to dosage, dosing schedule, and route of administration for the intended treatment. The kit may further comprise a description of selecting an individual suitable or treatment.

The present invention also provides kits comprising compositions (or unit dosages forms and/or articles of manufacture) provided herein and may further comprise instruction(s) on methods of using the composition, such as uses further described herein. In some embodiments, the kit of the invention comprises the packaging described above. In other variations, the kit of the invention comprises the packaging described above and a second packaging comprising a buffer. It may further include other materials desirable from a commercial and user standpoint, including other buffers, diluents, filters, needles, syringes, and package inserts with instructions for performing any methods described herein.

For combination therapies of the invention, the kit may contain instructions for administering the first and second therapies simultaneously and/or sequentially for the effective treatment of cancer. The first and second therapies can be present in separate containers or in a single container. It is understood that the kit may comprise one distinct composition or two or more compositions wherein one composition comprises a first therapy and one composition comprises a second therapy.

Kits may also be provided that contain sufficient dosages of the hydrophobic drug derivative to provide effective treatment for an individual for an extended period, such as any one of a week, 2 weeks, 3 weeks, 4 weeks, 6 weeks, 8 weeks, 3 months, 4 months, 5 months, 6 months, 7 months, 8 months, 9 months or more. Kits may also include multiple unit doses of the hydrophobic taxane derivative compositions, pharmaceutical compositions, and formulations described herein and instructions for use and packaged in quantities sufficient for storage and use in pharmacies, for example, hospital pharmacies and compounding pharmacies. In some embodiments, the kit comprises a dry (*e.g*., lyophilized) composition that can be reconstituted, resuspended, or rehydrated to form generally a stable aqueous suspension of nanoparticles comprising a hydrophobic drug derivative (*e.g*., hydrophobic taxane derivative) and albumin (*e.g*., a hydrophobic taxane derivative coated with albumin).

The kits of the invention are in suitable packaging. Suitable packaging include, but is not limited to, vials, bottles, jars, flexible packaging (*e.g*., sealed Mylar or plastic bags), and the like. Kits may optionally provide additional components such as buffers and interpretative information.

### Methods of Making the Nanoparticle Compositions

Methods of making compositions containing carrier proteins and poorly water soluble pharmaceutical agents are known in the art. For example, nanoparticles containing poorly water soluble pharmaceutical agents and carrier proteins (*e.g*., albumin) can be prepared under conditions of high shear forces (*e.g*., sonication, high pressure homogenization, or the like). These methods are disclosed in, for example, U.S. Patent Nos. 5,916,596; 6,096,331; 6,749,868; and 6,537,579; and PCT Application Pub. Nos. WO98/14174; WO99/00113; WO07/027941; and WO07/027819.

Briefly, the drug (*e.g.*, hydrophobic drug derivative, such as a hydrophobic taxane derivative) is dissolved in an organic solvent. Suitable organic solvents include, for example, ketones, esters, ethers, chlorinated solvents, and other solvents known in the art. For example, the organic solvent can be methylene chloride, chloroform/ethanol, or chloroform/t-butanol (for example with a ratio of about any one of 1:9, 1:8, 1:7, 1:6, 1:5, 1:4, 1:3, 1:2, 1:1, 2:1, 3:1, 4:1, 5:1, 6:1, 7:1, 8:1, or 9:1 or with a ratio of about any one of 3:7, 5:7, 4:6, 5:5, 6:5, 8:5, 9:5, 9.5:5, 5:3, 7:3, 6:4, or 9.5:0.5). The solution is added to a carrier protein (*e.g*., human serum albumin). The mixture is subjected to high pressure homogenization (*e.g*., using an Avestin, APV Gaulin, Microfluidizer™ such as a Microfluidizer™ Processor M-110EH from Microfluidics, Stansted, or Ultra Turrax homogenizer). The emulsion may be cycled through the high pressure homogenizer for between about 2 to about 100 cycles, such as about 5 to about 50 cycles or about 8 to about 20 cycles (*e.g*., about any one of 8, 10, 12, 14, 16, 18 or 20 cycles). The organic solvent can then be removed by evaporation utilizing suitable equipment known for this purpose, including, but not limited to, rotary evaporators, falling film evaporators, wiped film evaporators, spray driers, and the like that can be operated in batch mode or in continuous operation. The solvent may be removed at reduced pressure (such as at about any one of 25 mm Hg, 30 mm Hg, 40 mm Hg, 50 mm Hg, 100 mm Hg, 200 mm Hg, or 300 mm Hg). The amount of time used to remove the solvent under reduced pressure may be adjusted based on the volume of the formulation. For example, for a formulation produced on a 300 mL scale, the solvent can be removed at about 1 to about 300 mm Hg (*e.g*., about any one of 5-100 mm Hg, 10-50 mm Hg, 20-40 mm Hg, or 25 mm Hg) for about 5 to about 60 minutes (*e.g.*, about any one of 7, 8, 9, 10, 11, 12, 13, 14, 15 16, 18, 20, 25, or 30 minutes). The dispersion obtained can be further lyophilized.

If desired, human albumin solution may be added to the dispersion to adjust the human serum albumin to the drug (*e.g*., docetaxel) or hydrophobic drug derivative (*e.g*., hydrophobic taxane derivative) ratio, or to adjust the concentration of the hydrophobic taxane derivative in the dispersion. For example, human serum albumin solution (*e.g*., 25 % w/v) can be added to adjust the human serum albumin to hydrophobic drug derivative (*e.g*., a hydrophobic taxane derivative such as any one of compounds 1, 2, 3-23 and any compound of Formula I, II, III, IV, V, or VI) ratio to about any one of 18:1, 15:1 14:1, 13:1, 12:1, 11:1, 10:1, 9:1, 8:1, 7.5:1, 7:1, 6:1, 5:1, 4:1, or 3:1. For example, human serum albumin solution (*e.g*., 25 % w/v) or another solution is added to adjust the concentration of a drug or hydrophobic drug derivative in the dispersion to about any one of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml. The dispersion may be serially filtered through multiple filters, such as a combination of 1.2 µm and 0.8/0.2 µm filters; the combination of 1.2 µm, 0.8 µm, 0.45 µm, and 0.22 µm filters; or the combination of any other filters known in the art. The dispersion obtained can be further lyophilized. The nanoparticle compositions may be made using a batch process or a continuous process (*e.g*., the production of a composition on a large scale).

If desired, a second therapy (*e.g*., one or more compounds useful for treating cancer), an antimicrobial agent, sugar, and/or stabilizing agent can also be included in the composition. For example, this additional agent can either be admixed with a hydrophobic drug derivative (*e.g*., hydrophobic taxane derivative) and/or the carrier protein during preparation of a hydrophobic drug derivative (*e.g*., hydrophobic taxane derivative)/carrier protein composition, or added after the hydrophobic taxane derivative/carrier protein composition is prepared. In some examples, the agent is admixed with the hydrophobic taxane derivative/carrier protein composition prior to lyophilization. In some examples, the agent is added to the lyophilized hydrophobic taxane derivative/carrier protein composition. In some examples when the addition of the agent changes the pH of the composition, the pH in the composition are generally (but not necessarily) adjusted to a desired pH. Exemplary pH values of the compositions include, for example, in the range of about 5 to about 8.5. In some embodiments, the pH of the composition is adjusted to no less than about 6, including for example no less than any one of about 6.5, 7, or 8 (*e.g*., about 8).

In some examples disclosed herein is an emulsion comprising a drug or hydrophobic drug derivative (*e.g*., a hydrophobic taxane derivative such as any one of compounds 1, 2, 3-23 and any compound of Formula I, II, III, IV, V, or VI), the emulsion comprising: (a) a first phase comprising nanodroplets comprising at least a portion of the hydrophobic taxane derivative dissolved in an organic solvent for the hydrophobic taxane derivative and an alcohol solvent for the hydrophobic taxane derivative, and (b) a second phase comprising water and a biocompatible polymer, wherein the emulsion is substantially free of surfactants.

### Methods of Making Hydrophobic Drug Derivatives

The hydrophobic drug derivatives (*e.g.*, hydrophobic taxane derivatives) disclosed herein may be synthesized by an appropriate combination of generally well-known synthetic methods. Techniques useful in synthesizing the compounds are both readily apparent and accessible to those of skill in the relevant art, particularly in view of the teachings described herein. The discussion below is offered to illustrate certain of the diverse methods available for use in assembling the compounds. However, the discussion is not intended to define the scope of reactions or reaction sequences that are useful in preparing the compounds, nor is it intended to define the scope of the compounds themselves.

The synthesis of some compounds disclosed herein are disclosed in WO 2006/089207.

Some hydrophobic taxane derivatives may be synthesized by modifying the 2'-hydroxyl of the taxane as shown in Scheme 1. Treatment of the taxane (*e.g*., docetaxel) with about one equivalent of a reactive hydrophobic group (*e.g.*, a benzyl halide, such as benzoyl chloride) in the presence of a base (such as triethylamine or pyridine) provides the desired hydrophobic taxane derivative. Alternatively, treatment of the taxane with about one equivalent of a reactive hydrophobic group (*e.g*., benzoic acid) in the presence of a coupling agent (*e.g*., dicyclohexylcarbodiimide) and optionally a catalytic amount of 4-pyrrolidinopyridine or 4-dimethylaminopyridine provides the desired hydrophobic taxane derivative (*e.g*., 2'-benzoyl docetaxel shown in scheme 1).

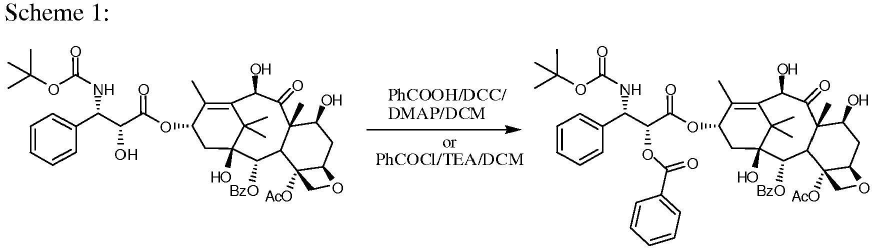

Some hydrophobic taxane derivatives may be synthesized by modifying the 7 position of the taxane as shown in Scheme 2. To introduce new functionality of a hydrophobic group at the 7-position, the reactivity of the 2'-hydroxyl group of the taxane may be blocked with a protecting group. The use of selective protecting groups, such as triethylsilyl, may be used as it is easily removed from the 2'-hydroxyl by treatment with acid (*e.g*., hydrochloric acid in methanol, or hydrofluoric acid in pyridine). Thus, upon treatment of the taxane (*e.g*., docetaxel) with about one equivalent of chlorotriethylsilane (TESC1) in the presence of a base (*e.g.*, triethylamine (TEA) or pyridine), 2'-protected hydroxyl taxane (*e.g*., 2'-triethylsilyl paclitaxel) can be afforded in good yield, as shown in Scheme 2. Alternatively, other protecting groups, such as 2,2,2-Trichloroethyl-oxycarbonyl derivatives can also be used and subsequently removed by treatment with zinc and acid (*e.g*., acetic acid).

The taxane containing the 2'-protected hydroxyl can then be subjected to the reactive hydrophobic group as described herein (*e.g*., benzoic acid in the presence of dicyclohexylcarbodiimide and a catalytic amount of 4-pyrrolidinopyridine or 4-dimethylaminopyridine), to generate a hydrophobic taxane derivative modified at the 7 position. The 2'-protected hydroxyl (*e.g*., 2'-triethylsilyl group) can readily be liberated by removing the protecting group (*e.g*., under mild acidic conditions), generating the desired product.

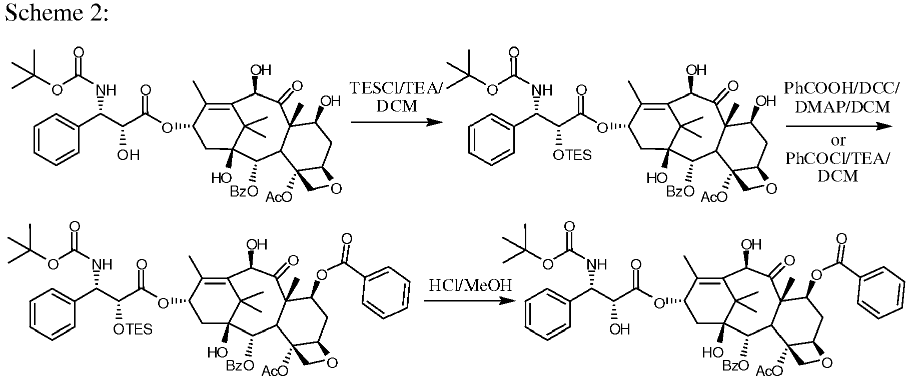

Some hydrophobic taxane derivatives may be synthesized by modifying the 10-position of the taxane as shown in Scheme 3. To introduce new functionality of a hydrophobic group at the 10-position, the reactivity of both the 2'-hydroxyl group and the 7-hydroxyl group of the taxane may be blocked with a protecting group. Upon treatment of the taxane (*e.g*., docetaxel) with about two equivalents of a suitable protecting group (*e.g*., chlorotriethylsilane (TESC1)), in the presence of a base (such as pyridine), the doubly protected taxane can be produced (*e.g*., 2',7-bis(triethylsilyl) docetaxel) can be produced. When this protected taxane is subjected to the reactive hydrophobic group as described herein (*e.g*., benzoyl chloride/pyridine or benzoic acid, in the presence of dicyclohexylcarbodiimide and a catalytic amount of 4-dimethylaminopyridine), the desired 10-acylation product is obtained (*e.g*., 10-acylation), from which both protecting groups can readily be removed (*e.g*., under mild acidic conditions).

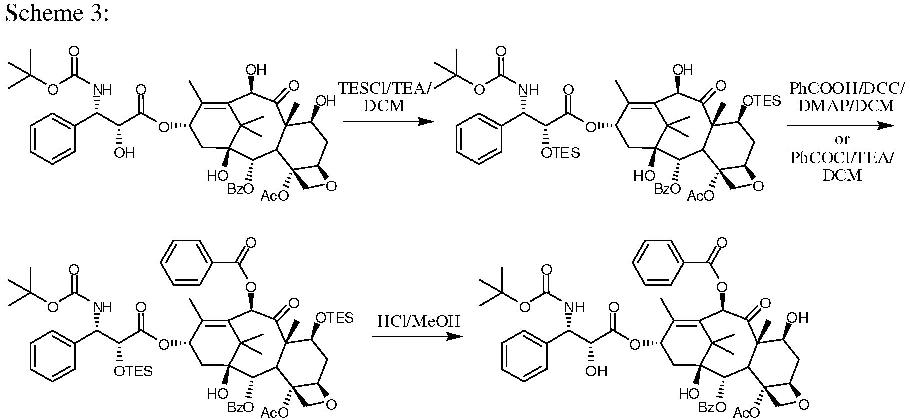

With the availability of the 2'-and 7-protected taxane, further modification at the 10-position with acyl functionality can be accomplished by using different functional groups to link the hydrophobic groups and taxanes (*e.g*., esters, carbonates, carbamates, and the like).

In accordance with one example, the hydrophobic group, such as benzoyl, may be conjugated to virtually any drug compound or diagnostic agent, formulated and used according to the methods disclosed herein. Pharmaceutical agents include the following categories and specific examples. It is not intended that the category be limited by the specific examples. Those of ordinary skill in the art will, in light of the teachings disclosed herein, recognize numerous other compounds that fall within the categories and that are useful.

Also disclosed are products made by the methods described herein.

### Methods of Measuring Anticancer Activity

Anticancer activity of the compounds described herein (*e.g*., hydrophobic taxane derivatives) or nanoparticle compositions thereof can be examined *in vitro,* for example, by incubating a cancer cell culture with the derivative, and then evaluating cell growth inhibition in the culture. Suitable cells for such testing include murine P388 leukemia, B16 melanoma and Lewis lung cancer cells, as well as human mammary MCF7, ovarian OVCAR-3, A549 lung cancer cells, MX-1 (human breast tumor cell), HT29 (colon cancer cell line), HepG2 (liver cancer cell lines), and HCT116 (colon cancer cell lines). Alternatively, for example, a hydrophobic drug derivative (or composition comprising hydrophobic drug derivative) can be tested *in vivo* for antitumor activity, for example, by first establishing tumors in suitable test animals, *e.g.*, nude mice. Cells suitable for establishing tumors include those described above for *in vitro* testing, as well as other cells generally accepted in the art for establishing tumors. Subsequently, the drug or hydrophobic drug derivative (*e.g*., hydrophobic taxane derivative) is administered to the animal; ED₅₀ values, that is, the amount of the derivative (or composition) required to achieve 50% inhibition of tumor growth in the animal are then determined, as are survival rates. Ordinarily skilled artisans, given the teachings described herein, are well able to select particular compounds described herein (or nanoparticle compositions comprising the compounds described herein) for application against certain cancers, on the basis of such factors as ED₅₀ and survival values.

### Methods of Treatment

The nanoparticle compositions of the present invention may be used to treat diseases associated with cellular proliferation or hyperproliferation, such as cancers. In some embodiments are provided compositions of the invention for use in methods of treating a proliferative disease (*e.g.*, cancer) in an individual, wherein the method comprises administering to the individual an effective amount of the composition of the invention.

Examples of cancers that may be treated by the compositions of the invention include, but are not limited to, multiple myeloma, renal cell carcinoma, prostate cancer, lung cancer, melanoma, colon cancer, colorectal cancer, ovarian cancer, liver, renal, gastric, and breast cancer.

In some variations, the individual being treated for a proliferative disease has been identified as having one or more of the conditions described herein. Identification of the conditions as described herein by a skilled physician is routine in the art (*e.g*., via blood tests, X-rays, CT scans, endoscopy, biopsy, etc.) and may also be suspected by the individual or others, for example, due to tumor growth, hemorrhage, ulceration, pain, enlarged lyph nodes, cough, jaundice, swelling, weight loss, cachexia, sweating, anemia, paraneoplastic phenomena, thrombosis, etc. In some embodiments, the individual has been identified as susceptible to one or more of the conditions as described herein. The susceptibility of an individual may be based on any one or more of a number of risk factors and/or diagnostic approaches appreciated by the skilled artisan, including, but not limited to, genetic profiling, family history, medical history (*e.g*., appearance of related conditions), lifestyle or habits.

In some embodiments, the methods and/or compositions used herein reduce the severity of one or more symptoms associated with proliferative disease (*e.g*., cancer) by at least about any one of 10%, 20%, 30%, 40%, 50%, 60%, 70%, 80%, 90%, 95%, or 100% compared to the corresponding symptom in the same individual prior to treatment or compared to the corresponding symptom in other individuals not receiving the methods and/or compositions.

### Combination Therapy

In some embodiments, the invention provides a composition of the invention for use in a method of treating cancer in an individual, wherein the method comprises administering to the individual an effective amount of a combination of a) a first therapy that comprises a composition of the invention; and b) a second therapy useful for treating cancer. In some embodiments, the second therapy includes surgery, radiation, gene therapy, immunotherapy, bone marrow transplantation, stem cell transplantation, hormone therapy, targeted therapy, cryotherapy, ultrasound therapy, photodynamic therapy, and/or chemotherapy (*e.g*., one or more compounds useful for treating cancer). It is understood that reference to and description of methods of treating cancer below is exemplary and that this description applies equally to and includes methods of treating cancer using combination therapy.

### Dosing and Method of Administration

The amount of the inventive composition administered to an individual (such as a human) may vary with the particular composition, the method of administration, and the particular type of recurrent cancer being treated. The amount should be sufficient to produce a desirable beneficial effect. For example, in some embodiments, the amount of the composition is effective to result in an objective response (such as a partial response or a complete response). In some embodiments, the amount of nanoparticle composition is sufficient to result in a complete response in the individual. In some embodiments, the amount of the composition is sufficient to result in a partial response in the individual. In some embodiments, the amount of the composition administered alone is sufficient to produce an overall response rate of more than about any one of 40%, 50%, 60%, or 64% among a population of individuals treated with the composition. Responses of an individual to the treatment of the methods described herein can be determined, for example, based on RECIST or CA-125 level. For example, when CA-125 is used, a complete response can be defined as a return to a normal range value of at least 28 days from the pretreatment value. A particle response can be defined as a sustained over 50% reduction from the pretreatment value.

In some embodiments, the amount of nanoparticle composition is sufficient to prolong progress-free survival of the individual (for example as measured by RECIST or CA-125 changes). In some embodiments, the amount of the nanoparticle composition is sufficient to prolong overall survival of the individual. In some embodiments, the amount of the composition is sufficient to produce clinical benefit of more than about any one of 50%, 60%, 70%, or 77% among a population of individuals treated with the composition.

In some embodiments, the amount of drug in the composition is below the level that induces a toxicological effect (*i.e*., an effect above a clinically acceptable level of toxicity) or is at a level where a potential side effect can be controlled or tolerated when the composition is administered to the individual. In some embodiments, the amount of the composition is close to a maximum tolerated dose (MTD) of the composition following the same dosing regime. In some embodiments, the amount of the composition is more than about any one of 80%, 90%, 95%, or 98% of the MTD.

In some embodiments, the amount of the compound and/or composition is an amount sufficient to decrease the size of a tumor, decrease the number of cancer cells, or decrease the growth rate of a tumor by at least about any one of 10%, 20%, 30%, 40%, 50%, 60%, 70%, 80%, 90%, 95% or 100% compared to the corresponding tumor size, number of cancer cells, or tumor growth rate in the same subject prior to treatment or compared to the corresponding activity in other subjects not receiving the treatment. Standard methods can be used to measure the magnitude of this effect, such as *in vitro* assays with purified enzyme, cell-based assays, animal models, or human testing.

In some embodiments, the amount of drug in the composition is included in any one of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of the hydrophobic taxane derivative in the effective amount of the composition (*e.g*., a unit dosage form) is in the range of about 5 mg to about 500 mg, such as about 30 mg to about 300 mg or about 50 mg to about 200 mg. In some embodiments, the concentration of the hydrophobic taxane derivative in the composition is dilute (about 0.1 mg/ml) or concentrated (about 100 mg/ml), including for example any one of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some embodiments, the concentration of the hydrophobic taxane derivative is at least about any one of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of the drug of the invention in the nanoparticle composition include, but are not limited to, about any one of 25 mg/m², 30 mg/m², 50 mg/m², 60 mg/m², 75 mg/m², 80 mg/m², 90 mg/m², 100 mg/m², 120 mg/m², 160 mg/m², 175 mg/m², 180 mg/m², 200 mg/m², 210 mg/m², 220 mg/m², 250 mg/m², 260 mg/m², 300 mg/m², 350 mg/m², 400 mg/m², 500 mg/m², 540 mg/m², 750 mg/m², 1000 mg/m², or 1080 mg/m² of the hydrophobic taxane derivative. In various variations, the composition includes less than about any one of 350 mg/m², 300 mg/m², 250 mg/m², 200 mg/m², 150 mg/m², 120 mg/m², 100 mg/m², 90 mg/m², 50 mg/m², or 30 mg/m² of the hydrophobic taxane derivative. In some embodiments, the amount of the hydrophobic taxane derivative per administration is less than about any one of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some embodiments, the effective amount of the hydrophobic taxane derivative in the composition is included in any one of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². Preferably, the effective amount of the hydrophobic taxane derivative in the composition is about 5 to about 300 mg/m², such as about 100 to about 150 mg/m², about 120 mg/m², about 130 mg/m², or about 140 mg/m². In some examples, the nanoparticles comprising paclitaxel are not administered at a dose of 300 mg/m² or 900 mg/m².

In some embodiments of any of the above embodiments, the effective amount of the drug in the composition includes at least about any one of 1 mg/kg, 2.5 mg/kg, 3.5 mg/kg, 5 mg/kg, 6.5 mg/kg, 7.5 mg/kg, 10 mg/kg, 15 mg/kg, or 20 mg/kg. In various variations, the effective amount of a hydrophobic taxane derivative in the composition includes less than about any one of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of the hydrophobic taxane derivative. In some embodiments, the nanoparticles comprising compound 2 are not administered at a dose of 60 mg/kg or 90 mg/kg.

Exemplary dosing frequencies include, but are not limited to, any one of weekly without break; weekly, three out of four weeks; once every three weeks; once every two weeks; weekly, two out of three weeks. In some embodiments, the composition is administered about once every 2 weeks, once every 3 weeks, once every 4 weeks, once every 6 weeks, or once every 8 weeks. In some embodiments, the composition is administered at least about any one of 1x, 2x, 3x, 4x, 5x, 6x, or 7x (*i.e*., daily) a week. In some embodiments, the intervals between each administration are less than about any one of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any one of 1 month, 2 months, 3 months, 4 months, 5 months, 6 months, 8 months, or 12 months. In some embodiments, there is no break in the dosing schedule. In some embodiments, the interval between each administration is no more than about a week.

The administration of the composition can be extended over an extended period of time, such as from about a month up to about seven years. In some embodiments, the composition is administered over a period of at least about any one of 2, 3, 4, 5, 6, 7, 8, 9, 10, 11, 12, 18, 24, 30, 36, 48, 60, 72, or 84 months. In some embodiments, the composition is administered over a period of at least one month, wherein the interval between each administration is no more than about a week, and wherein the dose of the hydrophobic taxane derivative at each administration is about 0.25 mg/m² to about 75 mg/m², such as about 0.25 mg/m² to about 25 mg/m² or about 25 mg/m² to about 50 mg/m².

In some embodiments, the dosage of drug in the nanoparticle composition can be in the range of 5-400 mg/m² when given on a 3 week schedule, or 5-250 mg/m² when given on a weekly schedule. For example, the amount of the hydrophobic taxane derivative is about 60 to about 300 mg/m² (*e.g*., about 260 mg/m²).

Other exemplary dosing schedules for the administration of the nanoparticle composition (*e.g*., hydrophobic taxane derivative/albumin nanoparticle composition) include, but are not limited to, any one of 100 mg/m², weekly, without break; 75 mg/m² weekly, 3 out of four weeks; 100 mg/m², weekly, 3 out of 4 weeks; 125 mg/m², weekly, 3 out of 4 weeks; 125 mg/m², weekly, 2 out of 3 weeks; 130 mg/m2, weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m² twice a week; and 150-250 mg/m² twice a week. The dosing frequency of the composition may be adjusted over the course of the treatment based on the judgment of the administering physician.

The compositions provided herein allow infusion of the composition to an individual over an infusion time that is shorter than about 24 hours. For example, in some embodiments, the composition is administered over an infusion period of less than about any one of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes.

The rate of infusion may play a significant role in the size and/or dissolution profile of the nanoparticle compositions described herein. For example, shorter infusion times may lead to higher blood concentrations of the nanoparticle composition, which may result in stabilizing the nanoparticle, preventing or reducing dissolution and maintaining and/or increasing the nanoparticle size. Stabilizing the nanoparticle form of the drug or hydrophobic drug derivative with carrier and attenuating the nanoparticle size following infusion may improve efficacy (*e.g*., by improved delivery to the desired receptor site, such as gp60 and/or SPARC) and lead to a desired therapeutic effect.

In one aspect, the compositions provided herein are infused into an individual over a shortened infusion time. In some embodiments, a composition provided herein is infused in an individual over an infusion time that is less than any of about 30 minutes, or 20 minutes, or 10 minutes, or 7 minutes, or 5 minutes, or 3 minutes, or 2 minutes, or 1 minute. In some embodiments, a composition provided herein is infused in an individual over an infusion time that is 30 minutes or less, or 20 minutes or less, or 10 minutes or less, or 7 minutes or less, or 5 minutes or less, or 3 minutes or less, or 2 minutes or less, or 1 minute or less.In some examples, the composition comprises an unmodified drug. In some examples, the composition comprises a hydrophobic drug derivative. In some examples, the composition comprises a drug (*e.g*., paclitaxel or docetaxel) and a carrier protein (*e.g*., albumin such as human serum albumin). In some examples, the composition comprises a drug other than paclitaxel or docetaxel, and a carrier protein (*e.g*., albumin such as human serum albumin). In some examples, a nanoparticle composition described herein comprising paclitaxel and a carrier protein (*e.g*., albumin such as human serum albumin) is not infused in an individual over an infusion of about 30 minutes. In some examples, the composition comprises a hydrophobic drug derivative (*e.g*., a hydrophobic taxane derivative) and a carrier protein (*e.g*., albumin such as human serum albumin). In some examples, the composition comprises a hydrophobic paclitaxel derivative or a hydrophobic docetaxel derivative (*e.g*., any one of compounds 1, 2, 3-23 and any compound of Formula I, II, III, IV, V, or VI) and a carrier protein (*e.g*., albumin such as human serum albumin).

In some embodiments, the rate of infusion of a dosage of 300 mg/m³ or 900 mg/m³ of nanoparticles in an individual is sufficient to provide nanoparticles in the blood with an average diameter between about any one of 5 nm and 900 nm, 10 nm and 800 nm, 20 nm and 700 nm, 30 nm and 600 nm, 40 nm and 500 nm, 50 nm and 250 nm, 75 nm and 200 nm, or 100 nm and 150 nm, at more than 1 min, or 2 min, or 3 min, or 5 min, or 10 min, or 20 min, or 30 min, or 45 min, or 1 hr, or 2 hr, following infusion.

In one example, there is a method of treating a proliferative disease in an individual (*e.g*., cancer), comprising administering to the individual in less than 10 minutes an effective amount of a composition comprising a drug (*e.g*., a drug not modified with a hydrophobic group at about 5 to about 300 mg/m², such as about 100 to about 150 mg/m², about 120 mg/m², about 130 mg/m², or about 140 mg/m²) and a carrier protein. In some of these examples, the drug is a taxane (*e.g*., such as paclitaxel or docetaxel). In another example, there is a method of treating a proliferative disease in an individual (*e.g*., cancer), comprising administering to the individual (*e.g*., by infusion) in less than 5 minutes (or less than 3 minutes, or less than 1 minute) an effective amount of a composition comprising paclitaxel or docetaxel (*e.g*., at about 5 to about 300 mg/m², such as about 150 to about 250 mg/m²) and a carrier protein (*e.g*., albumin). In any of these examples, the method is conducted at an interval of once per month, or once per three weeks, or once per two weeks, or once per week, or twice per week, or three times per week.

In another embodiment, provided is a composition of the invention for use in a method of treating a proliferative disease in an individual (*e.g.*, cancer), wherein the method comprises administering to the individual (*e.g*., by infusion) in less than 30 minutes (or less than 20 minutes, or less than 10 minutes, or less than 5 minutes, or less than 2 minutes) an effective amount of the composition comprising the hydrophobic drug derivative (*e.g.*, at about 5 to about 300 mg/m², such as about 100 to about 150 mg/m²) and the carrier protein (i.e., albumin). In any of these embodiments, the method is conducted at an interval of once per month, or once per three weeks, or once per two weeks, or once per week.

In one example, there is a method of treating a proliferative disease in an individual (*e.g*., cancer), comprising administering to the individual in 10 minutes or less an effective amount of a composition comprising a drug (*e.g.*, a drug not modified with a hydrophobic group at about 5 to about 300 mg/m², such as about 100 to about 150 mg/m², about 120 mg/m², about 130 mg/m², or about 140 mg/m²) and a carrier protein. In some of these examples, the drug is a taxane (*e.g.*, such as paclitaxel or docetaxel). In another example, there is a method of treating a proliferative disease in an individual (*e.g*., cancer), comprising administering to the individual (*e.g*., by infusion) in 5 minutes or less (or 3 minutes or less, or 1 minute or less) an effective amount of a composition comprising paclitaxel or docetaxel (*e.g*., at about 5 to about 300 mg/m², such as about 150 to about 250 mg/m²) and a carrier protein (*e.g*., albumin). In any of these examples, the method is conducted at an interval of once per month, or once per three weeks, or once per two weeks, or once per week, or twice per week, or three times per week.

In another embodiment, provided is a composition of the invention for use in a method of treating a proliferative disease in an individual (*e.g.*, cancer), wherein the method comprises administering to the individual (*e.g*., by infusion) in 30 minutes or less (or 20 minutes or less, or 10 minutes or less, or 5 minutes or less, or 2 minutes or less) an effective amount of the composition comprising the hydrophobic drug derivative (*e.g*., at about 5 to about 300 mg/m², such as about 100 to about 150 mg/m²) and the carrier protein (i.e., albumin). In any of these embodiments, the method is conducted at an interval of once per month, or once per three weeks, or once per two weeks, or once per week.

In some embodiments, the invention provides a composition of the invention for use in a method of treating cancer in an individual, wherein the method comprises parenterally administering to the individual (*e.g*., a human) an effective amount of the composition of the invention. The invention also provides a composition of the invention for use in a method of treating cancer in an individual, wherein the method comprises intravenous, intra-arterial, intramuscular, subcutaneous, inhalation, oral, intraperitoneal, nasally, or intra-tracheal administering to the individual (*e.g*., a human) an effective amount of the composition of the invention. In some embodiments, the route of administration is intraperitoneal. In some embodiments, the route of administration is intravenous, intra-arterial, intramuscular, or subcutaneous. In various variations, about 5 mg to about 500 mg, such as about 30 mg to about 300 mg or about 50 to about 500 mg, of the hydrophobic taxane derivative is administered per dose. In some embodiments, the hydrophobic taxane derivative is the only pharmaceutically active agent for the treatment of cancer that is contained in the composition.

Any of the compositions described herein can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some embodiments, sustained continuous release formulation of the composition may be used. In one variation of the invention, nanoparticles (such as albumin nanoparticles) of the inventive compounds can be administered by any acceptable route including, but not limited to, orally, intramuscularly, transdermally, intravenously, through an inhaler or other air borne delivery systems and the like.

In some embodiments, drug-containing nanoparticle compositions may be administered with a second therapeutic compound and/or a second therapy. The dosing frequency of the composition and the second compound may be adjusted over the course of the treatment based on the judgment of the administering physician. In some embodiments, the first and second therapies are administered simultaneously, sequentially, or concurrently. When administered separately, the nanoparticle composition (e.g., a hydrophobic taxane derivative-containing nanoparticle composition) and the second compound can be administered at different dosing frequency or intervals. For example, the composition can be administered weekly, while a second compound can be administered more or less frequently. In some embodiments, sustained continuous release formulation of hydrophobic taxane derivative-containing nanoparticle and/or second compound may be used. Various formulations and devices for achieving sustained release are known in the art. A combination of the administration configurations described herein can be used.

### Metronomic Therapy Regimes

Also disclosed are metronomic therapy regimes for any of the methods of treatment and methods of administration described herein. Exemplary metronomic therapy regimes and variations for the use of metronomic therapy regimes are discussed below and disclosed in U.S.S.N. 11/359,286, filed 2/21/2006, published as U.S. Pub. No. 2006/0263434 (such as those described in paragraphs [0138] to [0157] therein). In some embodiments, the nanoparticle composition is administered over a period of at least one month, wherein the interval between each administration is no more than about a week, and wherein the dose of the hydrophobic taxane derivative at each administration is about 0.25% to about 25% of its maximum tolerated dose following a traditional dosing regime. In some embodiments, the nanoparticle composition is administered over a period of at least two months, wherein the interval between each administration is no more than about a week, and wherein the dose of the drug at each administration is about 1% to about 20% of its maximum tolerated dose following a traditional dosing regime. In some embodiments, the dose of the hydrophobic taxane derivative per administration is less than about any one of 25%, 24%, 23%, 22%, 20%, 18%, 15%, 14%, 13%, 12%, 11%, 10%, 9%, 8%, 7%, 6%, 5%, 4%, 3%, 2%, or 1% of the maximum tolerated dose. In some embodiments, any nanoparticle composition is administered at least about any one of 1x, 2x, 3x, 4x, 5x, 6x, or 7x (*i.e*., daily) a week. In some embodiments, the intervals between each administration are less than about any one of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any one of 1 month, 2 months, 3 months, 4 months, 5 months, 6 months, 8 months, or 12 months. In some embodiments, the composition is administered over a period of at least about any one of 2, 3, 4, 5, 6, 7, 8, 9, 10, 11, 12, 18, 24, 30, 36, 48, 60, 72, or 84 months.

### Examples

The examples, which are intended to be purely exemplary of the invention and should therefore not be considered to limit the invention in any way, also describe and detail aspects and variations of the invention discussed above. Efforts have been made to ensure accuracy with respect to numbers used (for example, amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Reference Example 1: Preparation of 2'-benzoyl-docetaxel (1)

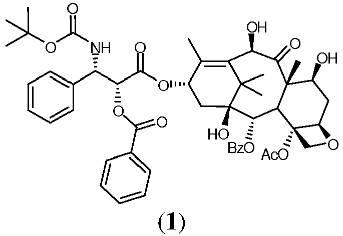

To a solution of docetaxel (201 mg, 0.25 mmol) in methylene chloride (6 mL) was added triethylamine (42 µL, 0.30 mmol), followed by benzoyl chloride (29 µL, 0.25 mmol) at 0 °C. The mixture was stirred at room temperature for 2 h, upon which TLC indicated the disappearance of the starting material. After quenched with adding saturated sodium bicarbonate solution, the mixture was extracted by ethyl ether. The organic layers were washed by brine, dried over anhydrous magnesium sulfate, filtered, and concentrated *in vacuo.* The residue was purified by flash silica gel column chromatography (hexane : DCM, 1 : 1) to afford the product as a white foam (181 mg, 80%). ¹H NMR (CDCl₃, 500 MHz): δ 8.10 (d, J = 7.5 Hz, 2H), 7.98 (d, J = 7.6 Hz, 2H), 7.61 (t, J = 7.4 Hz, 1H), 7.50 (t, J = 7.9 Hz, 2H), 7.45 (t, J = 7.8 Hz, 2H), 7.41-7.36 (m, 4H), 7.29-7.26 (m, 1H), 6.25 (t, J = 8.6 Hz, 1H), 5.67 (d, J = 7.0 Hz, 1H), 5.58-5.45 (m, 3H), 5.22 (s, 1H), 4.94 (dd, J = 9.6, 1.9 Hz, 1H), 4.31 (d, J = 8.5 Hz, 1H), 4.27 (dd, J = 10.9, 6.6 Hz, 1H), 4.19 (s, 1H), 4.18 (d, J = 8.5 Hz, 1H), 3.93 (d, J = 6.9 Hz, 1H), 2.60-2.58 (m, 1H), 2.43 (s, 3H), 2.32-2.25 (m, 1H), 2.17 (s, 3H), 2.15-2.05 (m, 1H), 1.98 (s, 3H), 1.88-1.80 (m, 1H), 1.75 (s, 3H), 1.34 (s, 9H), 1.22 (s, 3H), 1.11 (s, 3H). ESI-MS: calcd. for C₅₀H₅₇NO₁₅Na (M+Na)⁺: 934. Found: 934.

### Example 2: Preparation of 2'-hexanoyl docetaxel (2)

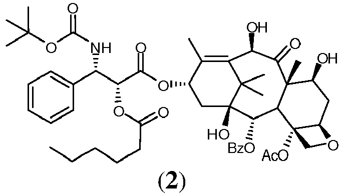

To a solution of docetaxel (2.20 g, 2.72 mmol) in methylene chloride (220 mL) was added triethylamine (0.95 ml, 6.80 mmol), followed by hexanoyl chloride (0.38 mL, 2.72 mmol) at 0 °C. The mixture was stirred at 0 °C for 1.5 h, upon which TLC indicated the disappearance of the starting material. After quenched with adding saturated sodium bicarbonate solution, the mixture was extracted by methylene chloride. The organic layers were washed by brine, dried over anhydrous sodium sulfate, filtered, and concentrated *in vacuo.* The residue was purified by flash silica gel column chromatography (10-50% ethyl acetate in hexanes) to afford the product as white solids (2.00 g, 81%). ¹H NMR (CDCl₃, 500 MHz): δ 8.10 (d, J = 7.3 Hz, 2H), 7.61 (t, J = 7.4 Hz, 1H), 7.50 (t, J = 7.9 Hz, 2H), 7.38 (t, J = 7.4 Hz, 2H), 7.30 (m, 3H), 6.25 (t, J = 8.6 Hz, 1H), 5.69 (d, J = 7.1 Hz, 1H), 5.46-5.37 (m, 3H), 5.21 (s, 1H), 4.96 (dd, J = 7.7, 2.0 Hz, 1H), 4.32 (d, J = 8.5 Hz, 1H), 4.27 (dd, J = 10.9, 6.6 Hz, 1H), 4.19 (d, J = 8.5 Hz, 2H), 3.93 (d, J = 7.2 Hz, 1H), 2.60-2.58 (m, 1H), 2.44 (s, 3H), 2.39-2.30 (m, 3H), 2.29-2.20 (m, 1H), 2.07 (s, 3H), 1.96-1.85 (m, 1H), 1.75 (s, 3H), 1.57-1.53 (m, 4H), 1.33 (s, 9H), 1.23 (s, 3H), 1.26-1.17 (m, 4H), 1.12 (s, 3H), 0.85 (t, J = 7.1 Hz, 3H). ESI-MS: calcd. for C49H63NO15Na (M+Na)⁺: 929. Found: 929.

### Reference Example 3: Preparation of 2'-decanoyl-docetaxel (3)

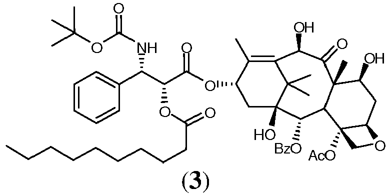

To a solution of docetaxel (144 mg, 0.18 mmol) in methylene chloride (10 mL) was added triethylamine (134 µL, 0.96 mmol), followed by decanoyl chloride (37 µL, 0.18 mmol) at 0 °C. The mixture was stirred at 0 °C for 4.5 h, upon which TLC indicated the disappearance of the starting material. After quenched with adding saturated sodium bicarbonate solution, the mixture was extracted by methylene chloride. The organic layers were washed by brine, dried over anhydrous sodium sulfate, filtered, and concentrated *in vacuo.* The residue was purified by flash silica gel column chromatography (10-50% ethyl acetate in hexanes) to afford the product as white solids (112 mg, 65%). ¹H NMR (CDCl₃, 500 MHz): δ 8.10 (d, J = 7.3 Hz, 2H), 7.61 (t, J = 7.4 Hz, 1H), 7.50 (t, J = 7.9 Hz, 2H), 7.38 (t, J = 7.4 Hz, 2H), 7.30 (m, 3H), 6.25 (t, J = 8.6 Hz, 1H), 5.69 (d, J = 7.1 Hz, 1H), 5.46-5.37 (m, 3H), 5.21 (s, 1H), 4.96 (d, J = 7.7 Hz, 1H), 4.32 (d, J = 8.5 Hz, 1H), 4.27 (m, 1H), 4.19 (m, 2H), 3.93 (d, J = 7.2 Hz, 1H), 2.60-2.58 (m, 1H), 2.44 (s, 3H), 2.39-2.30 (m, 3H), 2.29-2.20 (m, 1H), 2.07 (s, 3H), 1.96-1.85 (m, 1H), 1.75 (s, 3H), 1.57-1.45 (m, 4H), 1.33 (s, 9H), 1.23 (s, 3H), 1.26-1.21 (m, 12H), 1.12 (s, 3H), 0.85 (t, J = 7.1 Hz, 3H).

### Reference Example 4: Preparation of 2'-valeryl-docetaxel (4)

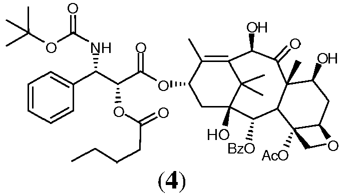

To a solution of docetaxel (144 mg, 0.18 mmol) in methylene chloride (10 mL) was added triethylamine (100 µL, 0.72 mmol), followed by valeryl chloride (44 µL, 0.18 mmol) at 0 °C. The mixture was stirred at 0 °C for 5.5 h, upon which TLC indicated the disappearance of the starting material. After quenched with adding saturated sodium bicarbonate solution, the mixture was extracted by methylene chloride. The organic layers were washed by brine, dried over anhydrous sodium sulfate, filtered, and concentrated *in vacuo.* The residue was purified by flash silica gel column chromatography (10-50% ethyl acetate in hexanes) to afford the product as white solids (100 mg, 62%). ¹H NMR (CDCl₃, 500 MHz): δ 8.10 (d, J = 7.3 Hz, 2H), 7.61 (t, J = 7.4 Hz, 1H), 7.50 (t, J = 7.9 Hz, 2H), 7.38 (t, J = 7.4 Hz, 2H), 7.30 (m, 3H), 6.25 (t, J = 8.6 Hz, 1H), 5.69 (d, J = 7.1 Hz, 1H), 5.46-5.37 (m, 3H), 5.21 (s, 1H), 4.96 (d, J = 7.7 Hz, 1H), 4.32 (d, J = 8.5 Hz, 1H), 4.27 (m, 1H), 4.19 (m, 2H), 3.93 (d, J = 7.2 Hz, 1H), 2.60-2.58 (m, 1H), 2.44 (s, 3H), 2.39-2.30 (m, 3H), 2.29-2.20 (m, 1H), 2.04 (s, 3H), 1.96-1.85 (m, 1H), 1.75 (s, 3H), 1.57-1.45 (m, 4H), 1.33 (s, 9H), 1.23 (s, 3H), 1.26-1.21 (m, 2H), 1.12 (s, 3H), 0.85 (t, J = 7.1 Hz, 3H).

### Reference Example 5: Preparation of 2'-propionyl-docetaxel (5)

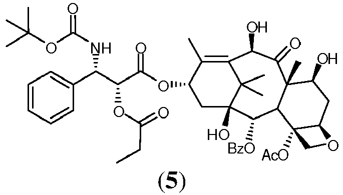

To a solution of docetaxel (195 mg, 0.24 mmol) in methylene chloride (10 mL) was added triethylamine (100 µL, 0.72 mmol), followed by propionyl chloride (20.8 µL, 0.24 mmol) at 0 °C. The mixture was stirred at 0 °C for 5 h, upon which TLC indicated the disappearance of the starting material. After quenched with adding saturated sodium bicarbonate solution, the mixture was extracted by methylene chloride. The organic layers were washed by brine, dried over anhydrous sodium sulfate, filtered, and concentrated *in vacuo.* The residue was purified by flash silica gel column chromatography (10-50% ethyl acetate in hexanes) to afford the product as white solids (100 mg, 48%). ¹H NMR (CDCl₃, 500 MHz): δ 8.10 (d, J = 7.3 Hz, 2H), 7.61 (t, J = 7.4 Hz, 1H), 7.50 (t, J = 7.9 Hz, 2H), 7.38 (t, J = 7.4 Hz, 2H), 7.30 (m, 3H), 6.25 (t, J = 8.6 Hz, 1H), 5.69 (d, J = 7.1 Hz, 1H), 5.46-5.37 (m, 3H), 5.21 (s, 1H), 4.96 (d, J = 7.7 Hz, 1H), 4.32 (d, J = 8.5 Hz, 1H), 4.27 (m, 1H), 4.19 (m, 2H), 3.93 (d, J = 7.2 Hz, 1H), 2.60-2.58 (m, 1H), 2.44 (s, 3H), 2.39-2.30 (m, 3H), 2.29-2.20 (m, 1H), 2.04 (s, 3H), 1.96-1.85 (m, 1H), 1.75 (s, 3H), 1.57-1.45 (m, 2H), 1.33 (s, 9H), 1.23 (s, 3H), 1.12 (s, 3H), 1.10 (t, J = 7.1 Hz, 3H).

### Reference Example 6: Preparation of 2'-benzoyl-paclitaxel (6)

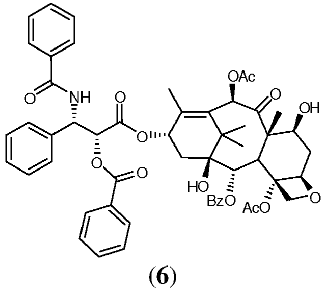

To a solution of docetaxel (502 mg, 0.62 mmol) in methylene chloride (5 mL) at 0 °C was added triethylamine (104 µL, 0.74 mmol), followed by benzoyl chloride (72 µL, 0.62 mmol). The mixture was stirred at room temperature for 2 h, upon which TLC indicated the disappearance of the starting material. After quenched with adding saturated sodium bicarbonate solution, the mixture was extracted by ethyl ether. The organic layers were washed by brine, dried over anhydrous magnesium sulfate, filtered, and concentrated *in vacuo.* The residue was purified by flash silica gel column chromatography (hexane/methylene chloride, 1/1) to afford the product as a white foam (531 mg, 94 %). ¹H NMR (CDCl₃, 500 MHz): δ 8.10 (d, *J* = 7.6 Hz, 2H), 7.99 (d, *J* = 7.5 Hz, 2H), 7.76 (d, *J* = 7.1 Hz, 2H), 7.62-7.56 (m, 2H), 7.55-7.39 (m, 13H), 7.32 (d, *J* = 7.1 Hz, 1H), 7.05 (d, *J* = 9.0 Hz, 1H), 6.30 (s, 1H), 6.25 (t, *J* = 8.6 Hz, 1H), 6.04 (dd, *J* = 8.9, 3.8 Hz, 1H), 5.68 (d, *J* = 3.8 Hz, 1H), 5.67 (d, *J* = 7.6 Hz, 1H), 4.94 (dd, *J* = 9.7, 1.8 Hz, 1H), 4.46 (dd, *J* = 10.9, 6.6 Hz, 1H), 4.30 (d, *J* = 8.4 Hz, 1H), 4.19 (d, *J* = 8.5 Hz, 1H), 3.81 (d, *J* = 7.1 Hz, 1H), 2.56-2.48 (m, 1H), 2.43 (s, 3H), 2.36-2.31 (m, 1H), 2.23 (s, 3H), 2.17-2.12 (m, 1H), 1.96 (d, *J* = 0.8 Hz, 3H), 1.91-1.85 (m, 1H), 1.67 (s, 3H), 1.24 (s, 3H), 1.13 (s, 3H). ESI-MS: calcd. for C₅₄H₅₅NO₁₅Na (M+Na)⁺: 980. Found: 980.

### Reference Example 7: Preparation of 7-benzoyl-docetaxel (7)

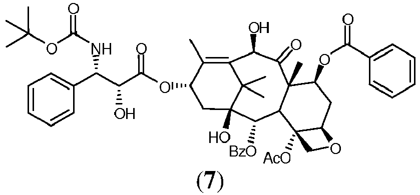

A solution of docetaxel in dichloromethane is mixed at room temperature under argon with imidazole and triethylsilyl chloride. The reaction mixture is stirred at room temperature, diluted with methylene chloride, washed with water, saturated aqueous sodium chloride, dried, and concentrated. The flash chromatography of the residue produces 2'-triethylsilyl docetaxel. A solution of 2'-triethylsilyl docetaxel in methylene chloride is mixed at ambient temperature under argon with pyridine and benzoyl chloride. The reaction mixture is stirred at room temperature, diluted with ether, and the organic layers are concentrated. The flash chromatography of the residue is performed to produce intermediate 2'-triethylsilyl 7-benzoyl docetaxel.

A solution of intermediate 2'-triethylsilyl 7-benzoyl docetaxel in methanol at 0 °C under argon is mixed with aqueous HCl and the reaction mixture is stirred at the same temperature. After dilution with ethyl ether and saturated sodium bicarbonate, the organic layers were washed by brine, dried over anhydrous magnesium sulfate, filtered, and concentrated *in vacuo.* The crude product was purified by flash silica gel column chromatography to produce 7-benzoyl docetaxel.

### Reference Example 8: Preparation of 10-benzoyl docetaxel (8)

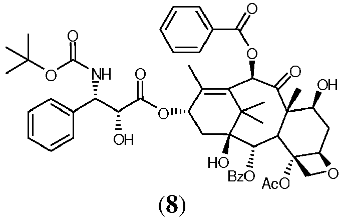

A solution of docetaxel in dichloromethane and pyridine is mixed at room temperature under argon with triethylsilyl chloride. The reaction mixture is stirred at room temperature, diluted with methylene chloride, washed with water, saturated aqueous sodium chloride, dried, and concentrated. The flash chromatography of the residue produces 2',7-bis(triethylsilyl)-docetaxel.

A solution of 2',7-bis(triethylsilyl)-docetaxel in methylene chloride is first mixed at room temperature under argon with sodium hydride at 0 °C, then benzoyl chloride was added. The reaction mixture is stirred at room temperature, diluted with ether, and the organic layers are concentrated. The flash chromatography of the residue is performed to produce intermediate 2',7-bis(triethylsilyl)-10-benzoyl docetaxel.

A solution of intermediate 2',7-bis(triethylsilyl)-10-benzoyl docetaxel in methanol at 0 °C under argon is mixed with aqueous HCl and the reaction mixture is stirred at the room temperature. After dilution with ethyl ether and saturated sodium bicarbonate, the organic layers were washed by brine, dried over anhydrous magnesium sulfate, filtered, and concentrated *in vacuo.* The crude product was purified by flash silica gel column chromatography to produce 10-benzoyl docetaxel.

### Example 9: In vitro growth inhibition for on MX-1 (human breast carcinoma) cells

A cytotoxicity assay was quantitated using the Promega CellTiter Blue Cell Viability Assay. Briefly, cells (5000 cells/well) were plated onto 96-well microtiter plates in RPMI 1640 medium supplemented with 10% FBS and incubated at 37 °C in a humidified 5% CO₂ atmosphere. After 24 hr., cells were exposed to various concentrations of hydrophobic taxane derivative in DMSO and cultured for another 72 hr. 100 uL of media were removed and 20 uL of Promega CellTiter Blue reagent were added to each well and shaken to mix. After 4 hours of incubation at 37 °C. in a humidified 5% CO₂ atmosphere, the plates were read at 544ex/620em. The fluorescence produced is proportional to the number of viable cells. After plotting fluorescence produced against drug concentration, the IC₅₀ was calculated as the half-life of the resulting non-linear regression. The data is presented in Table 1.

**Table 1: Cytotoxicity of hydrophobic docetaxel derivatives**

| | | |
|---|---|---|
| 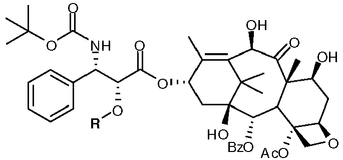 | | |

| **ID** | **R** | **IC50(MX-1) (µM)** |
|---|---|---|
| 2 | -CO(CH₂)₄CH₃ | 103 |
| ∗ 3 | -CO(CH₂)₈CH₃ | 8.8 |
| ∗ 4 | -CO(CH₂)₃CH₃ | 211 |
| ∗ 5 | -COCH₂CH₃ | 209 |
| ∗ 1 | -COPh | 38.4 |

| | | |
|---|---|---|
| Compounds in Table 1 marked with "^{∗}" are reference compounds. | | |

### Example 10: Conversion of hydrophobic docetaxel derivatives to docetaxel in human liver microsome

### Sample preparation and incubation

The drug stock solutions were made up to 5 mg/mL in DMSO and used the same day. For control solutions containing no microsome, the drug stock solution was spiked into the following incubation mixture: 83mM K₂HPO₄ buffer at pH 7.4, 13.3mM MgCl₂, NADPH regenerating system (NRS) containing 1.3mM NADP+, 3.3mM glucose-6-phosphate, 0.4 U/mL glucose-6-phosphate dehydrogenase, and 0.05mM sodium citrate to give a final drug concentration of 50 ug/mL with 1% DMSO. For control solutions containing no NADPH regenerating system, the drug stock solution was spiked into the following incubation mixture: 84mM K₂HPO₄ buffer at pH 7.4, 10mM MgCl₂, 12.5 mM sucrose, and 1 mg/mL human liver microsome (HLM) to give a final drug concentration of 50 ug/mL with 1% DMSO. For the active solutions, the drug stock solution was spiked into the following incubation mixture: 78mM K₂HPO₄ buffer at pH 7.4, 13.3mM MgCl₂, NADPH regenerating system (NRS) containing 1.3mM NADP+, 3.3mM glucose-6-phosphate, 0.4 U/mL glucose-6-phosphate dehydrogenase, 0.05mM sodium citrate, 12.5 mM sucrose, and 1 mg/mL HLM to give a final drug concentration of 50 ug/mL with 1% DMSO. The enzymatic reactions were initiated with the addition of HLM.

The control and active solutions were incubated in the Thermomixer for ∼5 minutes prior to spiking in HLM to initiate the reaction. The total sample volume was between 1 to 2.5 mL. Aliquots of the control and active solutions were retrieved at various time points for HPLC analysis. Prior to retrieving samples were briefly vortexed by flicking the vial to improve homogeneity of the solution.

Upon sample retrieving, the reaction aliquots were immediately diluted 1:2 with acetonitrile (ACN) to precipitate the proteins and quench the enzymatic reaction. Samples were vortexed and centrifuged at 14,000 rpm for 8 minutes. The supernatant was transferred to 1 mL auto sampler vials and injected into the HPLC.

### HPLC conditions

HPLC separation was achieved using a Synergi Fusion-RP column (Phenomenex, 150 x 4.6mm, 80A, 4 micron) and the following mobile phase gradient: mobile phase A: water; mobile phase B: acetonitrile; start with A/B (50:50) from 0 to 10 minute; go to A/B (10:90) from 10 to 30 minute; hold at A/B (10:90) from 30 to 40 minute; go back to A/B (50:50) at 40 minute; stop the run at 50 minute. Flow rate was 1 mL/min. Detection was at 228 nm. Oven temperature was kept at 35°C. Sample injection volume was 20 uL. HPLC retention time for various hydrophobic taxane derivatives are summarized in Table 2.

**Table 2: HPLC retention times of hydrophobic taxane derivatives**

| | | | | |
|---|---|---|---|---|
| 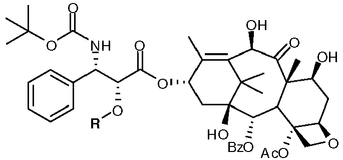 | | | | |

| **ID** | **R** | **HPLC retention time (min)** | **Relative Retention time (wrt docetaxel)** | **Log P** |
|---|---|---|---|---|
| 2 | -CO(CH₂)₄CH₃ | 23.0 | 2.77 | 6.44 |
| ∗ 3 | -CO(CH₂)₈CH₃ | 29.3 | 3.53 | 8.64 |
| ∗ 4 | -CO(CH₂)₃CH₃ | 21.2 | 2.55 | 6.37 |
| ∗ 5 | -COCH₂CH₃ | 16.9 | 2.04 | 5.31 |
| ∗ 1 | -COPh | 20.4 | 2.46 | 6.67 |
| ∗ Docetaxel | H | 8.3 | 1 | 4.20 |

| | | | | |
|---|---|---|---|---|
| Compounds in Table 2 marked with "^{∗}" are reference compounds. | | | | |

### Results

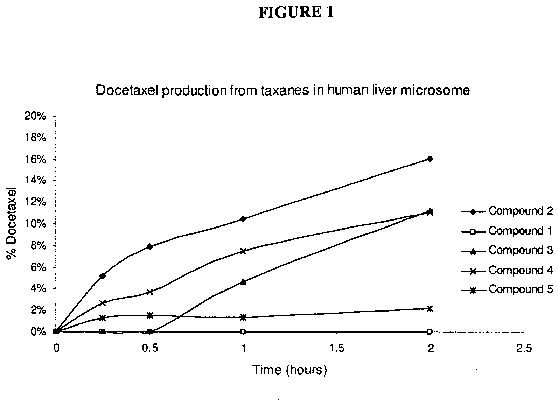

The production of docetaxel by the *in vitro* metabolism of the hydrophobic taxane derivatives in human liver microsome was determined by the relative percent peak area of docetaxel detected in the HPLC chromatograms. Docetaxel produced versus incubation time is plotted for each hydrophobic taxane derivative in Figure 1. The comparison of the plots indicates that the production of docetaxel was dependent on the structure of the hydrophobic taxane derivatives. There was no docetaxel produced in the hydrophobic taxane derivative with a benzoyl substitution on docetaxel side chain (compound-**1**). However, significant amount of docetaxel was produced in the hydrophobic taxane derivatives with alkyl substitution on docetaxel side chain. The length of the alkyl side chain cannot be correlated with the percent docetaxel produced, instead docetaxel production upon 2 hours incubation in human liver microsome was most significant (∼16%) in compound-**2** with a C6 side chain followed by compound-**4** (C5) and compound-**3** (C9) with very similar conversion at ∼11%. Much less docetaxel was produced by compound-**5** which has a shorter (C3) side chain. It was surprising that compound-**2** was metabolized and produced the most docetaxel. This could not have been predicted on the basis of the side-chain substitutions.

The dependence of the docetaxel production on the structure of the hydrophobic taxane derivatives could be related to the ability of the R side chain to fit within the active site of the enzyme responsible for the hydrolysis reaction. Without being bound by theory, among these five docetaxel hydrophobic taxane derivatives, compound-**2** containing a C6 alkyl substitution may stereochemically fit the best into the hydrophobic pockets at the active site of the enzyme. The rigid nature of the benzoyl group may prevent it from accessing the hydrophobic pocket altogether, or the different reactivity of this aromatic ester may prevent the enzymatic hydrolysis reaction from taking place.

### Reference Example 11: Preparation of nanoparticles of 2'-benzoyl docetaxel and albumin by high pressure homogenization

48.5 mg of 2'-benzoyl docetaxel (prepared in Example 1) was dissolved in 0.56 mL of chloroform-t-butyl alcohol mixture (10.2 : 1). The solution was added to 10.0 mL of human serum albumin solution (5 %, w/v). The mixture was pre-homogenized for 5 minutes at 10,000 rpm (VirTis homogenizer, model:Tempest I.Q.) in order to form a crude emulsion, and then transferred into a high pressure homogenizer (Avestin). The tip of the pre-homogenizer's rotor/stator assembly and the container of emulsion were washed with 3.0 mL of water and the washings were transferred into the high pressure homogenizer (Avestin). The emulsification was performed at 18,000-20,000 psi while recycling the emulsion for 3-12 passes. The resulting system was transferred into a Rotary evaporator, and chloroform and t-butyl alcohol were rapidly removed at 40°C, at reduced pressure (40 mm of Hg), for 10 minutes. The resulting dispersion was translucent, and the typical diameter of the resulting nanoparticles was found to be 121.7 ± 1.4 nm (Z-average, Malvern Zetasizer). The dispersion was directly filterable through 0.22 µm syringe filter (Costar, µstar, 8110).

The dispersion was further lyophilized for 48 hours optionally with or without adding any cryoprotectant or lyoprotectant. The resulting cake could be easily reconstituted to the original dispersion by addition of sterile water or saline. The particle size after reconstitution was the same as before lyophilization.

### Example 12: Preparation of nanoparticles of 2'-O-hexanoyldocetaxel and albumin by high pressure homogenization

64.9 mg of 2'-O-hexanoyldocetaxel was dissolved in 0.56 mL of chloroform-t-butanol (10.2:1,v/v). The solution was then added to 15 mL of 5 % (w/v) HSA solution. The mixture was pre-homogenized for 5 minutes at 10,000 rpm (Vitris homogenizer model Tempest I.Q.) in order to form a crude emulsion and then transferred into a high pressure homogenizer (Avestin). The tip of the pre-homogenizer's rotor/stator assembly and the container of emulsion were washed with 3.0 mL of 5 % (w/v) HSA solution and the washings were transferred into the high pressure homogenizer (Avestin). The emulsification was performed at 18,000-20,000 psi while recycling the emulsion for 3-12 passes. The resulting system was transferred into a Rotary evaporator, and chloroform and t-butyl alcohol were rapidly removed at 40 °C, at reduced pressure (40 mm of Hg), for 10 minutes. The resulting suspension was made to 20 mL using WFI and then characterized microscopically and size measurement. Microscopically the suspension size was so small that it was difficult to observe the particles. The suspension was filterable through 0.8 µm and the size of the filtered composition was 95 nm.

### Example 13.1: Preparation of pharmaceutical formulations comprising 2'-O-hexanoyldocetaxel and albumin

∼100 mL of 0.8 µm of the composition made in four separate batches by the method described as in Example 12 was filtered through 0.45 µm 1000 mL-size Steri-cup. The entire composition filtered through one of the above filters. The filtered composition was transferred to 20-mL serum vials with a fill volume of 5 mL and lyophilized following a protocol which is essentially primary drying at 25 °C for 840 mins and secondary drying at 30 °C for 480 mins. This resulted in a good cake of white to off-white color. The lyophilized cake was reconstitutable in less than 2 mins with 0.9 % (w/v) saline solution to a bluish translucent solution. The size of particles was 107 nm. This reconstituted composition maintained its integrity for 24 h at 4 °C. After 24h storage at 4 °C, the size of the particles was 108 nm and there was no appreciable change in size distribution.

### Example 13.2: Preparation of pharmaceutical formulations comprising 2'-O-hexanoyldocetaxel and albumin

The additives in this study were selected from the injectable excipients namely, tonicity modifiers, NaCl and d-mannitol. ∼20 mL of 0.8 µm of the composition made by the method described as in Example 12 was filtered through 0.45 µm syringe filter. The 0.45 µm filtered compositions were divided into two separate portions each of 20 mL. To one portion d-mannitol was added to a concentration of 5 % (w/v) and to the other portion NaCl was added to have a conc. of 150 mM. The d-mannitol and sodium chloride containing compositions were transferred to 20-mL serum vials with a fill volume of 5 mL and lyophilized following a protocol which is essentially primary drying at 25 °C for 840 mins and secondary drying at 30 °C for 480 mins. This resulted in a good cake of white to off-white color. The lyophilized cake was reconstitutable in less than 2 mins with WFI to a bluish translucent solution. The reconstituted composition maintained its integrity for 24 h at 4 °C. There was no appreciable change in size and size distribution before and after lyophilization and on storage.

### Example 13.3: Preparation of pharmaceutical formulations comprising 2'-O-hexanoyldocetaxel and albumin

This example demonstrates the preparation of pharmaceutical formulations comprising 2'-O-hexanoyldocetaxel and albumin where the composition particle size is less than 100 nm and the filterability and recovery are high. Eight batches of the compositions were prepared at the following parameters and their characteristics were put together in the Tabulated form (Table 3). Particle size distribution is shown, for example, in Figure 8.

HSA conc. = 5 % (w/v); Chloroform:t-Butyl alcohol = 10.2 :1; % organic solvent = 3.6; Batch size = 22.5 mL; HSA:Drug = 9-10

**Table 3: Batch preparation of 2'-O-hexanoyldocetaxel/albumin compositions**

| **Batch name** | **Amt. of drug, mg** | **Filtered (0.45 µm) Zₐᵥ (nm)** |
|---|---|---|
| 1 | 127.6 | 70.8 |
| 2 | 122.9 | 67.4 |
| 3 | 121.3 | 67.6 |
| 4 | 121.4 | 68.0 |
| 5 | 123.7 | 66.9 |
| 6 | 121.5 | 73.7 |
| 7 | 122.6 | 74.4 |
| 8 | 121.7 | 71.8 |

All the 0.45 µm filtered compositions in Table 3 were mixed together. The total volume of the mixed composition was ∼150 mL and could be filtered through one 250-mL size Steri-cup (0.22 µm pore size). All these compositions were put in 34 of 20-mL serum vials with 4 mL fill volume. The compositions were lyophilized and reconstituted with no appreciable change in particle size.

### Example 14: Nanoparticle stability

The stability of the lyophilized 2'-O-hexanoyldocetaxel/albumin compositions were assessed upon storage at 2-8°C, RT and 40°C to establish storage temperature/shelf-life and to identify possible degradation products under accelerated conditions for establishing handling and shipping protocols. Reconstitution stability at 2-8°C and RT is also performed to establish in-use shelf-life. Visual observation, reconstitution time, pH, RP-HPLC (for potency and % degradation), particle size by Malvern Nanosizer were measured to ascertain the integrity and stability of albumin containing formulation. The formulation was found to be stable for 3 months in terms of cake appearance, reconstitutability, size and size distribution. Results of this study are shown in Table 4.

**Table 4: 2'-O-hexanoyldocetaxel/albumin compositions storage characteristics**

| **Time point** | **Temp.** | **Cake details** | **Recon. soln.** | **Recon. vol. (mL)** | **Recon. time (min)** | **pH** | **Zₐᵥ (nm)** | **Visual** | **Microscope** |
|---|---|---|---|---|---|---|---|---|---|
| 0 | RT | Off-white, good cake | 0.9 % (w/v) NaCl | 2.0 | 3.1 | 6.60 | 68.7 | No visible particles | Particles to small to observe |
| 1 month | -20 °C | As above | As above | 2.0 | 2.8 | 6.62 | 67.2 | No visible particles | Particles to small to observe |
| | 2-8 °C | As above | As above | 2.0 | 2.6 | 6.63 | 68.8 | No visible particles | Particles to small to observe |
| | 25 °C | As above | As above | 2.0 | 3.2 | 6.64 | 68.6 | No visible particles | Particles to small to observe |
| | 40 °C | As above | As above | 2.0 | 3.8 | 6.61 | 82.6 | No visible particles | Particles to small to observe |

### Example 15: Preparation of nanoparticles of 2'-benzoypaclitaxel by high pressure homogenization

57.6 mg of 2'-benzoylpaclitaxel was dissolved in 0.6 mL of chloroform-ethyl alcohol mixture (9:1, v/v). The solution was added to 12.0 mL of human serum albumin solution (3 %, w/v). The mixture was pre-homogenized for 5 minutes at 10000 rpm in order to form a crude emulsion, and then transferred into a high pressure homogenizer. The emulsification was performed at 18,000-20,000 psi while recycling the emulsion for 3- 10 passes. The homogenized emulsion was transferred in a 500 mL flask of a rotary evaporator, and chloroform and ethyl alcohol were rapidly removed at 40°C, at reduced pressure (40 mm of Hg) for 20 minutes. The resulting dispersion was a bluish translucent solution. The diameter of the resulting 2'-benzoypaclitaxel nanoparticles was found to be 86.7 ± 3.1 nm (Z-average, Malvern Zetasizer). The dispersion was directly filterable through 0.22 µm syringe filter (Costar,µstar, 8110) and the size of the nanoparticles was 61.1 ± 0.2 nm. The dispersion was further lyophilized optionally with or without adding any cryoprotectant or lyoprotectant. The resulting cake could be easily reconstituted to the original dispersion by addition of sterile water or saline. The particle size after reconstitution was the same as before lyophilization.

### Example 16: Maximum tolerated dose (MTD) of pharmaceutical formulations comprising 2'-O-hexanoyldocetaxel and albumin

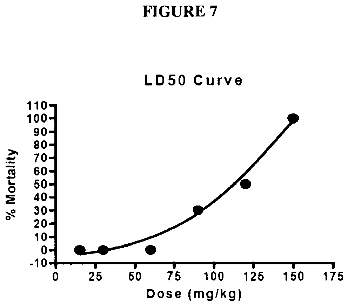

Mice were intravenously administered with saline (control) or nab-**2** (nanoparticles of 2'-hexanoyldocetaxel prepared in example 12) at 15, 30, 60, 90, 120, and 150 mg/kg (q4dx3) on days 1, 5, and 9. Mortality versus dose was fitted using a sigmoidal equation and shown in Figure 7. The Nab-2 formulation was well tolerated with LD₁₀=61 mg/kg and LD₅₀=113 mg/kg on q4dx3 schedule.

### Example 17: Anticancer activity of nab-2 (nanoparticles of 2'-hexanoyldocetaxel prepared in example 12) against breast cancer xenograft

Female nude mice were implanted with 10 million MDA-MB-231 cells s.c. near the right flank. Eleven days later, tumor-bearing mice (mean tumor size = 126 mm³) in groups of ten were treated with saline or varying doses (60, 90, and 120 mg/kg) of Nab-2 (prepared in example 12) or 15 mg/kg of Taxotere® administered i.v. on a q4d×3 schedule. Tumor measurements and animal weights were recorded twice weekly.

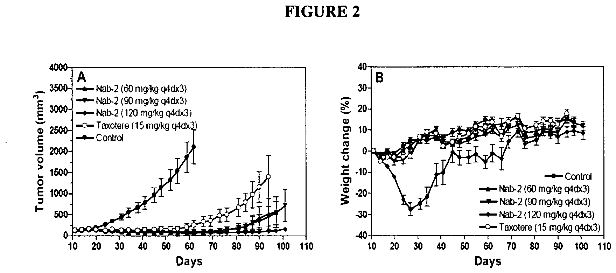

In the MDA-MB-231 breast cancer model, the tumors in the treated control animals grew well to the evaluation size in nine of ten mice with a median time to reach one tumor doubling of 12.2 days (Figure 2A; values are mean ± SEM). A maximum average weight loss of 1.3% was observed (Figures 2B values are mean ± SEM). Treatment with Nab-**2** effectively delayed the growth of the MDA-MB-231 human mammary tumor with T-C values of 83.1, 80.7, and >84.8 days at dosages of 120, 90, and 60 mg/kg/injection, respectively, with >96% tumor growth inhibition compared to vehicle control (*P* < 0.0001 *vs*. saline control, ANOVA statistic). Nab-**2** was not tolerated at the highest dosage tested, 120 mg/kg/injection and caused 50% mortality despite marked tumor growth inhibition. Treatment with Nab-**2** at dosages of 90 and 60 mg/kg/injection was well-tolerated with maximum average weight losses of 5% and 3%, respectively. In the MDA-MB-231 model, Taxotere® at a dosage of 15 mg/kg/injection was tolerated with a maximum average weight loss of 3.3%. Taxotere® delayed the growth of the MDA-MB-231 mammary tumor with a T-C value of 49.5 days and decreased tumor growth by 88% (*P* < 0.0001 *vs*. saline control). There was no significant difference in tumor growth inhibition between Taxotere® and Nab-**2** treated animals, however, at the equitoxic dose level superior anti-tumor efficacy with 30-40% tumor regression was achieved with Nab-**2** (see Table 5).

**Table 5: Anti-tumor activity of Nab-2 compared with Taxotere® in the s.c. human breast cancer xenograft model in nude mice**

| **Model** | ***n*** | **Agent** | **Dose (mg/kg on days 0, 4, 8)** | **TGI (%)^{a}** | **BWLma x (%) or deaths)^{b}** | **Tumor regressions** | |
|---|---|---|---|---|---|---|---|
| | | | | | | **Partial^{c}** | **Complete^{d}** |
| MDA-MB-231 (breast) | 10 | Saline | 0 | - | - | 0/10 | 0/10 |
| Study No. ABS-15 | 10 | Nab-**2**^{e} | 120 | 96 | 5/10 | 3/10 | 0/10 |
| | 10 | Nab-**2** | 90 | 96 | -5.00 | 2/10 | 1/10 |
| | 10 | Nab-**2** | 60 | 97 | -3.30 | 4/10 | 2/10 |
| | 10 | Taxotere® | 15 | 88 | -3.30 | 4/10 | 0/10 |

| | | | | | | | |
|---|---|---|---|---|---|---|---|
| ^{a}TGI, tumor growth inhibition; ^{b}BWLmax, percent maximum body weight loss; ^{c}Tumor becomes <50% of its size after treatment or becomes unpalpable, but subsequently recurs. ^{d}Tumor becomes unpalpable; ^{e}Nab, nanoparticle albumin-bound. | | | | | | | |

### Example 18: Anticancer activity of nab-2 (nanoparticles of 2'-hexanoyldocetaxel prepared in example 12) against lung cancer xenograft.

To determine the efficacy of Nab-**2** against lung cancer, male nude mice were implanted with one million H358 cells s.c. near the right flank. Eleven days later, tumor-bearing mice (mean tumor size = 422 mm³) in groups of seven or nine were treated with saline or 60 mg/kg of Nab-2 or 10 mg/kg of Taxotere® administered i.v. on a q4d×3 schedule. Tumor measurements and animal weights were recorded twice weekly.

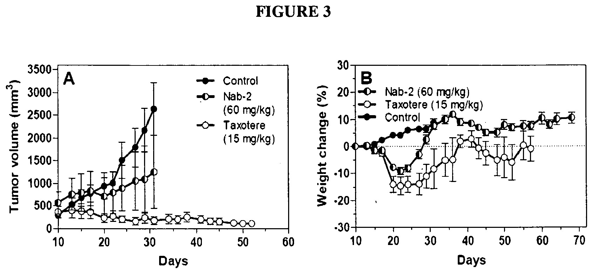

In the H358 human non-small cell lung cancer model, Nab-**2** at 60 mg/kg was well-tolerated with a maximum average weight loss of 9.1% and decreased tumor growth by 53% (*P* < 0.001 vs. saline control; Figure 3 (values are mean ± SEM) and Table 6). Nab-**2** at 90 mg/kg resulted in 100% mortality, Taxotere® at 10 mg/kg although decreased tumor growth by 93% (*P* < 0.001 vs. saline control), it caused 57% mortality. Unlike Taxotere®, Nab-**2** at 60 mg/kg dose level caused partial tumor regression in six of nine animals with minimal toxicity.

**Table 6: Anti-tumor activity of Nab-2 compared with Taxotere® in the s.c. human lung cancer xenograft model in nude mice**

| **Model** | ***n*** | **Agent** | **Dose (mg/kg on days 0, 4, 8)** | **TGI (%)^{a}** | **BWLmax (%) or deaths)^{b}** | **Tumor regressions** | |
|---|---|---|---|---|---|---|---|
| | | | | | | **Partial^{c}** | **Complete^{d}** |
| H358 (NSCLC)^{f} | 7 | Saline | 0 | - | - | 0/7 | 0/7 |
| Study No. CA-AB-24 | 7 | Nab-**2** | 90 | N/A | 7/7 | -- | -- |
| | 9 | Nab-**2** | 60 | 53 | -9.10 | 6/9 | 0/9 |
| | 7 | Taxotere® | 10 | 93 | 4/7 | 1/7 | 1/7 |

| | | | | | | | |
|---|---|---|---|---|---|---|---|
| ^{a}TGI, tumor growth inhibition; ^{b}BWLmax, percent maximum body weight loss; ^{c}Tumor becomes <50% of its size after treatment or becomes unpalpable, but subsequently recurs; ^{d}Tumor becomes unpalpable; ^{e}Nab, nanoparticle albumin-bound, ^{f}NSCLC, non-small cell lung cancer. | | | | | | | |

### Example 19: Anticancer activity of nab-2 (nanoparticles of 2'-hexanoyldocetaxel prepared in example 12) against colorectal cancer xenograft.

Two separate studies were conducted to determine the efficacy of Nab-**2** against colorectal cancer.

In study number CA-AB-6, male nude mice were implanted with 1 million HT29 s.c. into both the right and left flanks. Fifteen days later, tumor-bearing mice (mean tumor size = 143 mm³) in groups of three were treated with saline or 90 mg/kg of Nab-**2** or 22 mg/kg Nab-docetaxel administered i.v. on a q4d×3 schedule. Tumor measurements and animal weights were recorded three times weekly.

In study number ABS-18, male nude mice were implanted with 1 million HT29 cells s.c. near the right flank. Eleven days later, tumor-bearing mice (mean tumor size = 186 mm³) in groups of ten were treated with saline or 60 and 90 mg/kg of Nab-**2** or 15 mg/kg of Taxotere® administered i.v. on a q4d×3 schedule. Tumor measurements and animal weights were recorded twice weekly.

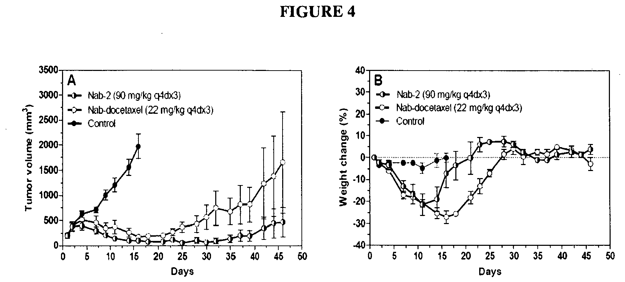

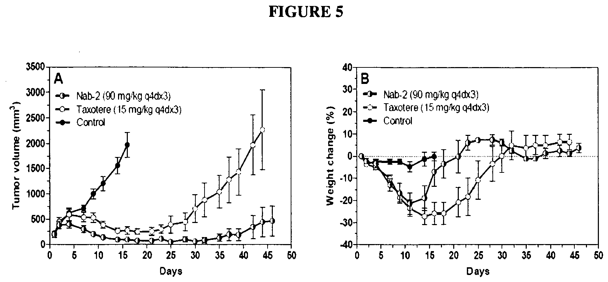

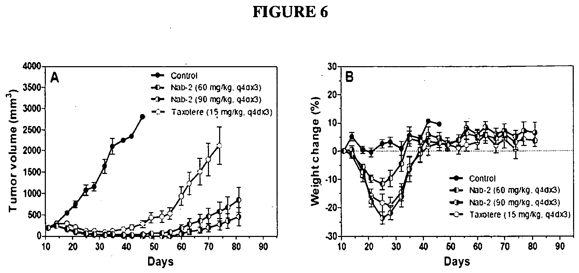

In the HT29 colon tumor xenografts (Study number ABS-18 and CA-AB-6), Nab-**2** induced marked tumor growth inhibition (95-99%) and tumor growth delay at dose levels of 60 and 90 mg/kg (*P* < 0.0001 vs. saline control; Figures 4-6 (values are mean ± SEM) and Table 7). Partial tumor regression occurred in nine of ten tumor-bearing mice treated with 90 or 60 mg/kg of Nab-**2** with a maximum average weight loss of 11.6% (ABS-18). At 90 mg/kg dose level, one animal died and one was euthanized. Under identical experimental conditions, Taxotere® at 15 mg/kg caused 94% TGI and 19% decrease in body weight.

**Table 5. Anti-tumor activity of Nab-2 compared with Nab-docetaxel and Taxotere® in the s.c. human colon cancer xenograft models in nude mice**

| **Model** | ***n*** | **Agent** | **Dose (mg/kg on days 0, 4, 8)** | **TGI (%)^{a}** | **BWLmax (% or deaths)^{b}** | **Tumor regressions** | |
|---|---|---|---|---|---|---|---|
| | | | | | | **Partial^{c}** | **Complete^{d}** |
| HT29 (colon) Study No. CA-AB-6 | 12 | Saline | 0 | - | - | 0/12 | 0/12 |
| | 3 | Nab-**2** | 90 | 95.1 | -19.00 | 1/3 | 0/3 |
| | 3 | Nab-**2** | 30 | -53.5 | -7.20 | 0/3 | 0/3 |
| | 3 | Nab-**2** | 15 | -5.1 | -9.60 | 0/3 | 0/3 |
| | 3 | Nab-docetaxel | 22 | 91.2 | -27.10 | 0/3 | 0/3 |
| | 3 | Taxotere® | 15 | 86.1 | -27.30 | 0/3 | 0/3 |
| HT29 (colon) Study No. ABS-18 | 10 | Saline | 0 | - | - | 0/10 | 0/10 |
| | 10 | Nab-**2** | 90 | 99 | 3/10 | 9/10 | 1/10 |
| | 10 | Nab-**2** | 60 | 98 | -11.60 | 9/10 | 0/10 |
| | 10 | Taxotere® | 15 | 94.3 | -19.10 | 3/10 | 0/10 |

| | | | | | | | |
|---|---|---|---|---|---|---|---|
| ^{a}TGI, tumor growth inhibition; ^{b}BWLmax, percent maximum body weight loss, ^{c}Tumor becomes <50% of its size after treatment or becomes unpalpable, but subsequently recurs; ^{d}Tumor becomes unpalpable; ^{e}Nab, nanoparticle albumin-bound. | | | | | | | |

Comparative efficacy studies with Nab-**2**, Nab-docetaxel, and Taxotere® in the HT29 colon tumor model (Study number CA-AB-6) revealed dose-limiting toxicity of Nab-docetaxel and Taxotere® with a 27% average weight loss despite 86 to 91% TGI (*P* < 0.01 vs. saline control). In contrast, Nab-2 at the 90 mg/kg dose level caused complete tumor regression with a maximum body weight loss of 19% (*P* < 0.0001 vs. saline control). Thus, unlike Nab-**2**, both Taxotere® and Nab-docetaxel at their MTD's did not cause either partial nor complete tumor regression in the HT-29 colon tumor model but led to marked weight loss.

### Example 20: Pharmacokinetic and safety of Nab-2 in monkeys

### Materials and Methods

The day of the initial dose administration was designated Study Day 1, with subsequent days consecutively numbered. Days on study prior to the initial dose administration were consecutively numbered with the final day of acclimation referenced as day -1.

Three non-naive, male cynomolgus monkeys, confirmed to have no prior exposure to albumin, were assigned to dose groups as specified in Table 6 shown below. Animals weighed 5.4 - 6.7 kg and were 4 to 7 years of age at study initiation.

**Table 6. Pharmacokinetic study design**

| **Group** | **Material** | **Route** | **Dose Level** | **Dosing Days** | **Number of Male Animals** |
|---|---|---|---|---|---|
| 3 | Nab-**2** | IV | 5 mg/kg | 1, 8, 15 | 1 |
| 4 | Nab-**2** | IV | 10 mg/kg | | 1 |
| 5 | Nab-**2** | IV | 20 mg/kg | 1 | 1 |
| | | | 10 mg/kg | 8, 15 | |

All animals were dosed once weekly, on days 1, 8 and 15. Animals in Groups 3 and 4 were administered Nab-**2** (lot: ABI139-2-83) at 5 and 10 mg/kg, respectively, and the Group 5 animal was administered Nab-**2** at 20, 10 and 10 mg/kg (on days 1, 8 and 15, respectively) via 30 minute intravenous infusion. Clinical observations were performed twice daily and body weights were recorded weekly throughout the study. Serial blood samples for pharmacokinetic analysis were collected at 8 time points for Group 1 animals and at 9 time points for Groups 3, 4 and 5, on days 1 and 15.

### Results and Conclusions

No treatment-related effects were found in body weights or clinical observations of Group 3 and 4 animals. In general, the nab-**2** test article was well tolerated following both oral and intravenous repeated dose administration. The Nab-2 test article was well tolerated following intravenous administration at 5 and 10 mg/kg.

In contrast, a single IV dose administration of Nab-**2** at 20 mg/kg (Group 5) resulted in adverse clinical signs and a decrease in body weight. For these reasons, the dose level for this animal was decreased to 10 mg/kg for dosing on days 8 and 15. Adverse clinical observations in the Group 5 animal included emesis, bloody, mucous, soft and liquid feces, and inappetence. On day 7, the Group 5 animal was observed lying down and showed hunched posture on day 17. Inappetence and a decline in overall condition, correlated with a loss of approximately 7% of its body weight by day 7 (compared to pre-dose weight measurements) and approximately 15% of its pre-dose body weight by day 15.

Analyte and PK analyses are shown below in Tables 7 and 8, respectively. Nab-**2** exhibited break down in blood with terminal half life of 3.0-3.7 hr and Tmax at earliest collection time of 0.083 hr. Nab-**2** and its metabolite (docetaxel) exhibited dose proportional increase in Cmax and AUC. Compound **2** exhibited large volume of distribution with Vz of 7-11 L/kg. The metabolite conversion rate (ratio of docetaxel AUC inf / Compound **2** AUC inf) was 4.8-5.9%.

These data clearly shown that Nab-**2** can be safely administered at 10 mg/kg or 120 mg/m² with dose proportional PK. Compound **2** was shown to produce docetaxel with a conversion rate of 4.8-5.9%.

**Table 7. Analyte data from Nab-2 IV administration**

| **Time (hr)** | **Concentration (ng/mL)** | **Dose (mg/kg)-Analyte** |
|---|---|---|
| 0 | 0 | 5-compound **2** |
| 0.083 | 1074.29 | 5-compound **2** |
| 0.25 | 939.925 | 5-compound **2** |
| 0.5 | 774.415 | 5-compound **2** |
| 1 | 534.319 | 5-compound **2** |
| 2 | 421.84 | 5-compound **2** |
| 8 | 136.199 | 5-compound **2** |
| 0 | 0 | 5-Docetaxel |
| 0.083 | 36.706 | 5-Docetaxel |
| 0.25 | 40.729 | 5-Docetaxel |
| 0.5 | 35.458 | 5-Docetaxel |
| 1 | 32.11 | 5-Docetaxel |
| 2 | 34.063 | 5-Docetaxel |
| 8 | 1.154 | 5-Docetaxel |
| 0 | 0 | 10-compound **2** |
| 0.083 | 1547.177 | 10-compound **2** |
| 0.25 | 1300.995 | 10-compound **2** |
| 0.5 | 935.651 | 10-compound **2** |
| 1 | 733.656 | 10-compound **2** |
| 2 | 657.257 | 10-compound **2** |
| 8 | 202.185 | 10-compound **2** |
| 0 | 0 | 10-Docetaxel |
| 0.083 | 99.44 | 10-Docetaxel |
| 0.25 | 70.117 | 10-Docetaxel |
| 0.5 | 63.362 | 10-Docetaxel |
| 1 | 47.475 | 10-Docetaxel |
| 2 | 44.227 | 10-Docetaxel |
| 8 | 0 | 10-Docetaxel |
| 0 | 0 | 20-compound **2** |
| 0.083 | 2387.681 | 20-compound **2** |
| 0.25 | 2617.855 | 20-compound **2** |
| 0.5 | 1819.776 | 20-compound **2** |
| 1 | 1365.583 | 20-compound **2** |
| 2 | 958.369 | 20-compound **2** |
| 8 | 264.147 | 20-compound **2** |
| 0 | 0 | 20-Docetaxel |
| 0.083 | 381.848 | 20-Docetaxel |
| 0.25 | 231.964 | 20-Docetaxel |
| 0.5 | 157.466 | 20-Docetaxel |
| 1 | 90.505 | 20-Docetaxel |
| 2 | 69.21 | 20-Docetaxel |
| 8 | 0 | 20-Docetaxel |

**Table 8. Pharmacokinetic analysis from Nab-2 IV administration**

| **Dose (mg/kg)/Analyte** | **HL_Lambda_z (hr)** | **Tmax (hr)** | **Cmax (ng/mL)** | **Cmax/D (kg^{∗}ng/ mL/mg)** | **AUCINF (hr^{∗}ng/mL)** |
|---|---|---|---|---|---|
| 10-compound **2** | 3.7 | 0.0830 | 1547 | 155 | 5346 |
| 10-Docetaxel | 3.2 | 0.0830 | 99 | 10 | 314 |
| 20-compound **2** | 3.0 | 0.2500 | 2618 | 131 | 7857 |
| 20-Docetaxel | 1.4 | 0.0830 | 382 | 19 | 394 |
| 5- compound **2** | 3.6 | 0.0830 | 1074 | 215 | 3613 |
| 5-Docetaxel | 1.4 | 0.2500 | 41 | 8 | 175 |
| | | | | | |

| **Dose (mg/kg) Analyte** | **AUCINF/D (hr^{∗}kg^{∗}ng/mL/mg)** | **Vz (mL/kg)** | **Cl (mL/hr/kg)** | **Vss (mL/kg)** | **Ratio: Docetaxel/compound 2** |
|---|---|---|---|---|---|
| 10-compound **2** | 535 | 9927 | 1871 | 8120 | 5.9% |
| 10-Docetaxel | 31 | | | | |
| 20-compound **2** | 393 | 11176 | 2545 | 8740 | 5.0% |
| 20-Docetaxel | 20 | | | | |
| 5-compound **2** | 723 | 7176 | 1384 | 5837 | 4.8% |
| 5-Docetaxel | 35 | | | | |

### Example 21: Dissolution profile of Nab-2 and reference composition Nab-docetaxel

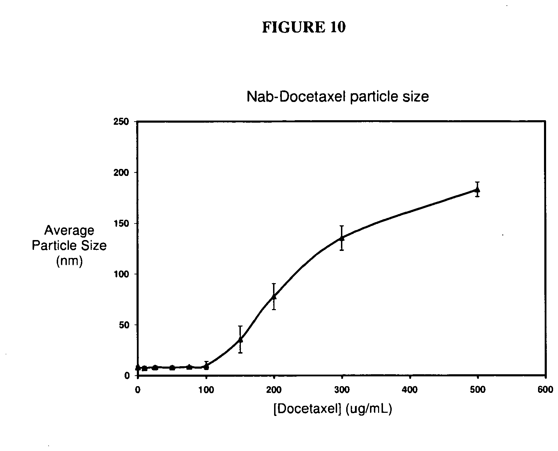

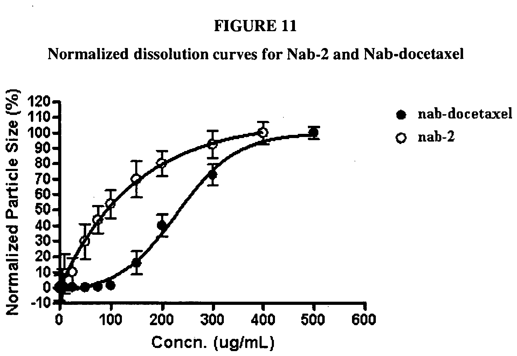

Dissolution experiments were carried out for Nab-2 and Nab-docetaxel (See Table 9/Figure 9 and Table 10/Figure 10 for Nab-2 and Nab-docetaxel, respectively). Particles of Nab-2 remained intact at the lowest concentrations tested (5 ug/mL). In contrast, nab-docetaxel rapidly breaks down to the albumin-drug complex with no detectable nanoparticles at 100 ug/mL (a 20-fold difference in stability). Correspondingly, the EC50 (the half point of the dissolution profile) was 103 ug/mL for nab-**2** and 230 ug/mL for nab-docetaxel (Figure 11)- a 2X difference. The EC90-the 90% dissolution- was 16 ug/ml for nab-**2** and 121 ug/ml for nab-docetaxel- a 7.6 X difference. Normalized dissolution curves for Nab-2 and Nab-docetaxel are shown in Figure 11.

**Table 9: Dissolution data for Nab-2**

| Drug conc. (µg/mL) | Particle Size (nm) | Standard Deviation (± nm) | |
|---|---|---|---|
| 0 | 23.9 | 3.5 | 0.2 µm filtered 5 % (w/v) HSA soln. |
| 0 | 23.9 | 3.2 | |
| 5 | 24.4 | 4.3 | |
| 10 | 27.6 | 5.1 | |
| 25 | 28 | 3.7 | |
| 50 | 35.9 | 4.5 | |
| 75 | 41.5 | 3.5 | 0.1 µm filtered 5 % (w/v) HSA soln. |
| 100 | 45.6 | 3.6 | |
| 150 | 52.1 | 4.7 | |
| 200 | 56.3 | 3.4 | |
| 300 | 61.3 | 3.5 | |
| 400 | 64.3 | 2.8 | |

**Table 10: Dissolution data for Nab-docetaxel**

| Drug conc. (µg/mL) | Particle Size (nm) | Standard Deviation (± nm) |
|---|---|---|
| 0 | 8.1 | 1.5 |
| 10 | 7.6 | 1.7 |
| 25 | 8.2 | 1.6 |
| 50 | 8.1 | 1.5 |
| 75 | 8.7 | 2.3 |
| 100 | 10.1 | 3.9 |
| 150 | 35.7 | 13.2 |
| 200 | 77.8 | 12.7 |
| 300 | 135.4 | 12 |
| 500 | 183.3 | 7.2 |

### Reference Reference Example 22: Dissolution profile of Nab-paclitaxel

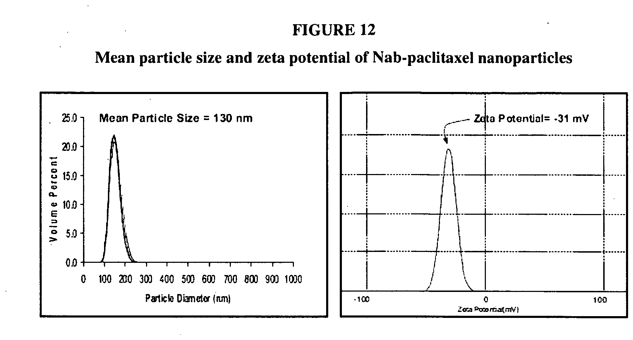

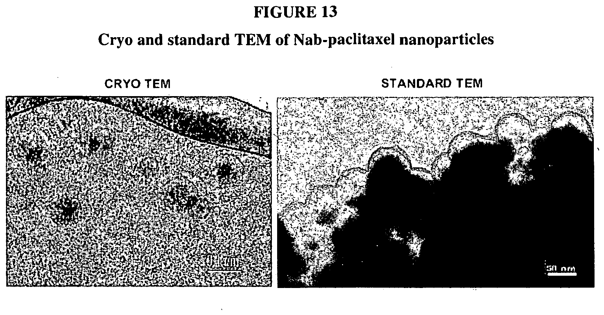

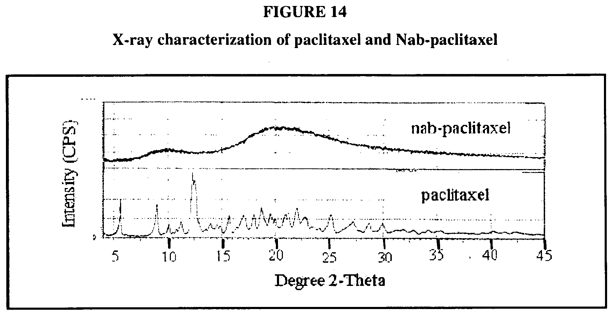

Nab-paclitaxel (Abraxane; 100 mg) was reconstituted with 20 mL of 0.9 % sodium chloride irrigation to obtain a suspension of paclitaxel (5 mg/mL) as prescribed in the package insert. Nanoparticle size of Abraxane was measured by quasi-elastic laser light scattering (DLS) with Malvern Zetasizer 3000. Zeta potential was measured with Malvern Zetasizer 3000. Abraxane nanoparticles were characterized by transmission electron microscopy (TEM) and cryo-TEM. Paclitaxel and nab-paclitaxel were analyzed by X-ray powder diffraction. The nanoparticle mean size of 130 nm was determined by TEM and DLS. XRD studies determined the paclitaxel in the nanoparticles to be in a noncrystalline, amorphous, and readily bioavailable state. Upon dilution, nab-paclitaxel nanoparticles quickly dissociated into soluble albumin-paclitaxel complexes with size similar to native albumin. Results are shown in Figures 12-14.

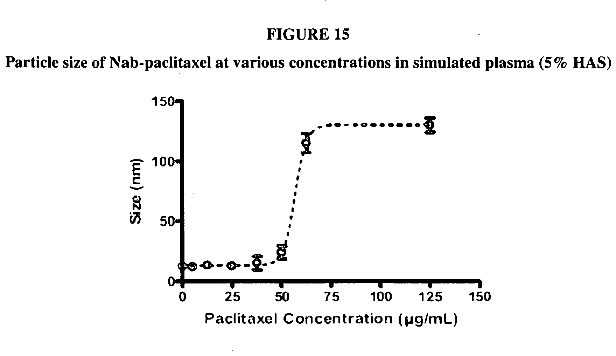

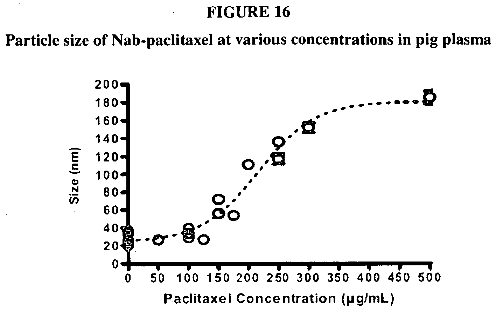

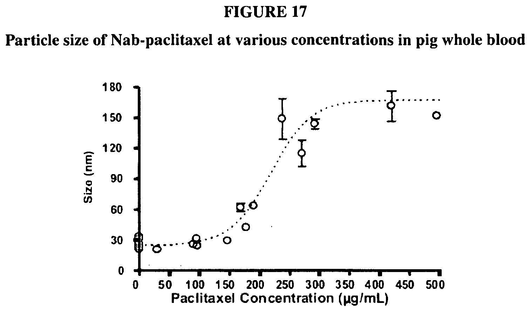

For the *in vitro* dissolution study, nab-paclitaxel suspension (5 mg/mL in 0.9% sodium chloride solution) was diluted to different concentrations in simulated plasma (5% HSA), pig plasma and pig whole blood. The pig blood was processed to plasma by centrifugation at 3000 rpm for 15 minutes. Particle size was measured by DLS with Malvern Zetasizer 3000. For dissolution in pig whole blood, fresh citrate anticoagulated pig whole blood containing Nab-paclitaxel was centrifuged at 3000 rpm for 15 min, which allows the complete removal of cell components of the blood without causing sedimentation of Abraxane nanoparticles. Particle size was determined across various concentrations of Abraxane by DLS. The *in vitro* dissolution study determined the threshold concentrations below which nab-paclitaxel nanoparticles would rapidly dissolve to be: 50-60 µg/mL in simulated plasma, 100 µg/mL in pig plasma, and 150 µg/mL in pig whole blood. Results are shown in Figures 15 to 17.

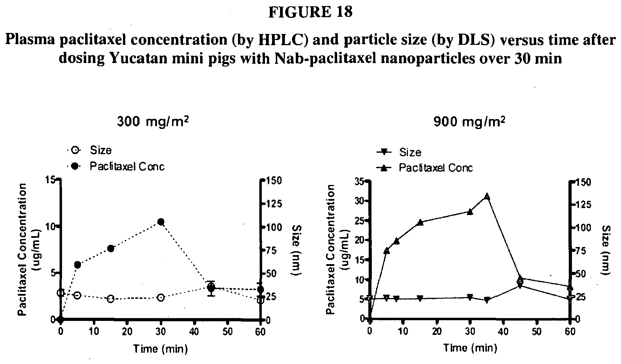

For the *in vivo* dissolution study, Yucatan minipigs received nab-paclitaxel via ear vein infusion at a dose of either 300 mg/m² (maximum tolerated dose, MTD) or 900 mg/m², administered over 30 minutes. Duplicate citrate anticoagulated blood samples were obtained prior to infusion and at indicated time points from the venae cavae (300 mg/m²) or the contralateral jugular vein (900 mg/m²). The blood samples were centrifuged at 3000 rpm for 15 min to obtain plasma and analyzed for particle size by DLS with Malvern Zetaszer 3000 and for paclitaxel concentration in blood by HPLC. *In vivo*, the peak concentrations of paclitaxel in circulation following the infusion of nab-paclitaxel at 300 mg/m² (MTD) or 900 mg/m² were 10.5 and 31.4 µg/mL, respectively, well below the dissolution threshold. As a result, no nanoparticles were detected from blood samples of pigs receiving nab-paclitaxel administration at any time point. Results are shown in Figure 18.

### Example 23: Tissue Distribution of 2'-hexanoyl docetaxel

Tumor bearing (HT29) mice were injected through the tail vei with 90mg/kg of Nab-**2** (Nab 2'-hexanoyl docetaxel). Animals sacrificed at 24hrs and various tissues analyzed for presence of 2'-hexanoyl docetaxel, docetaxel and other metabolites using LC/MS/MS. Majority concentration of the major metabolite docetaxel was found in the tumor relative to other organs. Results are shown in Figure 19.

## Claims

1. A composition comprising nanoparticles, wherein the nanoparticles comprise a hydrophobic taxane derivative coated with albumin, wherein at least about 5% of the albumin in the nanoparticle portion of the composition is crosslinked, and wherein the hydrophobic taxane derivative is of the formula: 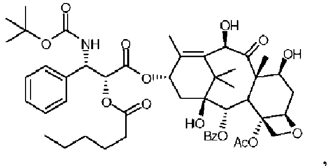 or a pharmaceutically acceptable salt, isomer, or solvate thereof.

2. The composition according to claim 1, wherein the albumin is human serum albumin.

3. The composition according to claim 1 or 2, wherein the composition comprises albumin in both nanoparticle form and non-nanoparticle form, and wherein less than about 25% of the albumin is in nanoparticle form.

4. The composition according to any one of claims 1 to 3, wherein the average diameter of the nanoparticles in the composition is less than about 200 nm, or is less than about 100 nm.

5. The composition according to any one of claims 1 to 4, wherein greater than 80%, or greater than 90% of the nanoparticles in the composition have a diameter of less than about 100 nm.

6. The composition according to any one of claims 1 to 5, wherein the nanoparticles have a size of more than about 20 nm at 10 µg/ml in a dissolution study in 5% HSA at 37 °C by HPLC.

7. The composition according to any one of claims 1 to 5, wherein the nanoparticles have a size of more than about 30 nm at 50 µg/ml in a dissolution study in 5% HSA at 37 °C by HPLC.

8. The composition according to any one of claims 1 to 5, wherein the nanoparticles exhibit one or more of the following dissolution profile when measured in 5% HSA at 37 °C by HPLC:
a) more than about 50 nm at 200 µg/ml;
b) more than about 40 nm at 100 µg/ml; and
c) more than about 20 nm at 10 µg/ml.

9. The composition according to any one of claims 1 to 5, wherein the nanoparticles exhibit one or more of the following dissolution profile when measured in 5% HSA at 37 °C by HPLC:
a) more than about 60 nm at 400 µg/ml;
b) more than about 50 nm at 200 µg/ml;
c) more than about 40 nm at 100 µg/ml;
d) more than about 20 nm at 10 µg/ml;
e) more than about 20 nm at 5 µg/ml.

10. The composition according to any one of claims 1 to 5, wherein the EC50 of the dissolution profile when measured in 5% HSA at 37 °C is less than about 25% of the EC50 for the unmodified taxane in the same nanoparticle formulation.

11. The composition according to any one of claims 1 to 5, wherein the nanoparticle composition when administered to a primate exhibits a Cmax in the blood at between about 0.05 hour to about 0.3 hour after administration.

12. The composition according to any one of claims 1 to 5, wherein the nanoparticle composition when administered in a primate exhibits break down in blood with terminal half life of about 1 hour to about 5 hours.

13. A composition as defined in any one of claims 1 to 12 for use in a method of treating a proliferative disease in an individual, said method comprising administering to the individual an effective amount of the composition.

14. The composition for use according to claim 13, wherein the proliferative disease is cancer, optionally wherein the cancer is a solid tumor; or wherein the cancer is selected from multiple myeloma, renal cell carcinoma, prostate cancer, lung cancer, melanoma, colon cancer, ovarian cancer, or breast cancer.

15. The composition for use according to claim 13 or 14, wherein said method comprises administration of the composition parenterally or intravenously.

## Patentansprüche

1. Zusammensetzung, umfassend Nanopartikel, wobei die Nanopartikel ein mit Albumin beschichtetes hydrophobes Taxanderivat umfassen, wobei mindestens etwa 5 % des Albumins in dem Nanopartikelteil der Zusammensetzung vernetzt ist und wobei das hydrophobe Taxanderivat die Formel: 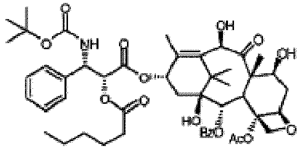 oder ein pharmazeutisch verträgliches Salz, Isomer oder Solvat davon aufweist.

2. Zusammensetzung nach Anspruch 1, wobei das Albumin ein Humanserumalbumin ist.

3. Zusammensetzung nach Anspruch 1 oder 2, wobei die Zusammensetzung Albumin sowohl in der Nanopartikelform als auch der Nicht-Nanopartikelform umfasst und wobei weniger als etwa 25 % des Albumins in der Nanopartikelform vorliegt.

4. Zusammensetzung nach einem der Ansprüche 1 bis 3, wobei der durchschnittliche Durchmesser der Nanopartikel in der Zusammensetzung weniger als etwa 200 nm beträgt oder weniger als etwa 100 nm beträgt.

5. Zusammensetzung nach einem der Ansprüche 1 bis 4, wobei mehr als 80 % oder mehr als 90 % der Nanopartikel in der Zusammensetzung einen Durchmesser von weniger als etwa 100 nm beträgt.

6. Zusammensetzung nach einem der Ansprüche 1 bis 5, wobei die Nanopartikel eine Größe von mehr als etwa 20 nm bei 10 µg/ml in einer Auflösungsstudie in 5 % HSA bei 37 °C mittels HPLC aufweisen.

7. Zusammensetzung nach einem der Ansprüche 1 bis 5, wobei die Nanopartikel eine Größe von mehr als etwa 30 nm bei 50 µg/ml in einer Auflösungsstudie in 5 % HSA bei 37 °C mittels HPLC aufweisen.

8. Zusammensetzung nach einem der Ansprüche 1 bis 5, wobei die Nanopartikel eines oder mehrere von dem folgenden Auflösungsprofil aufweisen, wenn sie in 5 % HSA bei 37 °C mittels HPLC gemessen werden:
a) mehr als etwa 50 nm bei 200 µg/ml;
b) mehr als etwa 40 nm bei 100 µg/ml; und
c) mehr als etwa 20 nm bei 10 µg/ml.

9. Zusammensetzung nach einem der Ansprüche 1 bis 5, wobei die Nanopartikel eines oder mehrere von dem folgenden Auflösungsprofil aufweisen, wenn sie in 5 % HSA bei 37 °C mittels HPLC gemessen werden:
a) mehr als etwa 60 nm bei 400 µg/ml;
b) mehr als etwa 50 nm bei 200 µg/ml;
c) mehr als etwa 40 nm bei 100 µg/ml;
d) mehr als etwa 20 nm bei 10 µg/ml;
e) mehr als etwa 20 nm bei 5 µg/ml.

10. Zusammensetzung nach einem der Ansprüche 1 bis 5, wobei die EC50 des Auflösungsprofils, wenn sie in 5 % HSA bei 37 °C gemessen wird, weniger als etwa 25 % der EC50 für das unmodifizierte Taxan in derselben Nanopartikel-Formulierung beträgt.

11. Zusammensetzung nach einem der Ansprüche 1 bis 5, wobei die Nanopartikelzusammensetzung zwischen etwa 0,05 Stunden bis etwa 0,3 Stunden nach der Verabreichung eine Cmax in Blut aufweist, wenn sie einem Primaten verabreicht wird.

12. Zusammensetzung nach einem der Ansprüche 1 bis 5, wobei die Nanopartikelzusammensetzung eine Zersetzung im Blut mit einer terminalen Halbwertszeit zwischen etwa 1 Stunde und etwa 5 Stunden aufweist, wenn sie einem Primaten verabreicht wird.

13. Zusammensetzung nach einem der Ansprüche 1 bis 12 zur Verwendung in einem Verfahren zur Behandlung einer proliferativen Erkrankung bei einem Individuum, wobei das Verfahren die Verabreichung einer wirksamen Menge der Zusammensetzung an das Individuum umfasst.

14. Zusammensetzung zur Verwendung nach Anspruch 13, wobei die proliferative Erkrankung Krebs ist, wobei der Krebs gegebenenfalls ein solider Tumor ist; oder wobei der Krebs aus Multiplem Myelom, Nierenzellkarzinom, Prostatakrebs, Lungenkrebs, Melanom, Darmkrebs, Eierstockkrebs oder Brustkrebs ausgewählt ist.

15. Zusammensetzung zur Verwendung nach Anspruch 13 oder 14, wobei das Verfahren eine parenterale oder intravenöse Verabreichung der Zusammensetzung umfasst.

## Revendications

1. Composition comprenant des nanoparticules, dans laquelle les nanoparticules comprennent un dérivé de taxane hydrophobe recouvert d'albumine, dans laquelle au moins environ 5 % de l'albumine dans la partie nanoparticulaire de la composition est réticulé, et dans laquelle le dérivé de taxane hydrophobe est de formule : 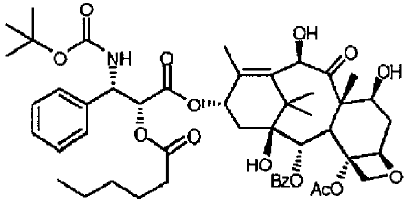 ou un sel, isomère ou solvate pharmaceutiquement acceptable de celui-ci.

2. Composition selon la revendication 1, dans laquelle l'albumine est une sérum albumine humaine.

3. Composition selon la revendication 1 ou 2, dans laquelle la composition comprend de l'albumine à la fois sous forme nanoparticulaire et sous forme non nanoparticulaire, et dans laquelle moins d'environ 25 % de l'albumine est sous forme nanoparticulaire.

4. Composition selon l'une quelconque des revendications 1 à 3, dans laquelle le diamètre moyen des nanoparticules dans la composition est inférieur à environ 200 nm ou est inférieur à environ 100 nm.

5. Composition selon l'une quelconque des revendications 1 à 4, dans laquelle plus de 80 % ou plus de 90 % des nanoparticules dans la composition ont un diamètre inférieur à environ 100 nm.

6. Composition selon l'une quelconque des revendications 1 à 5, dans laquelle les nanoparticules ont une taille supérieure à environ 20 nm à 10 µg/ml dans une étude de dissolution dans 5 % de SAH à 37 °C par CLHP.

7. Composition selon l'une quelconque des revendications 1 à 5, dans laquelle les nanoparticules ont une taille supérieure à environ 30 nm à 50 µg/ml dans une étude de dissolution dans 5 % de SAH à 37 °C par CLHP.

8. Composition selon l'une quelconque des revendications 1 à 5, dans laquelle les nanoparticules présentent un ou plusieurs des profils de dissolution suivants lorsqu'ils sont mesurés dans 5 % de SAH à 37 °C par CLHP :
a) plus d'environ 50 nm à 200 µg/ml ;
b) plus d'environ 40 nm à 100 µg/ml ; et
c) plus d'environ 20 nm à 10 µg/ml.

9. Composition selon l'une quelconque des revendications 1 à 5, dans laquelle les nanoparticules présentent un ou plusieurs des profils de dissolution suivants lorsqu'ils sont mesurés dans 5 % de SAH à 37 °C par CLHP :
a) plus d'environ 60 nm à 400 µg/ml ;
b) plus d'environ 50 nm à 200 µg/ml ;
c) plus d'environ 40 nm à 100 µg/ml ;
d) plus d'environ 20 nm à 10 µg/ml ;
e) plus d'environ 20 nm à 5 µg/ml.

10. Composition selon l'une quelconque des revendications 1 à 5, dans laquelle la CE50 du profil de dissolution mesurée dans 5 % de SAH à 37 °C est inférieure à environ 25 % de la CE50 pour le taxane non modifié dans la même formulation de nanoparticules.

11. Composition selon l'une quelconque des revendications 1 à 5, dans laquelle la composition des nanoparticules lorsqu'elle est administrée à un primate présente une Cmax dans le sang entre environ 0,05 h et environ 0,3 h après l'administration.

12. Composition selon l'une quelconque des revendications 1 à 5, dans laquelle la composition des nanoparticules lorsqu'elle est administrée à un primate présente une dégradation dans le sang avec une demi-vie terminale d'environ 1 h à environ 5 h.

13. Composition selon l'une quelconque des revendications 1 à 12, pour être utilisée dans une méthode de traitement d'une maladie proliférative chez un individu, ladite méthode comprenant l'administration à l'individu d'une quantité efficace de la composition.

14. Composition pour l'utilisation selon la revendication 13, dans laquelle la maladie proliférative est un cancer, éventuellement dans laquelle le cancer est une tumeur solide ; ou dans laquelle le cancer est choisi parmi le myélome multiple, le carcinome des cellules rénales, le cancer de la prostate, le cancer du poumon, le mélanome, le cancer du côlon, le cancer de l'ovaire ou le cancer du sein.

15. Composition pour l'utilisation selon la revendication 13 ou 14, dans laquelle ledit procédé comprend l'administration de la composition par voie parentérale ou intraveineuse.
